# EUROPEAN PATENT APPLICATION

(11) **EP 4 609 879 A2**
(43) Date of publication of application: **03.09.2025**
(21) Application number: 25186256.1
(22) Date of filing: 17.05.2019
(51) Int. Cl.: A61K 47/60

(54) **STARTING DOSE OF PTH CONJUGATES**

(30) Priority: 18.05.2018 EP 18173155
(62) Divisional of application: 19724825.5
(71) Applicant: Ascendis Pharma Bone Diseases A/S, 2900 Hellerup (DK)
(72) Inventor: Sprogøe, Kennett, 2900 Hellerup (DK); Karpf, David Brian, Mountain View, 94043 (US); Strange, Claus, 2900 Hellerup (DK)
(74) Representative: Büchel, Edwin

(57) **Abstract**

The present invention relates to a PTH conjugate, in which a PTH moiety is reversibly conjugated to a polymeric moiety or fatty acid-based moiety, or a pharmaceutically acceptable salt thereof or a pharmaceutical composition comprising said PTH conjugate or pharmaceutically acceptable salt thereof for use in the treatment, control, delay or prevention of a disease that can be treated, controlled, delayed or prevented with PTH, wherein the starting dose ranges from from 0.8 to 4.9 nmol/day and to the respective methods of treatment.

## Description

The present invention relates to a PTH conjugate, in which a PTH moiety is reversibly conjugated to a polymeric moiety or fatty acid-based moiety, or a pharmaceutically acceptable salt thereof or a pharmaceutical composition comprising said PTH conjugate or pharmaceutically acceptable salt thereof for use in the treatment, control, delay or prevention of a disease that can be treated, controlled, delayed or prevented with PTH, wherein the starting dose ranges from 0.8 to 4.9 nmol/day and to the respective methods of treatment.

Hypoparathyroidism (HP) is a rare disorder of mineral metabolism. The clinical presentation may include muscle cramping, spasms, or tetany and, rarely, life-threatening events such as seizure or laryngospasm. Etiology is often neck surgery in adults or genetic disorders in children.

Conventional therapy with vitamin D analogs and calcium can normalize serum calcium, via increased intestinal absorption, but does not restore PTH-dependent renal calcium reabsorption or correct diminished bone turnover. Hypercalciuria and hyperphosphatemia often results, even when serum calcium is maintained at below-normal levels. Chronic hypercalciuria can cause irreversible renal damage and renal failure, and sustained hyperphosphatemia, resulting in an increased CxP product, can cause ectopic calcification in the renal parenchyma, the vascular system, the lens of the eye, and the basal ganglia of the brain, leading to additional potential morbidity and mortality. To reduce this risk, the vitamin D analog and calcium dosages are often reduced to maintain serum calcium at the lowest tolerated level. Consequently, many hypoparathyroid patients suffer varying degrees of hypocalcemia to avoid hypercalciuria-induced renal damage. (Clin Endocrinol Metab. 2012 Feb; 97(2): 391-399).

It is well known that overproduction of PTH produces a distinct pathophysiologic syndrome, hyperparathyroidism, characterized by hypercalcemia, hypercalciuria, hypophosphatemia, phosphaturia, and increases in osteoclastic bone resorption. Continuous exposure to supraphysiological PTH following continuous infusion replicates many of the symptoms of hyperparathyroidism. In this study, both PTH doses infused led to hypercalcemia with a resultant marked reduction in endogenous PTH (1-84) secretion, consistent with supraphysiological PTH doses. The suppression of endogenous PTH(1-84) secretion occurs in response to increases in serum calcium. The maximum suppression in response to hypercalcemia has been shown to be down to about 3-5 pg/ml in healthy volounteers, irrespective of baseline serum calcium values. (Journal of Bone and Mineral Research, Vol. 26, No. 9, September 2011, pp 2287-2297)

As one goal of HP therapy is to normalize serum calcium, supraphysiological levels of PTH should be avoided.

Single or twice-daily injection of PTH 1-34 can restore serum and urine calcium to the normal or near-normal range. Teriparatide has a short half-life of about 5 minutes after IV administration and 1 hour after subcutaneous administration due to prolonged absorbtion (Forteo Prescribing Information). Better results from twice-daily dosing can be explained by the short half-life of PTH(1-34), whose calcium-normalizing effects do not last a full 24 h after single administration.

In 2015, Natpara, PTH(1-84), was approved for once-daily subcutaneous injection as an adjunct to vitamin D and calcium in patients with hypoparathyroidism. While this represents an important advance in the treatment of the disease, Natpara has not demonstrated an ability to reduce incidences of hypercalcemia, hypocalcemia, or hypercalciuria relative to conventional therapy in treated patients, likely due to its short half-life (about 5 minutes IV; about 3 hours after subcutaneous administration).

As such, there is a high need for improved PTH based therapies for hypoparathyroidism that can maintain PTH levels and serum calcium in the physiological range. One such approach is to provide continuous exposure to PTH. For example, to facilitate more physiological PTH levels, clinical studies have been conducted with PTH(1-34) administered by pump delivery in comparison with twice-daily injections. Pump delivery of PTH(1-34) achieved simultaneous normalization of markers of bone turnover, serum calcium, serum phosphate, serum magnesium, and urine calcium excretion.

However, the requirement to be reliant on an infusion pump is cumbersome for patients, and it would be a significant improvement if a continuous infusion-like exposure to PTH could be achieved with a single daily injection.

In order to address this issue reversible polymer conjugates of PTH were developed (WO2017/148883A1), which provide a sustained continuous release of PTH with daily injections. As both hypo- and hypercalcemia is associated with significant morbidity, it is paramount that the starting dose of such daily therapy is properly selected to ensure that patients receive both a clinical benefit while reducing the risk of adverse effects. So far, no information regarding suitable and safe starting doses for such PTH conjugates is available.

Thus, there is a need for identifying a safe and efficacious starting dose of a long acting PTH conjugates. This is the objective of the present invention.

This object is achieved with a PTH conjugate, in which a PTH moiety is reversibly conjugated to a polymeric moiety or fatty acid-based moiety, or a pharmaceutically acceptable salt thereof or a pharmaceutical composition comprising said PTH conjugate or pharmaceutically acceptable salt thereof for use in the treatment, control, delay or prevention of a disease that can be treated, controlled, delayed or prevented with PTH, wherein the starting dose ranges from 0.8 to 4.9 nmol/day. In certain embodiments the starting dose ranges from 0.8 to 3.9 nmol/day.

In another aspect the present invention relates to a method of treating, controlling, delaying or preventing in a patient suffering from a disease which can be treated, controlled, delayed or prevented with PTH, comprising administering an effective amount of a PTH conjugate, in which a PTH moiety is reversibly conjugated to a polymeric moiety or fatty acid-based moiety, or a pharmaceutically acceptable salt thereof or a pharmaceutical composition comprising said PTH conjugate or pharmaceutically acceptable salt thereof to the patient in a starting dose ranging from 0.8 to 4.9 nmol/day. In certain embodiments the starting dose ranges from 0.8 to 3.9 nmol/day.

It was surprisingly found that a dose in the range 0.8 to 4.9 nmol/day, was able to induce suppression of endogenous PTH(1-84) secretion, while not inducing hypercalcemia and that a dose in the range 0.8 to 3.9 nmol/day was able to increase serum calcium, while not completely suppressing endogenous PTH(1-84) secretion.

Within the present invention the terms are used having the meaning as follows.

As used herein the term "starting dose" refers to the dose of the PTH conjugate of the present invention that is administered to a patient when first initiating therapy with a PTH conjugate, i.e. such patient has not previously received a dose of said PTH conjugate. It is understood, that such starting dose may be continued for a period of time, such as several weeks or months, to allow the patient to stabilize and adjust other medication, such as oral calcium and active vitamin D.

As used herein the term "PTH" refers to all PTH polypeptides, preferably from mammalian species, more preferably from human and primate species, more preferably from human, as well as their variants, analogs, orthologs, homologs, and derivatives and fragments thereof, that are characterized by raising serum calcium and renal phosphorus excretion and lowering serum phosphorus and renal calcium excretion. The term "PTH" also refers to all PTHrP polypeptides, such as the polypeptide of SEQ ID NO:121, that bind to and activate the common PTH/PTHrP1 receptor. Preferably, the term "PTH" refers to the PTH polypeptide of SEQ ID NO:51 as well as its variants, homologs and derivatives exhibiting essentially the same biological activity, i.e. raising serum calcium and renal phosphorus excretion, and lowering serum phosphorus and renal calcium excretion.

Preferably, the term "PTH" refers to a polypeptide sequence selected from the group consisting of:
SEQ ID NO:1 (PTH 1-84) 
SVSEIQLMHNLGKHLNSMERVEWLRKKLQDVHNFVALGAPLAPRDAGSQRPRKKE
DNVLVESHEKSLGEADKADVNVLTKAKSQ
SEQ ID NO:2 (PTH 1-83) 
SVSEIQLMHNLGKHLNSMERVEWLRKKLQDVHNFVALGAPLAPRDAGSQRPRKKE
DNVLVESHEKSLGEADKADVNVLTKAKS
SEQ ID NO:3 (PTH 1-82) 
SVSEIQLMHNLGKHLNSMERVEWLRKKLQDVHNFVALGAPLAPRDAGSQRPRKKE
DNVLVESHEKSLGEADKADVNVLTKAK
SEQ ID NO:4 (PTH 1-81) 
SVSEIQLMHNLGKHLNSMERVEWLRKKLQDVHNFVALGAPLAPRDAGSQRPRKKE
DNVLVESHEKSLGEADKADVNVLTKA
SEQ ID NO:5 (PTH 1-80) 
SVSEIQLMHNLGKHLNSMERVEWLRKKLQDVHNFVALGAPLAPRDAGSQRPRKKE
DNVLVESHEKSLGEADKADVNVLTK
SEQ ID NO:6 (PTH 1-79) 
SVSEIQLMHNLGKHLNSMERVEWLRKKLQDVHNFVALGAPLAPRDAGSQRPRKKE
DNVLVESHEKSLGEADKADVNVLT
SEQ ID NO:7 (PTH 1-78) 
SVSEIQLMHNLGKHLNSMERVEWLRKKLQDVHNFVALGAPLAPRDAGSQRPRKKE
DNVLVESHEKSLGEADKADVNVL
SEQ ID NO:8 (PTH 1-77) 
SVSEIQLMHNLGKHLNSMERVEWLRKKLQDVHNFVALGAPLAPRDAGSQRPRKKE
DNVLVESHEKSLGEADKADVNV
SEQ ID NO:9 (PTH 1-76) 
SVSEIQLMHNLGKHLNSMERVEWLRKKLQDVHNFVALGAPLAPRDAGSQRPRKKE
DNVLVESHEKSLGEADKADVN
SEQ ID NO:10 (PTH 1-75) 
SVSEIQLMHNLGKHLNSMERVEWLRKKLQDVHNFVALGAPLAPRDAGSQRPRKKE
DNVLVESHEKSLGEADKADV
SEQ ID NO:11 (PTH 1-74) 
SVSEIQLMHNLGKHLNSMERVEWLRKKLQDVHNFVALGAPLAPRDAGSQRPRKKE
DNVLVESHEKSLGEADKAD
SEQ ID NO:12 (PTH 1-73) 
SVSEIQLMHNLGKHLNSMERVEWLRKKLQDVHNFVALGAPLAPRDAGSQRPRKKE
DNVLVESHEKSLGEADKA
SEQ ID NO:13 (PTH 1-72) 
SVSEIQLMHNLGKHLNSMERVEWLRKKLQDVHNFVALGAPLAPRDAGSQRPRKKE
DNVLVESHEKSLGEADK
SEQ ID NO:14 (PTH 1-71) 
SVSEIQLMHNLGKHLNSMERVEWLRKKLQDVHNFVALGAPLAPRDAGSQRPRKKE
DNVLVESHEKSLGEAD
SEQ ID NO:15 (PTH 1-70) 
SVSEIQLMHNLGKHLNSMERVEWLRKKLQDVHNFVALGAPLAPRDAGSQRPRKKE
DNVLVESHEKSLGEA
SEQ ID NO:16 (PTH 1-69) 
SVSEIQLMHNLGKHLNSMERVEWLRKKLQDVHNFVALGAPLAPRDAGSQRPRKKE
DNVLVESHEKSLGE
SEQ ID NO:17 (PTH 1-68) 
SVSEIQLMHNLGKHLNSMERVEWLRKKLQDVHNFVALGAPLAPRDAGSQRPRKKE
DNVLVESHEKSLG
SEQ ID NO:18 (PTH 1-67) 
SVSEIQLMHNLGKHLNSMERVEWLRKKLQDVHNFVALGAPLAPRDAGSQRPRKKE
DNVLVESHEKSL
SEQ ID NO:19 (PTH 1-66) 
SVSEIQLMHNLGKHLNSMERVEWLRKKLQDVHNFVALGAPLAPRDAGSQRPRKKE
DNVLVESHEKS
SEQ ID NO:20 (PTH 1-65) 
SVSEIQLMHNLGKHLNSMERVEWLRKKLQDVHNFVALGAPLAPRDAGSQRPRKKE
DNVLVESHEK
SEQ ID NO:21 (PTH 1-64) 
SVSEIQLMHNLGKHLNSMERVEWLRKKLQDVHNFVALGAPLAPRDAGSQRPRKKE
DNVLVESHE
SEQ ID NO:22 (PTH 1-63) 
SVSEIQLMHNLGKHLNSMERVEWLRKKLQDVHNFVALGAPLAPRDAGSQRPRKKE
DNVLVESH
SEQ ID NO:23 (PTH 1-62) 
SVSEIQLMHNLGKHLNSMERVEWLRKKLQDVHNFVALGAPLAPRDAGSQRPRKKE
DNVLVES
SEQ ID NO:24 (PTH 1-61) 
SVSEIQLMHNLGKHLNSMERVEWLRKKLQDVHNFVALGAPLAPRDAGSQRPRKKE
DNVLVE
SEQ ID NO:25 (PTH 1-60) 
SVSEIQLMHNLGKHLNSMERVEWLRKKLQDVHNFVALGAPLAPRDAGSQRPRKKE
DNVLV
SEQ ID NO:26 (PTH 1-59) 
SVSEIQLMHNLGKHLNSMERVEWLRKKLQDVHNFVALGAPLAPRDAGSQRPRKKE
DNVL
SEQ ID NO:27 (PTH 1-58) 
SVSEIQLMHNLGKHLNSMERVEWLRKKLQDVHNFVALGAPLAPRDAGSQRPRKKE
DNV
SEQ ID NO:28 (PTH 1-57) 
SVSEIQLMHNLGKHLNSMERVEWLRKKLQDVHNFVALGAPLAPRDAGSQRPRKKE
DN
SEQ ID NO:29 (PTH 1-56) 
SVSEIQLMHNLGKHLNSMERVEWLRKKLQDVHNFVALGAPLAPRDAGSQRPRKKE
D
SEQ ID NO:30 (PTH 1-55)
   SVSEIQLMHNLGKHLNSMERVEWLRKKLQDVHNFVALGAPLAPRDAGSQRPRKKE
SEQ ID NO:31 (PTH 1-54)
   SVSEIQLMHNLGKHLNSMERVEWLRKKLQDVHNFVALGAPLAPRDAGSQRPRKK
SEQ ID NO:32 (PTH 1-53)
   SVSEIQLMHNLGKHLNSMERVEWLRKKLQDVHNFVALGAPLAPRDAGSQRPRK
SEQ ID NO:33 (PTH 1-52)
   SVSEIQLMHNLGKHLNSMERVEWLRKKLQDVHNFVALGAPLAPRDAGSQRPR
SEQ ID NO:34 (PTH 1-51)
   SVSEIQLMHNLGKHLNSMERVEWLRKKLQDVHNFVALGAPLAPRDAGSQRP
SEQ ID NO:35 (PTH 1-50)
   SVSEIQLMHNLGKHLNSMERVEWLRKKLQDVHNFVALGAPLAPRDAGSQR
SEQ ID NO:36 (PTH 1-49)
   SVSEIQLMHNLGKHLNSMERVEWLRKKLQDVHNFVALGAPLAPRDAGSQ
SEQ ID NO:37 (PTH 1-48)
   SVSEIQLMHNLGKHLNSMERVEWLRKKLQDVHNFVALGAPLAPRDAGS
SEQ ID NO:38 (PTH 1-47)
   SVSEIQLMHNLGKHLNSMERVEWLRKKLQDVHNFVALGAPLAPRDAG
SEQ ID NO:39 (PTH 1-46)
   SVSEIQLMHNLGKHLNSMERVEWLRKKLQDVHNFVALGAPLAPRDA
SEQ ID NO:40 (PTH 1-45)
   SVSEIQLMHNLGKHLNSMERVEWLRKKLQDVHNFVALGAPLAPRD
SEQ ID NO:41 (PTH 1-44)
   SVSEIQLMHNLGKHLNSMERVEWLRKKLQDVHNFVALGAPLAPR
SEQ ID NO:42 (PTH 1-43)
   SVSEIQLMHNLGKHLNSMERVEWLRKKLQDVHNFVALGAPLAP
SEQ ID NO:43 (PTH 1-42)
   SVSEIQLMHNLGKHLNSMERVEWLRKKLQDVHNFVALGAPLA
SEQ ID NO:44 (PTH 1-41)
   SVSEIQLMHNLGKHLNSMERVEWLRKKLQDVHNFVALGAPL
SEQ ID NO:45 (PTH 1-40)
   SVSEIQLMHNLGKHLNSMERVEWLRKKLQDVHNFVALGAP
SEQ ID NO:46 (PTH 1-39)
   SVSEIQLMHNLGKHLNSMERVEWLRKKLQDVHNFVALGA
SEQ ID NO:47 (PTH 1-38)
   SVSEIQLMHNLGKHLNSMERVEWLRKKLQDVHNFVALG
SEQ ID NO:48 (PTH 1-37)
   SVSEIQLMHNLGKHLNSMERVEWLRKKLQDVHNFVAL
SEQ ID NO:49 (PTH 1-36)
   SVSEIQLMHNLGKHLNSMERVEWLRKKLQDVHNFVA
SEQ ID NO:50 (PTH 1-35)
   SVSEIQLMHNLGKHLNSMERVEWLRKKLQDVHNFV
SEQ ID NO:51 (PTH 1-34)
   SVSEIQLMHNLGKHLNSMERVEWLRKKLQDVHNF
SEQ ID NO:52 (PTH 1-33)
   SVSEIQLMHNLGKHLNSMERVEWLRKKLQDVHN
SEQ ID NO:53 (PTH 1-32)
   SVSEIQLMHNLGKHLNSMERVEWLRKKLQDVH
SEQ ID NO:54 (PTH 1-31)
   SVSEIQLMHNLGKHLNSMERVEWLRKKLQDV
SEQ ID NO:55 (PTH 1-30)
   SVSEIQLMHNLGKHLNSMERVEWLRKKLQD
SEQ ID NO:56 (PTH 1-29)
   SVSEIQLMHNLGKHLNSMERVEWLRKKLQ
SEQ ID NO:57 (PTH 1-28)
   SVSEIQLMHNLGKHLNSMERVEWLRKKL
SEQ ID NO:58 (PTH 1-27)
   SVSEIQLMHNLGKHLNSMERVEWLRKK
SEQ ID NO:59 (PTH 1-26)
   SVSEIQLMHNLGKHLNSMERVEWLRK
SEQ ID NO:60 (PTH 1-25)
   SVSEIQLMHNLGKHLNSMERVEWLR
SEQ ID NO:61 (amidated PTH 1-84)
   SVSEIQLMHNLGKHLNSMERVEWLRKKLQDVHNFVALGAPLAPRDAGSQRPRKKE DNVLVESHEKSLGEADKADVNVLTKAKSQ; wherein the C-terminus is amidated
SEQ ID NO:62 (amidated PTH 1-83)
   SVSEIQLMHNLGKHLNSMERVEWLRKKLQDVHNFVALGAPLAPRDAGSQRPRKKE DNVLVESHEKSLGEADKADVNVLTKAKS; wherein the C-terminus is amidated
SEQ ID NO:63 (amidated PTH 1-82)
   SVSEIQLMHNLGKHLNSMERVEWLRKKLQDVHNFVALGAPLAPRDAGSQRPRKKE DNVLVESHEKSLGEADKADVNVLTKAK; wherein the C-terminus is amidated
SEQ ID NO:64 (amidated PTH 1-81)
   SVSEIQLMHNLGKHLNSMERVEWLRKKLQDVHNFVALGAPLAPRDAGSQRPRKKE DNVLVESHEKSLGEADKADVNVLTKA; wherein the C-terminus is amidated
SEQ ID NO:65 (amidated PTH 1-80)
   SVSEIQLMHNLGKHLNSMERVEWLRKKLQDVHNFVALGAPLAPRDAGSQRPRKKE DNVLVESHEKSLGEADKADVNVLTK; wherein the C-terminus is amidated
SEQ ID NO:66 (amidated PTH 1-79)
   SVSEIQLMHNLGKHLNSMERVEWLRKKLQDVHNFVALGAPLAPRDAGSQRPRKKE DNVLVESHEKSLGEADKADVNVLT; wherein the C-terminus is amidated
SEQ ID NO:67 (amidated PTH 1-78)
   SVSEIQLMHNLGKHLNSMERVEWLRKKLQDVHNFVALGAPLAPRDAGSQRPRKKE DNVLVESHEKSLGEADKADVNVL; wherein the C-terminus is amidated
SEQ ID NO:68 (amidated PTH 1-77)
   SVSEIQLMHNLGKHLNSMERVEWLRKKLQDVHNFVALGAPLAPRDAGSQRPRKKE DNVLVESHEKSLGEADKADVNV; wherein the C-terminus is amidated
SEQ ID NO:69 (amidated PTH 1-76)
   SVSEIQLMHNLGKHLNSMERVEWLRKKLQDVHNFVALGAPLAPRDAGSQRPRKKE DNVLVESHEKSLGEADKADVN; wherein the C-terminus is amidated
SEQ ID NO:70 (amidated PTH 1-75)
   SVSEIQLMHNLGKHLNSMERVEWLRKKLQDVHNFVALGAPLAPRDAGSQRPRKKE DNVLVESHEKSLGEADKADV; wherein the C-terminus is amidated
SEQ ID NO:71 (amidated PTH 1-74)
   SVSEIQLMHNLGKHLNSMERVEWLRKKLQDVHNFVALGAPLAPRDAGSQRPRKKE DNVLVESHEKSLGEADKAD; wherein the C-terminus is amidated
SEQ ID NO:72 (amidated PTH 1-73)
   SVSEIQLMHNLGKHLNSMERVEWLRKKLQDVHNFVALGAPLAPRDAGSQRPRKKE DNVLVESHEKSLGEADKA; wherein the C-terminus is amidated
SEQ ID NO:73 (amidated PTH 1-72)
   SVSEIQLMHNLGKHLNSMERVEWLRKKLQDVHNFVALGAPLAPRDAGSQRPRKKE DNVLVESHEKSLGEADK; wherein the C-terminus is amidated
SEQ ID NO:74 (amidated PTH 1-71)
   SVSEIQLMHNLGKHLNSMERVEWLRKKLQDVHNFVALGAPLAPRDAGSQRPRKKE DNVLVESHEKSLGEAD; wherein the C-terminus is amidated
SEQ ID NO:75 (amidated PTH 1-70)
   SVSEIQLMHNLGKHLNSMERVEWLRKKLQDVHNFVALGAPLAPRDAGSQRPRKKE DNVLVESHEKSLGEA; wherein the C-terminus is amidated
SEQ ID NO:76 (amidated PTH 1-69)
   SVSEIQLMHNLGKHLNSMERVEWLRKKLQDVHNFVALGAPLAPRDAGSQRPRKKE DNVLVESHEKSLGE; wherein the C-terminus is amidated
SEQ ID NO:77 (amidated PTH 1-68)
   SVSEIQLMHNLGKHLNSMERVEWLRKKLQDVHNFVALGAPLAPRDAGSQRPRKKE DNVLVESHEKSLG; wherein the C-terminus is amidated
SEQ ID NO:78 (amidated PTH 1-67)
   SVSEIQLMHNLGKHLNSMERVEWLRKKLQDVHNFVALGAPLAPRDAGSQRPRKKE DNVLVESHEKSL; wherein the C-terminus is amidated
SEQ ID NO:79 (amidated PTH 1-66)
   SVSEIQLMHNLGKHLNSMERVEWLRKKLQDVHNFVALGAPLAPRDAGSQRPRKKE DNVLVESHEKS; wherein the C-terminus is amidated
SEQ ID NO:80 (amidated PTH 1-65)
   SVSEIQLMHNLGKHLNSMERVEWLRKKLQDVHNFVALGAPLAPRDAGSQRPRKKE DNVLVESHEK; wherein the C-terminus is amidated
SEQ ID NO:81 (amidated PTH 1-64)
   SVSEIQLMHNLGKHLNSMERVEWLRKKLQDVHNFVALGAPLAPRDAGSQRPRKKE DNVLVESHE; wherein the C-terminus is amidated
SEQ ID NO:82 (amidated PTH 1-63)
   SVSEIQLMHNLGKHLNSMERVEWLRKKLQDVHNFVALGAPLAPRDAGSQRPRKKE DNVLVESH; wherein the C-terminus is amidated
SEQ ID NO:83 (amidated PTH 1-62)
   SVSEIQLMHNLGKHLNSMERVEWLRKKLQDVHNFVALGAPLAPRDAGSQRPRKKE DNVLVES; wherein the C-terminus is amidated
SEQ ID NO:84 (amidated PTH 1-61)
   SVSEIQLMHNLGKHLNSMERVEWLRKKLQDVHNFVALGAPLAPRDAGSQRPRKKE DNVLVE; wherein the C-terminus is amidated
SEQ ID NO:85 (amidated PTH 1-60)
   SVSEIQLMHNLGKHLNSMERVEWLRKKLQDVHNFVALGAPLAPRDAGSQRPRKKE DNVLV; wherein the C-terminus is amidated
SEQ ID NO:86 (amidated PTH 1-59)
   SVSEIQLMHNLGKHLNSMERVEWLRKKLQDVHNFVALGAPLAPRDAGSQRPRKKE DNVL; wherein the C-terminus is amidated
SEQ ID NO:87 (amidated PTH 1-58)
   SVSEIQLMHNLGKHLNSMERVEWLRKKLQDVHNFVALGAPLAPRDAGSQRPRKKE DNV; wherein the C-terminus is amidated
SEQ ID NO:88 (amidated PTH 1-57)
   SVSEIQLMHNLGKHLNSMERVEWLRKKLQDVHNFVALGAPLAPRDAGSQRPRKKE DN; wherein the C-terminus is amidated
SEQ ID NO:89 (amidated PTH 1-56)
   SVSEIQLMHNLGKHLNSMERVEWLRKKLQDVHNFVALGAPLAPRDAGSQRPRKKE D; wherein the C-terminus is amidated
SEQ ID NO:90 (amidated PTH 1-55)
   SVSEIQLMHNLGKHLNSMERVEWLRKKLQDVHNFVALGAPLAPRDAGSQRPRKKE; wherein the C-terminus is amidated
SEQ ID NO:91 (amidated PTH 1-54)
   SVSEIQLMHNLGKHLNSMERVEWLRKKLQDVHNFVALGAPLAPRDAGSQRPRKK; wherein the C-terminus is amidated
SEQ ID NO:92 (amidated PTH 1-53)
   SVSEIQLMHNLGKHLNSMERVEWLRKKLQDVHNFVALGAPLAPRDAGSQRPRK; wherein the C-terminus is amidated
SEQ ID NO:93 (amidated PTH 1-52)
   SVSEIQLMHNLGKHLNSMERVEWLRKKLQDVHNFVALGAPLAPRDAGSQRPR; wherein the C-terminus is amidated
SEQ ID NO:94 (amidated PTH 1-51)
   SVSEIQLMHNLGKHLNSMERVEWLRKKLQDVHNFVALGAPLAPRDAGSQRP; wherein the C-terminus is amidated
SEQ ID NO:95 (amidated PTH 1-50)
   SVSEIQLMHNLGKHLNSMERVEWLRKKLQDVHNFVALGAPLAPRDAGSQR; wherein the C-terminus is amidated
SEQ ID NO:96 (amidated PTH 1-49)
   SVSEIQLMHNLGKHLNSMERVEWLRKKLQDVHNFVALGAPLAPRDAGSQ; wherein the C-terminus is amidated
SEQ ID NO:97 (amidated PTH 1-48)
   SVSEIQLMHNLGKHLNSMERVEWLRKKLQDVHNFVALGAPLAPRDAGS; wherein the C-terminus is amidated
SEQ ID NO:98 (amidated PTH 1-47)
   SVSEIQLMHNLGKHLNSMERVEWLRKKLQDVHNFVALGAPLAPRDAG; wherein the C-terminus is amidated
SEQ ID NO:99 (amidated PTH 1-46)
   SVSEIQLMHNLGKHLNSMERVEWLRKKLQDVHNFVALGAPLAPRDA; wherein the C-terminus is amidated
SEQ ID NO:100 (amidated PTH 1-45)
   SVSEIQLMHNLGKHLNSMERVEWLRKKLQDVHNFVALGAPLAPRD; wherein the C-terminus is amidated
SEQ ID NO:101 (amidated PTH 1-44)
   SVSEIQLMHNLGKHLNSMERVEWLRKKLQDVHNFVALGAPLAPR; wherein the C-terminus is amidated
SEQ ID NO:102 (amidated PTH 1-43)
   SVSEIQLMHNLGKHLNSMERVEWLRKKLQDVHNFVALGAPLAP; wherein the C-terminus is amidated
SEQ ID NO:103 (amidated PTH 1-42)
   SVSEIQLMHNLGKHLNSMERVEWLRKKLQDVHNFVALGAPLA; wherein the C-terminus is amidated
SEQ ID NO:104 (amidated PTH 1-41)
   SVSEIQLMHNLGKHLNSMERVEWLRKKLQDVHNFVALGAPL; wherein the C-terminus is amidated
SEQ ID NO:105 (amidated PTH 1-40)
   SVSEIQLMHNLGKHLNSMERVEWLRKKLQDVHNFVALGAP; wherein the C-terminus is amidated
SEQ ID NO:106 (amidated PTH 1-39)
   SVSEIQLMHNLGKHLNSMERVEWLRKKLQDVHNFVALGA; wherein the C-terminus is amidated
SEQ ID NO:107 (amidated PTH 1-38)
   SVSEIQLMHNLGKHLNSMERVEWLRKKLQDVHNFVALG; wherein the C-terminus is amidated
SEQ ID NO:108 (amidated PTH 1-37)
   SVSEIQLMHNLGKHLNSMERVEWLRKKLQDVHNFVAL; wherein the C-terminus is amidated
SEQ ID NO:109 (amidated PTH 1-36)
   SVSEIQLMHNLGKHLNSMERVEWLRKKLQDVHNFVA; wherein the C-terminus is amidated
SEQ ID NO:110 (amidated PTH 1-35)
   SVSEIQLMHNLGKHLNSMERVEWLRKKLQDVHNFV; wherein the C-terminus is amidated
SEQ ID NO:111 (amidated PTH 1-34)
   SVSEIQLMHNLGKHLNSMERVEWLRKKLQDVHNF; wherein the C-terminus is amidated
SEQ ID NO:112 (amidated PTH 1-33)
   SVSEIQLMHNLGKHLNSMERVEWLRKKLQDVHN; wherein the C-terminus is amidated
SEQ ID NO:113 (amidated PTH 1-32)
   SVSEIQLMHNLGKHLNSMERVEWLRKKLQDVH; wherein the C-terminus is amidated
SEQ ID NO:114 (amidated PTH 1-31)
   SVSEIQLMHNLGKHLNSMERVEWLRKKLQDV; wherein the C-terminus is amidated
SEQ ID NO:115 (amidated PTH 1-30)
   SVSEIQLMHNLGKHLNSMERVEWLRKKLQD; wherein the C-terminus is amidated
SEQ ID NO:116 (amidated PTH 1-29)
   SVSEIQLMHNLGKHLNSMERVEWLRKKLQ; wherein the C-terminus is amidated
SEQ ID NO:117 (amidated PTH 1-28)
   SVSEIQLMHNLGKHLNSMERVEWLRKKL; wherein the C-terminus is amidated
SEQ ID NO:118 (amidated PTH 1-27)
   SVSEIQLMHNLGKHLNSMERVEWLRKK; wherein the C-terminus is amidated
SEQ ID NO:119 (amidated PTH 1-26)
   SVSEIQLMHNLGKHLNSMERVEWLRK; wherein the C-terminus is amidated
SEQ ID NO:120 (amidated PTH 1-25)
   SVSEIQLMHNLGKHLNSMERVEWLR; wherein the C-terminus is amidated
SEQ ID NO:121 (PTHrP)

AVSEHQLLHDKGKSIQDLRRRFFLHHLIAEIHTAEIRATSEVSPNSKPSPNTKNHPVRF
GSDDEGRYLTQETNKVETYKEQPLKTPGKKKKGKPGKRKEQEKKKRRTRSAWLDS
GVTGSGLEGDHLSDTSTTSLELDSRRH

More preferably, the term "PTH" refers to a sequence selected from the group consisting of SEQ ID No:47, 48, 49, 50, 51, 52, 53, 54, 55, 107, 108, 109, 110, 111, 112, 113, 114 and 115. Even more preferably, the term "PTH" refers to a sequence selected from the group consisting of SEQ ID NO:50, 51, 52, 110, 111 and 112. In a particularly preferred embodiment the term "PTH" refers to the sequence of SEQ ID NO:51.

As used herein, the term "PTH polypeptide variant" refers to a polypeptide from the same species that differs from a reference PTH or PTHrP polypeptide. Preferably, such reference is a PTH polypeptide sequence and has the sequence of SEQ ID NO:51. Generally, differences are limited so that the amino acid sequence of the reference and the variant are closely similar overall and, in many regions, identical. Preferably, PTH polypeptide variants are at least 70%, 80%, 90%, or 95% identical to a reference PTH or PTHrP polypeptide, preferably to the PTH polypeptide of SEQ ID NO:51. By a polypeptide having an amino acid sequence at least, for example, 95% "identical" to a query amino acid sequence, it is intended that the amino acid sequence of the subject polypeptide is identical to the query sequence except that the subject polypeptide sequence may include up to five amino acid alterations per each 100 amino acids of the query amino acid sequence. These alterations of the reference sequence may occur at the amino (N-terminal) or carboxy terminal (C-terminal) positions of the reference amino acid sequence or anywhere between those terminal positions, interspersed either individually among residues in the reference sequence or in one or more contiguous groups within the reference sequence. The query sequence may be an entire amino acid sequence of the reference sequence or any fragment specified as described herein. Preferably, the query sequence is the sequence of SEQ ID NO:51.

Such PTH polypeptide variants may be naturally occurring variants, such as naturally occurring allelic variants encoded by one of several alternate forms of a PTH or PTHrP occupying a given locus on a chromosome or an organism, or isoforms encoded by naturally occurring splice variants originating from a single primary transcript. Alternatively, a PTH polypeptide variant may be a variant that is not known to occur naturally and that can be made by mutagenesis techniques known in the art.

It is known in the art that one or more amino acids may be deleted from the N-terminus or C-terminus of a bioactive polypeptide without substantial loss of biological function. Such N- and/or C-terminal deletions are also encompassed by the term PTH polypeptide variant.

It is also recognized by one of ordinary skill in the art that some amino acid sequences of PTH or PTHrP polypeptides can be varied without significant effect of the structure or function of the polypeptide. Such mutants include deletions, insertions, inversions, repeats, and substitutions selected according to general rules known in the art so as to have little effect on activity. For example, guidance concerning how to make phenotypically silent amino acid substitutions is provided in Bowie et al. (1990), Science 247:1306-1310, which is hereby incorporated by reference in its entirety, wherein the authors indicate that there are two main approaches for studying the tolerance of the amino acid sequence to change.

The term PTH polypeptide also encompasses all PTH and PTHrP polypeptides encoded by PTH and PTHrP analogs, orthologs, and/or species homologs. It is also recognized by one of ordinary skill in the art that PTHrP and PTHrP analogs bind to activate the common PTH/PTHrP1 receptor, so the term PTH polypeptide also encompasses all PTHrP analogs. As used herein, the term "PTH analog" refers to PTH and PTHrP of different and unrelated organisms which perform the same functions in each organism but which did not originate from an ancestral structure that the organisms' ancestors had in common. Instead, analogous PTH and PTHrP arose separately and then later evolved to perform the same or similar functions. In other words, analogous PTH and PTHrP polypeptides are polypeptides with quite different amino acid sequences but that perform the same biological activity, namely raising serum calcium and renal phosphorus excretion, and lowering serum phosphorus and renal calcium excretion.

As used herein the term "PTH ortholog" refers to PTH and PTHrP within two different species which sequences are related to each other via a common homologous PTH or PTHrP in an ancestral species, but which have evolved to become different from each other.

As used herein, the term "PTH homolog" refers to PTH and PTHrP of different organisms which perform the same functions in each organism and which originate from an ancestral structure that the organisms' ancestors had in common. In other words, homologous PTH polypeptides are polypeptides with quite similar amino acid sequences that perform the same biological activity, namely raising serum calcium and renal phosphorus excretion, and lowering serum phosphorus and renal calcium excretion. Preferably, PTH polypeptide homologs may be defined as polypeptides exhibiting at least 40%, 50%, 60%, 70%, 80%, 90% or 95% identity to a reference PTH or PTHrP polypeptide, preferably the PTH polypeptide of SEQ ID NO:51.

Thus, a PTH polypeptide according to the invention may be, for example: (i) one in which at least one of the amino acids residues is substituted with a conserved or non-conserved amino acid residue, preferably a conserved amino acid residue, and such substituted amino acid residue may or may not be one encoded by the genetic code; and/or (ii) one in which at least one of the amino acid residues includes a substituent group; and/or (iii) one in which the PTH polypeptide is fused with another compound, such as a compound to increase the half-life of the polypeptide (for example, polyethylene glycol); and/or (iv) one in which additional amino acids are fused to the PTH polypeptide, such as an IgG Fc fusion region polypeptide or leader or secretory sequence or a sequence which is employed for purification of the above form of the polypeptide or a pre-protein sequence.

As used herein, the term "PTH polypeptide fragment" refers to any polypeptide comprising a contiguous span of a part of the amino acid sequence of a PTH or PTHrP polypeptide, preferably the polypeptide of SEQ ID NO:51.

More specifically, a PTH polypeptide fragment comprises at least 6, such as at least 8, at least 10 or at least 17 consecutive amino acids of a PTH or PTHrP polypeptide, more preferably of the polypeptide of SEQ ID NO:51. A PTH polypeptide fragment may additionally be described as sub-genuses of PTH or PTHrP polypeptides comprising at least 6 amino acids, wherein "at least 6" is defined as any integer between 6 and the integer representing the C-terminal amino acid of a PTH or PTHrP polypeptide, preferably of the polypeptide of SEQ ID No:51. Further included are species of PTH or PTHrP polypeptide fragments at least 6 amino acids in length, as described above, that are further specified in terms of their N-terminal and C-terminal positions. Also encompassed by the term "PTH polypeptide fragment" as individual species are all PTH or PTHrP polypeptide fragments, at least 6 amino acids in length, as described above, that may be particularly specified by a N-terminal and C-terminal position. That is, every combination of a N-terminal and C-terminal position that a fragment at least 6 contiguous amino acid residues in length could occupy, on any given amino acid sequence of a PTH or PTHrP polypeptide, preferably the PTH polypeptide of SEQ ID:NO51, is included in the present invention.

The term "PTH" also includes poly(amino acid) conjugates which have a sequence as described above, but having a backbone that comprises both amide and non-amide linkages, such as ester linkages, like for example depsipeptides. Depsipeptides are chains of amino acid residues in which the backbone comprises both amide (peptide) and ester bonds. Accordingly, the term "side chain" as used herein refers either to the moiety attached to the alpha-carbon of an amino acid moiety, if the amino acid moiety is connected through amine bonds such as in polypeptides, or to any carbon atom-comprising moiety attached to the backbone of a poly(amino acid) conjugate, such as for example in the case of depsipeptides.

Preferably, the term "PTH" refers to polypeptides having a backbone formed through amide (peptide) bonds.

As the term PTH includes the above-described variants, analogs, orthologs, homologs, derivatives and fragments of PTH and PTHrP, all references to specific positions within a reference sequence also include the equivalent positions in variants, analogs, orthologs, homologs, derivatives and fragments of a PTH or PTHrP moiety, even if not specifically mentioned.

As used herein, the term "random coil" refers to a peptide or protein adopting/having/forming, preferably having, a conformation which substantially lacks a defined secondary and tertiary structure as determined by circular dichroism spectroscopy performed in aqueous buffer at ambient temperature, and pH 7.4. Preferably, ambient temperature is about 20°C, i.e. between 18°C and 22°C, most preferably ambient temperature is 20°C.

As used herein the term "pharmaceutical composition" refers to a composition containing one or more active ingredients, for example a drug or a conjugate, here specifically the PTH conjugate of the present invention, and optionally one or more excipients, as well as any product which results, directly or indirectly, from combination, complexation or aggregation of any two or more of the ingredients of the composition, or from dissociation of one or more of the ingredients, or from other types of reactions or interactions of one or more of the ingredients. Accordingly, the pharmaceutical compositions of the present invention encompass any composition made by admixing one or more PTH conjugates of the present invention and optionally a pharmaceutically acceptable excipient.

As used herein the term "liquid composition" refers to a mixture comprising water-soluble PTH conjugate and one or more solvents, such as water.

As used herein, the term "dry composition" means that a pharmaceutical composition is provided in a dry form. Suitable methods for drying are spray-drying and lyophilization, i.e. freeze-drying. Such dry composition of for example the PTH conjugate of the present invention has a residual water content of a maximum of 10%, preferably less than 5% and more preferably less than 2%, determined according to Karl Fischer. Preferably, the pharmaceutical composition of the present invention is dried by lyophilization.

The term "drug" as used herein refers to a substance used in the treatment, cure, prevention, or diagnosis of a disease or used to otherwise enhance physical or mental well-being. If a drug is conjugated to another moiety, the moiety of the resulting product that originated from the drug is referred to as "biologically active moiety". The PTH conjugate of the present invention comprise a PTH moiety, which is released from the PTH conjugate in the form of the drug PTH.

It is understood that the PTH conjugates of the present invention are PTH produgs. As used herein the term "prodrug" refers to a conjugate comprising a biologically active moiety reversibly and covalently connected to a specialized protective group through a reversible linker moiety, also called prodrug linker moiety, which is a linker moiety comprising a reversible linkage with the biologically active moiety and wherein the specialized protective group alters or eliminates undesirable properties in the parent molecule. This also includes the enhancement of desirable properties in the drug and the suppression of undesirable properties. The specialized non-toxic protective group is referred to as "carrier". A prodrug releases the reversibly and covalently bound biologically active moiety in the form of its corresponding drug. In other words, a prodrug is a conjugate comprising a biologically active moiety, which is covalently and reversibly conjugated to a carrier moiety via a reversible linker moiety, which covalent and reversible conjugation of the carrier to the reversible linker moiety is either directly or through a spacer. Such conjugate releases the formerly conjugated biologically active moiety in its free form.

A "biodegradable linkage" or a "reversible linkage" is a linkage that is degradable, i.e. cleavable, for example by hydrolysis, in the absence of enzymes under physiological conditions (aqueous buffer at pH 7.4, 37°C) with a half-life ranging from one hour to two months, preferably from three hours to one month, even more preferably from 6 hours to two weeks. Accordingly, a stable linkage is a linkage having a half-life under physiological conditions (aqueous buffer at pH 7.4, 37°C) of more than two months.

Accordingly, a "reversible prodrug linker moiety" or "reversible linker moiety" is a moiety, which is covalently conjugated to a biologically active moiety, such as PTH, through a reversible linkage and is covalently conjugated to a carrier moiety, such as -Z, wherein the covalent conjugation to said carrier moiety is either directly or through a spacer moiety, such as -L²-. Preferably the linkage between -Z and -L²- is a stable linkage.

As used herein, the term "traceless prodrug linker" means a reversible prodrug linker, which upon cleavage releases the drug in its free form. As used herein, the term "free form" of a drug means the drug in its unmodified, pharmacologically active form.

As used herein, the term "excipient" refers to a diluent, adjuvant, or vehicle with which the therapeutic, such as a drug or the PTH conjugate of the present invention, is administered. Such pharmaceutical excipient can be sterile liquids, such as water and oils, including those of petroleum, animal, vegetable or synthetic origin, including but not limited to peanut oil, soybean oil, mineral oil, sesame oil and the like. Water is a preferred excipient when the pharmaceutical composition is administered orally. Saline and aqueous dextrose are preferred excipients when the pharmaceutical composition is administered intravenously. Saline solutions and aqueous dextrose and glycerol solutions are preferably employed as liquid excipients for injectable solutions. Suitable pharmaceutical excipients include starch, glucose, lactose, sucrose, mannitol, trehalose, gelatin, malt, rice, flour, chalk, silica gel, sodium stearate, glycerol monostearate, talc, sodium chloride, dried skim milk, glycerol, propylene, glycol, water, ethanol and the like. The pharmaceutical composition, if desired, can also contain minor amounts of wetting or emulsifying agents, pH buffering agents, like, for example, acetate, succinate, tris, carbonate, phosphate, HEPES (4-(2-hydroxyethyl)-1-piperazineethanesulfonic acid), MES (2-(N-morpholino)ethanesulfonic acid), or can contain detergents, like Tween, poloxamers, poloxamines, CHAPS, Igepal, or amino acids like, for example, glycine, lysine, or histidine. These pharmaceutical compositions can take the form of solutions, suspensions, emulsions, tablets, pills, capsules, powders, sustained-release formulations and the like. The pharmaceutical composition can be formulated as a suppository, with traditional binders and excipients such as triglycerides. Oral formulation can include standard excipients such as pharmaceutical grades of mannitol, lactose, starch, magnesium stearate, sodium saccharine, cellulose, magnesium carbonate, etc. Such compositions will contain a therapeutically effective amount of the drug or biologically active moiety, together with a suitable amount of excipient to provide the form for proper administration to the patient. The formulation should suit the mode of administration.

As used herein, the term "reagent" means a chemical compound, which comprises at least one functional group for reaction with the functional group of another chemical compound or drug. It is understood that a drug comprising a functional group (such as a primary or secondary amine or hydroxyl functional group) is also a reagent.

As used herein, the term "moiety" means a part of a molecule, which lacks one or more atom(s) compared to the corresponding reagent. If, for example, a reagent of the formula "H-X-H" reacts with another reagent and becomes part of the reaction product, the corresponding moiety of the reaction product has the structure "H-X-" or "-X-", whereas each "-" indicates attachment to another moiety. Accordingly, a biologically active moiety is released from the conjugates of the present invention as a drug.

It is understood that if the sequence or chemical structure of a group of atoms is provided which group of atoms is attached to two moieties or is interrupting a moiety, said sequence or chemical structure can be attached to the two moieties in either orientation, unless explicitly stated otherwise. For example, a moiety "-C(O)N(R)-" can be attached to two moieties or interrupting a moiety either as "-C(O)N(R)-" or as "-N(R)C(O)-". Similarly, a moiety 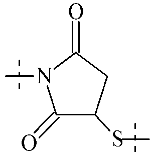 can be attached to two moieties or can interrupt a moiety either as

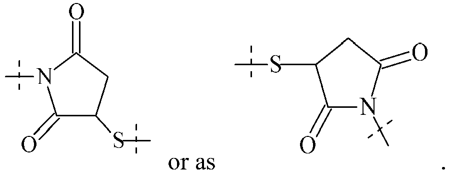

As used herein, the term "functional group" means a group of atoms, which can react with other groups of atoms. Functional groups include but are not limited to the following groups: carboxylic acid (-(C=O)OH), primary or secondary amine (-NH₂, -NH-), maleimide, thiol (-SH), sulfonic acid (-(O=S=O)OH), carbonate, carbamate (-O(C=O)N<), hydroxyl (-OH), aldehyde (-(C=O)H), ketone (-(C=O)-), hydrazine (>N-N<), isocyanate, isothiocyanate, phosphoric acid (-O(P=O)OHOH), phosphonic acid (-O(P=O)OHH), haloacetyl, alkyl halide, acryloyl, aryl fluoride, hydroxylamine, disulfide, sulfonamides, sulfuric acid, vinyl sulfone, vinyl ketone, diazoalkane, oxirane, and aziridine.

In case the conjugates of the present invention comprise one or more acidic or basic groups, the invention also comprises their corresponding pharmaceutically or toxicologically acceptable salts, in particular their pharmaceutically utilizable salts. Thus, the conjugates of the present invention comprising acidic groups can be used according to the invention, for example, as alkali metal salts, alkaline earth metal salts or as ammonium salts. More precise examples of such salts include sodium salts, potassium salts, calcium salts, magnesium salts or salts with ammonia or organic amines such as, for example, ethylamine, ethanolamine, triethanolamine or amino acids. Conjugates of the present invention comprising one or more basic groups, i.e. groups which can be protonated, can be present and can be used according to the invention in the form of their addition salts with inorganic or organic acids. Examples for suitable acids include hydrogen chloride, hydrogen bromide, phosphoric acid, sulfuric acid, nitric acid, methanesulfonic acid, p-toluenesulfonic acid, naphthalenedisulfonic acids, oxalic acid, acetic acid, tartaric acid, lactic acid, salicylic acid, benzoic acid, formic acid, propionic acid, pivalic acid, diethylacetic acid, malonic acid, succinic acid, pimelic acid, fumaric acid, maleic acid, malic acid, sulfaminic acid, phenylpropionic acid, gluconic acid, ascorbic acid, isonicotinic acid, citric acid, adipic acid, and other acids known to the person skilled in the art. For the person skilled in the art further methods are known for converting the basic group into a cation like the alkylation of an amine group resulting in a positively-charge ammonium group and an appropriate counterion of the salt. If the conjugates of the present invention simultaneously comprise acidic and basic groups, the invention also includes, in addition to the salt forms mentioned, inner salts or betaines (zwitterions). The respective salts can be obtained by customary methods, which are known to the person skilled in the art like, for example by contacting these conjugates with an organic or inorganic acid or base in a solvent or dispersant, or by anion exchange or cation exchange with other salts. The present invention also includes all salts of the conjugates of the present invention which, owing to low physiological compatibility, are not directly suitable for use in pharmaceuticals but which can be used, for example, as intermediates for chemical reactions or for the preparation of pharmaceutically acceptable salts.

The term "pharmaceutically acceptable" means a substance that does not cause harm when administered to a patient and preferably means approved by a regulatory agency, such as the EMA (Europe) and/or the FDA (US) and/or any other national regulatory agency for use in animals, preferably for use in humans.

As used herein the term "about" in combination with a numerical value is used to indicate a range ranging from and including the numerical value plus and minus no more than 20% of said numerical value, more preferably no more than 10% of said numerical value, even more preferably no more than 5% of said numerical value and most preferably no more than 2% of said numerical value. For example, the phrase "about 200" is used to mean a range ranging from and including 200 +/- 20%, i.e. ranging from and including 160 to 240; preferably 200 +/- 10%, i.e. ranging from and including 180 to 220; even more preferably ranging from and including 200 +/-5%, i.e. ranging from and including 190 to 210; and most preferably 200 +/-2%, i.e. ranging from and including 196 to 204. It is understood that a percentage given as "about 20%" does not mean "20% +/- 10%", i.e. ranging from and including 10 to 30%, but "about 20%" means ranging from and including 18 to 22%, i.e. plus and minus 10% of the numerical value which is 20.

As used herein, the term "polymer" means a molecule comprising repeating structural units, i.e. the monomers, connected by chemical bonds in a linear, circular, branched, crosslinked or dendrimeric way or a combination thereof, which may be of synthetic or biological origin or a combination of both. It is understood that a polymer may also comprise one or more other chemical group(s) and/or moiety/moieties, such as, for example, one or more functional group(s). Preferably, a soluble polymer has a molecular weight of at least 0.5 kDa, e.g. a molecular weight of at least 1 kDa, a molecular weight of at least 2 kDa, a molecular weight of at least 3 kDa or a molecular weight of at least 5 kDa. If the polymer is soluble, it preferable has a molecular weight of at most 1000 kDa, such as at most 750 kDa, such as at most 500 kDa, such as at most 300 kDa, such as at most 200 kDa, such as at most 100 kDa.

As used herein, the term "polymeric" means a reagent or a moiety comprising one or more polymer(s) or polymer moiety/moieties. A polymeric reagent or moiety may optionally also comprise one or more other moiety/moieties, which are preferably selected from the group consisting of:
- C₁₋₅₀ alkyl, C₂₋₅₀ alkenyl, C₂₋₅₀ alkynyl, C₃₋₁₀ cycloalkyl, 3- to 10-membered heterocyclyl, 8- to 11-membered heterobicyclyl, phenyl, naphthyl, indenyl, indanyl, and tetralinyl; and
- linkages selected from the group comprising 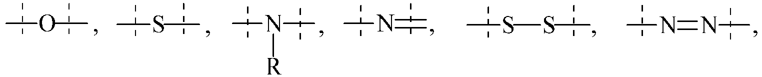 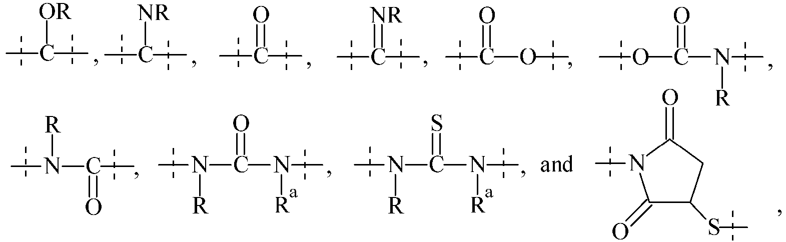 wherein
   dashed lines indicate attachment to the remainder of the moiety or reagent, and
   -R and -R^{a} are independently of each other selected from the group consisting of -H, methyl, ethyl, propyl, butyl, pentyl and hexyl.

The person skilled in the art understands that the polymerization products obtained from a polymerization reaction do not all have the same molecular weight, but rather exhibit a molecular weight distribution. Consequently, the molecular weight ranges, molecular weights, ranges of numbers of monomers in a polymer and numbers of monomers in a polymer as used herein, refer to the number average molecular weight and number average of monomers, i.e. to the arithmetic mean of the molecular weight of the polymer or polymeric moiety and the arithmetic mean of the number of monomers of the polymer or polymeric moiety.

Accordingly, in a polymeric moiety comprising "x" monomer units any integer given for "x" therefore corresponds to the arithmetic mean number of monomers. Any range of integers given for "x" provides the range of integers in which the arithmetic mean numbers of monomers lies. An integer for "x" given as "about x" means that the arithmetic mean numbers of monomers lies in a range of integers of x +/- 20%, preferably x +/- 10%, more preferably x +/- 5% and most preferably x +/- 2%.

As used herein, the term "number average molecular weight" means the ordinary arithmetic mean of the molecular weights of the individual polymers.

As used herein the term "water-soluble" with reference to a carrier means that when such carrier is part of the PTH conjugates of the present invention at least 1 g of the PTH conjugate comprising such water-soluble carrier can be dissolved in one liter of water at 20°C to form a homogeneous solution. Accordingly, the term "water-insoluble" with reference to a carrier means that when such carrier is part of the PTH conjugate of the present invention less than 1 g of the PTH conjugate comprising such water-insoluble carrier can be dissolved in one liter of water at 20°C to form a homogeneous solution.

As used herein, the term "PEG-based" in relation to a moiety or reagent means that said moiety or reagent comprises PEG. Preferably, a PEG-based moiety or reagent comprises at least 10% (w/w) PEG, such as at least 20% (w/w) PEG, such as at least 30% (w/w) PEG, such as at least 40% (w/w) PEG, such as at least 50% (w/w), such as at least 60 (w/w) PEG, such as at least 70% (w/w) PEG, such as at least 80% (w/w) PEG, such as at least 90% (w/w) PEG, such as at least 95%. The remaining weight percentage of the PEG-based moiety or reagent are other moieties preferably selected from the following moieties and linkages:
- C₁₋₅₀ alkyl, C₂₋₅₀ alkenyl, C₂₋₅₀ alkynyl, C₃₋₁₀ cycloalkyl, 3- to 10-membered heterocyclyl, 8- to 11-membered heterobicyclyl, phenyl, naphthyl, indenyl, indanyl, and tetralinyl; and
- linkages selected from the group comprising 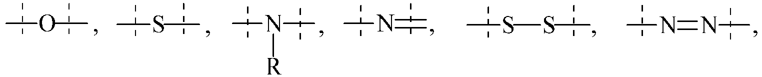 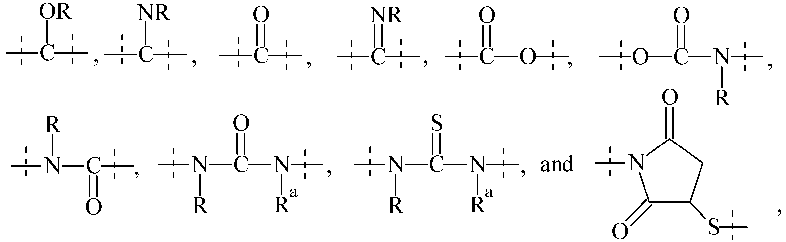 wherein
   dashed lines indicate attachment to the remainder of the moiety or reagent, and
   -R and -R^{a} are independently of each other selected from the group consisting of -H, methyl, ethyl, propyl, butyl, pentyl and hexyl.

As used herein, the term "PEG-based comprising at least X% PEG" in relation to a moiety or reagent means that said moiety or reagent comprises at least X% (w/w) ethylene glycol units (-CH₂CH₂O-), wherein the ethylene glycol units may be arranged blockwise, alternating or may be randomly distributed within the moiety or reagent and preferably all ethylene glycol units of said moiety or reagent are present in one block; the remaining weight percentage of the PEG-based moiety or reagent are other moieties preferably selected from the following moieties and linkages:
- C₁₋₅₀ alkyl, C₂₋₅₀ alkenyl, C₂₋₅₀ alkynyl, C₃₋₁₀ cycloalkyl, 3- to 10-membered heterocyclyl, 8- to 11-membered heterobicyclyl, phenyl, naphthyl, indenyl, indanyl, and tetralinyl; and
- linkages selected from the group comprising 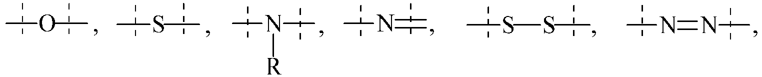 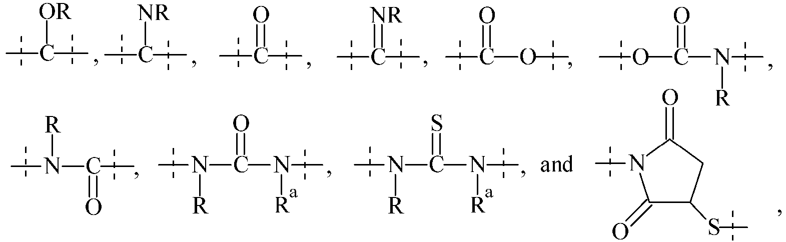 wherein
   dashed lines indicate attachment to the remainder of the moiety or reagent, and
   -R and -R^{a} are independently of each other selected from the group consisting of -H, methyl, ethyl, propyl, butyl, pentyl and hexyl.

The term "hyaluronic acid-based comprising at least X% hyaluronic acid" is used accordingly.

The term "substituted" as used herein means that one or more -H atom(s) of a molecule or moiety are replaced by a different atom or a group of atoms, which are referred to as "substituent".

Preferably, the one or more further optional substituents are independently of each other selected from the group consisting of halogen, -CN, -COOR^{x1}, -OR^{x1}, -C(O)R^{x1}, -C(O)N(R^{x1}R^{x1a}), -S(O)₂N(R^{x1}R^{x1a}), -S(O)N(R^{x1}R^{x1a}), -S(O)₂R^{x1}, -S(O)R^{x1}, -N(R^{x1})S(O)₂N(R^{x1a}R^{x1b}), -SR^{x1}, -N(R^{x1}R^{x1a}), -NO₂, -OC(O)R^{x1}, -N(R^{x1})C(O)R^{x1a}, -N(R^{x1})S(O)₂R^{x1a}, -N(R^{x1})S(O)R^{x1a}, -N(R^{x1})C(O)OR^{x1a}, -N(R^{x1})C(O)N(R^{x1a}R^{x1b}), -OC(O)N(R^{x1}R^{x1a}), -T⁰, C₁₋₅₀ alkyl, C₂₋₅₀ alkenyl, and C₂₋₅₀ alkynyl; wherein -T⁰, C₁₋₅₀ alkyl, C₂₋₅₀ alkenyl, and C₂₋₅₀ alkynyl are optionally substituted with one or more -R^{x2}, which are the same or different and wherein C₁₋₅₀ alkyl, C₂₋₅₀ alkenyl, and C₂₋₅₀ alkynyl are optionally interrupted by one or more groups selected from the group consisting of -T⁰-, -C(O)O-, -O-, -C(O)-, -C(O)N(R^{x3})-, -S(O)₂N(R^{x3})-, -S(O)N(R^{x3})-, -S(O)₂-, -S(O)-, -N(R^{x3})S(O)₂N(R^{x1a})-, -S-, -N(R^{x3})-, -OC(OR^{x3})(R^{x3a})-, -N(R^{x3})C(O)N(R^{x3a})-, and -OC(O)N(R^{x3})-;
-R^{x1}, -R^{x1a}, -R^{x1b} are independently of each other selected from the group consisting of -H, -T⁰, C₁₋₅₀ alkyl, C₂₋₅₀ alkenyl, and C₂₋₅₀ alkynyl; wherein -T⁰, C₁₋₅₀ alkyl, C₂₋₅₀ alkenyl, and C₂₋₅₀ alkynyl are optionally substituted with one or more -R^{x2}, which are the same or different and wherein C₁₋₅₀ alkyl, C₂₋₅₀ alkenyl, and C₂₋₅₀ alkynyl are optionally interrupted by one or more groups selected from the group consisting of -T⁰-, -C(O)O-, -O-, -C(O)-, -C(O)N(R^{x3})-, -S(O)₂N(R^{x3})-, -S(O)N(R^{x3})-; -S(O)₂-, -S(O)-, -N(R^{x3})S(O)₂N(R^{x3a})-, -S-, -N(R^{x3})-, -OC(OR^{x3})(R^{x3a})-, -N(R^{x3})C(O)N(R^{x3a})-, and -OC(O)N(R^{x3})-;
each T⁰ is independently selected from the group consisting of phenyl, naphthyl, indenyl, indanyl, tetralinyl, C₃₋₁₀ cycloalkyl, 3- to 10-membered heterocyclyl, and 8- to 11-membered heterobicyclyl; wherein each T⁰ is independently optionally substituted with one or more -R^{x2}, which are the same or different;
each -R^{x2} is independently selected from the group consisting of halogen, -CN, oxo (=O), -COOR^{x4}, -OR^{x4}, -C(O)R^{x4}, -C(O)N(R^{x4}R^{x4a}), -S(O)₂N(R^{x4}R^{x4a}), -S(O)N(R^{x4}R^{x4a}), -S(O)₂R^{x4}, -S(O)R^{x4}, -N(R^{x4})S(O)₂N(R^{x4a}R^{x4b}), -SR^{x4}, -N(R^{x4}R^{x4a}), -NO₂, -OC(O)R^{x4} -N(R^{x4})C(O)R^{x4a}, -N(R^{x4})S(O)₂R^{x4a}, -N(R^{x4})S(O)R^{x4a}, -N(R^{x4})C(O)OR^{x4a}, -N(R^{x4})C(O)N(R^{x4a}R^{x4b}), -OC(O)N(R^{x4}R^{x4a}), and C₁₋₆ alkyl; wherein C₁₋₆ alkyl is optionally substituted with one or more halogen, which are the same or different;
each -R^{x3}, -R^{x3a}, -R^{x4}, -R^{x4a}, -R^{x4b} is independently selected from the group consisting of -H and C₁₋₆ alkyl; wherein C₁₋₆ alkyl is optionally substituted with one or more halogen, which are the same or different.

More preferably, the one or more further optional substituents are independently of each other selected from the group consisting of halogen, -CN, -COOR^{x1}, -OR^{x1}, -C(O)R^{x1}, -C(O)N(R^{x1}R^{x1a}), -S(O)₂N(R^{x1}R^{x1a}), -S(O)N(R^{x1}R^{x1a}), -S(O)₂R^{x1}, -S(O)R^{x1}, -N(R^{x1})S(O)₂N(R^{x1a}R^{x1b}), -SR^{x1}, -N(R^{x1}R^{x1a}), -NO₂, -OC(O)R^{x1}, -N(R^{x1})C(O)R^{x1a}, -N(R^{x1})S(O)₂R^{x1a}, -N(R^{x1})S(O)R^{x1a}, -N(R^{x1})C(O)OR^{x1a}, -N(R^{x1})C(O)N(R^{x1a}R^{x1b}), -OC(O)N(R^{x1}R^{x1a}), -T⁰, C₁₋₁₀ alkyl, C₂₋₁₀ alkenyl, and C₂₋₁₀ alkynyl; wherein -T⁰, C₁₋₁₀ alkyl, C₂₋₁₀ alkenyl, and C₂₋₁₀ alkynyl are optionally substituted with one or more -R^{x2}, which are the same or different and wherein C₁₋₁₀ alkyl, C₂₋₁₀ alkenyl, and C₂₋₁₀ alkynyl are optionally interrupted by one or more groups selected from the group consisting of -T⁰-, -C(O)O-, -O-, -C(O)-, -C(O)N(R^{x3})-, -S(O)₂N(R^{x3})-, -S(O)N(R^{x3})-, -S(O)₂-, -N(R^{x3})S(O)₂N(R^{x3a})-, -S-, -N(R^{x3})-, -OC(OR^{x3})(R^{x3a})-, -N(R^{x3})C(O)N(R^{x3a})-, and -OC(O)N(R^{x3})-;
each -R^{x1}, -R^{x1a}, -R^{x1b}, -R^{x3}, -R^{x3a} is independently selected from the group consisting of -H, halogen, C₁₋₆ alkyl, C₂₋₆ alkenyl, and C₂₋₆ alkynyl;
each T⁰ is independently selected from the group consisting of phenyl, naphthyl, indenyl, indanyl, tetralinyl, C₃₋₁₀ cycloalkyl, 3- to 10-membered heterocyclyl, and 8- to 11-membered heterobicyclyl; wherein each T⁰ is independently optionally substituted with one or more -R^{x2}, which are the same or different;
each -R^{x2} is independently selected from the group consisting of halogen, -CN, oxo (=O), -COOR^{x4}, -OR^{x4}, -C(O)R^{x4}, -C(O)N(R^{x4}R^{x4a}), -S(O)₂N(R^{x4}R^{x4a}), -S(O)N(R^{x4}R^{x4a}), -S(O)₂R^{x4}, -S(O)R^{x4}, -N(R^{x4})S(O)₂N(R^{x4a}R^{x4b}), -SR^{x4}, -N(R^{x4}R^{x4a}), -NO₂, -OC(O)R^{x4}, -N(R^{x4})C(O)R^{x4a}, -N(R^{x4})S(O)₂R^{x4a}, -N(R^{x4})S(O)R^{x4a}, -N(R^{x4})C(O)OR^{x4a}, -N(R^{x4})C(O)N(R^{x4a}R^{x46}), -OC(O)N(R^{x4}R^{x4a}), and C₁₋₆ alkyl; wherein C₁₋₆ alkyl is optionally substituted with one or more halogen, which are the same or different;
each -R^{x4}, -R^{x4a}, -R^{x4b} is independently selected from the group consisting of -H, halogen, C₁₋₆ alkyl, C₂₋₆ alkenyl, and C₂₋₆ alkynyl;
Even more preferably, the one or more further optional substituents are independently of each other selected from the group consisting of halogen, -CN, -COOR^{x1}, -OR^{x1}, -C(O)R^{x1}, -C(O)N(R^{x1}R^{x1a}), -S(O)₂N(R^{x1}R^{x1a}), -S(O)N(R^{x1}R^{x1a}), -S(O)₂R^{x1}, -S(O)R^{x1}, -N(R^{x1})S(O)₂N(R^{x1a}R^{x1b}), -SR^{x1}, -N(R^{x1}R^{x1a}), -NO₂, -OC(O)R^{x1}, -N(R^{x1})C(O)R^{x1a}, -N(R^{x1})S(O)₂R^{x1a}, -N(R^{x1})S(O)R^{x1a}, -N(R^{x1})C(O)OR^{x1a}, -N(R^{x1})C(O)N(R^{x1a}R^{x1b}), -OC(O)N(R^{x1}R^{x1a}), -T⁰, C₁₋₆ alkyl, C₂₋₆ alkenyl, and C₂₋₆ alkynyl; wherein -T⁰, C₁₋₆ alkyl, C₂₋₆ alkenyl, and C₂₋₆ alkynyl are optionally substituted with one or more -R^{x2}, which are the same or different and wherein C₁₋₆ alkyl, C₂₋₆ alkenyl, and C₂₋₆ alkynyl are optionally interrupted by one or more groups selected from the group consisting of -T⁰-, -C(O)O-, -O-, -C(O)-, -C(O)N(R^{x3})-, -S(O)₂N(R^{x3})-, -S(O)N(R^{x3})-, -S(O)₂-, -N(R^{x3})S(O)₂N(R^{x3a})-, -S-, -N(R^{x3})-, -OC(OR^{x3})(R^{x3a})-, -N(R^{x3})C(O)N(R^{x3a})-, and -OC(O)N(R^{x3})-;
each -R^{x1}, -R^{x1a}, -R^{x1b}, -R^{x2}, -R^{x3}, -R^{x3a} is independently selected from the group consisting of -H, halogen, C₁₋₆ alkyl, C₂₋₆ alkenyl, and C₂₋₆ alkynyl;
each T⁰ is independently selected from the group consisting of phenyl, naphthyl, indenyl, indanyl, tetralinyl, C₃₋₁₀ cycloalkyl, 3- to 10-membered heterocyclyl, and 8- to 11-membered heterobicyclyl; wherein each T⁰ is independently optionally substituted with one or more -R^{x2}, which are the same or different.

Preferably, a maximum of 6 -H atoms of an optionally substituted molecule or moiety are independently replaced by a substituent, e.g. 5 -H atoms are independently replaced by a substituent, 4 -H atoms are independently replaced by a substituent, 3 -H atoms are independently replaced by a substituent, 2 -H atoms are independently replaced by a substituent, or 1 -H atom is replaced by a substituent.

The term "interrupted" means that a moiety is inserted between two carbon atoms or - if the insertion is at one of the moiety's ends - between a carbon or heteroatom and a hydrogen atom, preferably between a carbon and a hydrogen atom.

The term "spacer" refers to any moiety that is suitable to connect two moieties. Preferably, a spacer is selected from the group consisting of -T-, -C(O)O-, -O-, -C(O)-, -C(O)N(R^{y1})-, -S(O)₂N(R^{y1})-, -S(O)N(R^{y1})-, -S(O)₂-, -N(R^{y1})S(O)₂N(R^{y1a})-, -S-, -N(R^{y1})-, -OC(OR^{y1})(R^{y1a})-, -N(R^{y1})C(O)N(R^{y1a})-, -OC(O)N(R^{y1})-, C₁₋₅₀ alkyl, C₂₋₅₀ alkenyl, and C₂₋₅₀ alkynyl; wherein -T-, C₁₋₅₀ alkyl, C₂₋₅₀ alkenyl, and C₂₋₅₀ alkynyl are optionally substituted with one or more -R^{y2}, which are the same or different and wherein C₁₋₅₀ alkyl, C₂₋₅₀ alkenyl, and C₂₋₅₀ alkynyl are optionally interrupted by one or more groups selected from the group consisting of -T-, -C(O)O-, -O-, -C(O)-, -C(O)N(R^{y3})-, -S(O)₂N(R^{y3})-, -S(O)N(R^{y3})-, -S(O)₂-, -S(O)-, -N(R^{y3})S(O)₂N(R^{y3a})-, -S-, -N(R^{y3})-, -OC(OR^{y3})(R^{y3a})-, -N(R^{y3})C(O)N(R^{y3a})-, and -OC(O)N(R^{y3})-;
-R^{y1} and -R^{y1a} are independently of each other selected from the group consisting of -H, -T, C₁₋₅₀ alkyl, C₂₋₅₀ alkenyl, and C₂₋₅₀ alkynyl; wherein -T, C₁₋₅₀ alkyl, C₂₋₅₀ alkenyl, and C₂₋₅₀ alkynyl are optionally substituted with one or more -R^{y2}, which are the same or different, and wherein C₁₋₅₀ alkyl, C₂₋₅₀ alkenyl, and C₂₋₅₀ alkynyl are optionally interrupted by one or more groups selected from the group consisting of -T-, -C(O)O-, -O-, -C(O)-, -C(O)N(R^{y4})-, -S(O)₂N(R^{y4})-, -S(O)N(R^{y4})-, -S(O)₂-, -S(O)-, -N(R^{y4})S(O)₂N(R^{y4a})-, -S-, -N(R^{y4})-, -OC(OR^{y4})(R^{y4a})-, -N(R^{y4})C(O)N(R^{y4a})-, and -OC(O)N(R^{y4})-;
each T is independently selected from the group consisting of phenyl, naphthyl, indenyl, indanyl, tetralinyl, C₃₋₁₀ cycloalkyl, 3- to 10-membered heterocyclyl, 8- to 11-membered heterobicyclyl, 8-to 30-membered carbopolycyclyl, and 8- to 30-membered heteropolycyclyl; wherein each T is independently optionally substituted with one or more -R^{y2}, which are the same or different;
each -R^{y2} is independently selected from the group consisting of halogen, -CN, oxo (=O), -COOR^{y5}, -OR^{y5}, -C(O)R^{y5}, -C(O)N(R^{y5}R^{y5a}), -S(O)₂N(R^{y5}R^{y5a}), -S(O)N(R^{y5}R^{y5a}), -S(O)₂R^{y5}, -S(O)R^{y5}, -N(R^{y5})S(O)₂N(R^{y5a}R^{y5b}), -SR^{y5}, -N(R^{y5}R^{y5a}), -NO₂, -OC(O)R^{y5}, -N(R^{y5})C(O)R^{y5a}, -N(R^{y5})S(O)₂R^{y5a}, -N(R^{y5})S(O)R^{y5a}, -N(R^{y5})C(O)OR^{y5a}, -N(R^{y5})C(O)N(R^{y5a}R^{y5b}), -OC(O)N(R^{y5}R^{y5a}), and C₁₋₆ alkyl; wherein C₁₋₆ alkyl is optionally substituted with one or more halogen, which are the same or different; and
each -R^{y3}, -R^{y3a}, -R^{y4}, -R^{y4a}, -R^{y5}, -R^{y5a} and -R^{y5b} is independently selected from the group consisting of -H, and C₁₋₆ alkyl, wherein C₁₋₆ alkyl is optionally substituted with one or more halogen, which are the same or different.

Even more preferably the spacer is selected from -T-, -C(O)O-, -O-, -C(O)-, -C(O)N(R^{y1})-, -S(O)₂N(R^{y1})-, -S(O)N(R^{y1})-, -S(O)₂-, -N(R^{y1})S(O)₂N(R^{y1a})-, -S-, -N(R^{y1})-, -OC(OR^{y1})(R^{y1a})-, -N(R^{y1})C(O)N(R^{y1a})-, -OC(O)N(R^{y1})-, C₁₋₅₀ alkyl, C₂₋₅₀ alkenyl, and C₂₋₅₀ alkynyl; wherein -T-, C₁₋₂₀ alkyl, C₂₋₂₀ alkenyl, and C₂₋₂₀ alkynyl are optionally substituted with one or more -R^{y2}, which are the same or different and wherein C₁₋₂₀ alkyl, C₂₋₂₀ alkenyl, and C₂₋₂₀ alkynyl are optionally interrupted by one or more groups selected from the group consisting of -T-, -C(O)O-, -O-, -C(O)-, -C(O)N(R^{y3})-, -S(O)₂N(R^{y3})-, -S(O)N(R^{y3})-, -S(O)₂-, -N(R^{y3})S(O)₂N(R^{y3a})-, -S-, -N(R^{y3})-, -OC(OR^{y3})(R^{y3a})-, -N(R^{y3})C(O)N(R^{y3a})-, and -OC(O)N(R^{y3})-;
-R^{y1} and -R^{y1a} are independently of each other selected from the group consisting of -H, -T, C₁₋₁₀ alkyl, C₂₋₁₀ alkenyl, and C₂₋₁₀ alkynyl; wherein -T, C₁₋₁₀ alkyl, C₂₋₁₀ alkenyl, and C₂₋₁₀ alkynyl are optionally substituted with one or more -R^{y2}, which are the same or different, and wherein C₁₋₁₀ alkyl, C₂₋₁₀ alkenyl, and C₂₋₁₀ alkynyl are optionally interrupted by one or more groups selected from the group consisting of -T-, -C(O)O-, -O-, -C(O)-, -C(O)N(R^{y4})-, -S(O)₂N(R^{y4})-, -S(O)N(R^{y4})-, -S(O)₂-, -S(O)-, -N(R^{y4})S(O)₂N(R^{y4a})-, -S-, -N(R^{y4})-, -OC(OR^{y4})(R^{y4a})-, -N(R^{y4})C(O)N(R^{y4a})-, and -OC(O)N(R^{y4})-;
each T is independently selected from the group consisting of phenyl, naphthyl, indenyl, indanyl, tetralinyl, C₃₋₁₀ cycloalkyl, 3- to 10-membered heterocyclyl, 8- to 11-membered heterobicyclyl, 8-to 30-membered carbopolycyclyl, and 8- to 30-membered heteropolycyclyl; wherein each T is independently optionally substituted with one or more -R^{y2}, which are the same or different;
-R^{y2} is selected from the group consisting of halogen, -CN, oxo (=O), -COOR^{y5}, -OR^{y5}, -C(O)R^{y5}, -C(O)N(R^{y5}R^{y5a}), -S(O)₂N(R^{y5}R^{y5a}), -S(O)N(R^{y5}R^{y5a}), -S(O)₂R^{y5}, -S(O)R^{y5}, -N(R^{y5})S(O)₂N(R^{y5a}R^{y5b}), -SR^{y5}, -N(R^{y5}R^{y5a}), -NO₂, -OC(O)R^{y5}, -N(R^{y5})C(O)R^{y5a}, -N(R^{y5})S(O)₂R^{y5a}, -N(R^{y5})S(O)R^{y5a}, -N(R^{y5})C(O)OR^{y5a}, -N(R^{y5})C(O)N(R^{y5a}R^{y5b}), -OC(O)N(R^{y5}R^{y5a}), and C₁₋₆ alkyl; wherein C₁₋₆ alkyl is optionally substituted with one or more halogen, which are the same or different; and
each -R^{y3}, -R^{y3a}, -R^{y4}, -R^{y4a}, -R^{y5}, -R^{y5a} and -R^{y5b} is independently of each other selected from the group consisting of -H, and C₁₋₆ alkyl; wherein C₁₋₆ alkyl is optionally substituted with one or more halogen, which are the same or different.

Even more preferably the spacer is selected from the group consisting of -T-, -C(O)O-, -O-, -C(O)-, -C(O)N(R^{y1})-, -S(O)₂N(R^{y1})-, -S(O)N(R^{y1})-, -S(O)₂-, -N(R^{y1})S(O)₂N(R^{y1a})-, -S-, -N(R^{y1})-, -OC(OR^{y1})(R^{y1a})-, -N(R^{y1})C(O)N(R^{y1a})-, -OC(O)N(R^{y1})-, C₁₋₅₀ alkyl, C₂₋₅₀ alkenyl, and C₂₋₅₀ alkynyl; wherein -T-, C₁₋₅₀ alkyl, C₂₋₅₀ alkenyl, and C₂₋₅₀ alkynyl are optionally substituted with one or more -R^{y2}, which are the same or different and wherein C₁₋₅₀ alkyl, C₂₋₅₀ alkenyl, and C₂₋₅₀ alkynyl are optionally interrupted by one or more groups selected from the group consisting of -T-, -C(O)O-, -O-, -C(O)-, -C(O)N(R^{y3})-, -S(O)₂N(R^{y3})-, -S(O)N(R^{y3})-, -S(O)₂-, -S(O)-, -N(R^{y3})S(O)₂N(R^{y3a})-, -S-, -N(R^{y3})-, -OC(OR^{y3})(R^{y3a})-, -N(R^{y3})C(O)N(R^{y3a})-, and -OC(O)N(R^{y3})-;
-R^{y1} and -R^{y1a} are independently selected from the group consisting of -H, -T, C₁₋₁₀ alkyl, C₂₋₁₀ alkenyl, and C₂₋₁₀ alkynyl;
each T is independently selected from the group consisting of phenyl, naphthyl, indenyl, indanyl, tetralinyl, C₃₋₁₀ cycloalkyl, 3- to 10-membered heterocyclyl, 8- to 11-membered heterobicyclyl, 8-to 30-membered carbopolycyclyl, and 8- to 30-membered heteropolycyclyl; each -R^{y2} is independently selected from the group consisting of halogen, and C₁₋₆ alkyl; and
each -R^{y3}, -R^{y3a}, -R^{y4}, -R^{y4a}, -R^{y5}, -R^{y5a} and -R^{y5b} is independently of each other selected from the group consisting of -H, and C₁₋₆ alkyl; wherein C₁₋₆ alkyl is optionally substituted with one or more halogen, which are the same or different.

As used herein, the term "C₁₋₄ alkyl" alone or in combination means a straight-chain or branched alkyl moiety having 1 to 4 carbon atoms. If present at the end of a molecule, examples of straight-chain or branched C₁₋₄ alkyl are methyl, ethyl, n-propyl, isopropyl, n-butyl, isobutyl, sec-butyl and tert-butyl. When two moieties of a molecule are linked by the C₁₋₄ alkyl, then examples for such C₁₋₄ alkyl groups are -CH₂-, -CH₂-CH₂-, -CH(CH₃)-, -CH₂-CH₂-CH₂-, -CH(C₂H₅)-, -C(CH₃)₂-. Each hydrogen of a C₁₋₄ alkyl carbon may optionally be replaced by a substituent as defined above. Optionally, a C₁₋₄ alkyl may be interrupted by one or more moieties as defined below.

As used herein, the term "C₁₋₆ alkyl" alone or in combination means a straight-chain or branched alkyl moiety having 1 to 6 carbon atoms. If present at the end of a molecule, examples of straight-chain and branched C₁₋₆ alkyl groups are methyl, ethyl, n-propyl, isopropyl, n-butyl, isobutyl, sec-butyl, tert-butyl, n-pentyl, 2-methylbutyl, 2,2-dimethylpropyl, n-hexyl, 2-methylpentyl, 3-methylpentyl, 2,2-dimethylbutyl, 2,3-dimethylbutyl and 3,3-dimethylpropyl. When two moieties of a molecule are linked by the C₁₋₆ alkyl group, then examples for such C₁₋₆ alkyl groups are -CH₂-, -CH₂-CH₂-, -CH(CH₃)-, -CH₂-CH₂-CH₂-, -CH(C₂H₅)- and -C(CH₃)₂-. Each hydrogen atom of a C₁₋₆ carbon may optionally be replaced by a substituent as defined above. Optionally, a C₁₋₆ alkyl may be interrupted by one or more moieties as defined below.

Accordingly, "C₁₋₁₀ alkyl", "C₁₋₂₀ alkyl" or "C₁₋₅₀ alkyl" means an alkyl chain having 1 to 10, 1 to 20 or 1 to 50 carbon atoms, respectively, wherein each hydrogen atom of the C₁₋₁₀, C₁₋₂₀ or C₁₋₅₀ carbon may optionally be replaced by a substituent as defined above. Optionally, a C₁₋₁₀ or C₁₋₅₀ alkyl may be interrupted by one or more moieties as defined below.

As used herein, the term "C₂₋₆ alkenyl" alone or in combination means a straight-chain or branched hydrocarbon moiety comprising at least one carbon-carbon double bond having 2 to 6 carbon atoms. If present at the end of a molecule, examples are -CH=CH₂, -CH=CH-CH₃, -CH₂-CH=CH₂, -CH=CHCH₂-CH₃ and -CH=CH-CH=CH₂. When two moieties of a molecule are linked by the C₂₋₆ alkenyl group, then an example for such C₂₋₆ alkenyl is - CH=CH-. Each hydrogen atom of a C₂₋₆ alkenyl moiety may optionally be replaced by a substituent as defined above. Optionally, a C₂₋₆ alkenyl may be interrupted by one or more moieties as defined below.

Accordingly, the term "C₂₋₁₀ alkenyl", "C₂₋₂₀ alkenyl" or "C₂₋₅₀ alkenyl" alone or in combination means a straight-chain or branched hydrocarbon moiety comprising at least one carbon-carbon double bond having 2 to 10, 2 to 20 or 2 to 50 carbon atoms. Each hydrogen atom of a C₂₋₁₀ alkenyl, C₂₋₂₀ alkenyl or C₂₋₅₀ alkenyl group may optionally be replaced by a substituent as defined above. Optionally, a C₂₋₁₀ alkenyl, C₂₋₂₀ alkenyl or C₂₋₅₀ alkenyl may be interrupted by one or more moieties as defined below.

As used herein, the term "C₂₋₆ alkynyl" alone or in combination means straight-chain or branched hydrocarbon moiety comprising at least one carbon-carbon triple bond having 2 to 6 carbon atoms. If present at the end of a molecule, examples are -C≡CH, -CH₂-C≡CH, CH₂-CH₂-C≡CH and CH₂-C≡C-CH₃. When two moieties of a molecule are linked by the alkynyl group, then an example is -C=C-. Each hydrogen atom of a C₂₋₆ alkynyl group may optionally be replaced by a substituent as defined above. Optionally, one or more double bond(s) may occur. Optionally, a C₂₋₆ alkynyl may be interrupted by one or more moieties as defined below.

Accordingly, as used herein, the term "C₂₋₁₀ alkynyl", "C₂₋₂₀ alkynyl" and "C₂₋₅₀ alkynyl" alone or in combination means a straight-chain or branched hydrocarbon moiety comprising at least one carbon-carbon triple bond having 2 to 10, 2 to 20 or 2 to 50 carbon atoms, respectively. Each hydrogen atom of a C₂₋₁₀ alkynyl, C₂₋₂₀ alkynyl or C₂₋₅₀ alkynyl group may optionally be replaced by a substituent as defined above. Optionally, one or more double bond(s) may occur. Optionally, a C₂₋₁₀ alkynyl, C₂₋₂₀ alkynyl or C₂₋₅₀ alkynyl may be interrupted by one or more moieties as defined below.

As mentioned above, a C₁₋₄ alkyl, C₁₋₆ alkyl, C₁₋₁₀ alkyl, C₁₋₂₀ alkyl, C₁₋₅₀ alkyl, C₂₋₆ alkenyl, C₂₋₁₀ alkenyl, C₂₋₂₀ alkenyl, C₂₋₅₀ alkenyl, C₂₋₆ alkynyl, C₂₋₁₀ alkynyl, C₂₋₂₀ alkenyl or C₂₋₅₀ alkynyl may optionally be interrupted by one or more moieties, which are preferably selected from the group consisting of 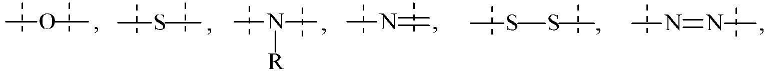 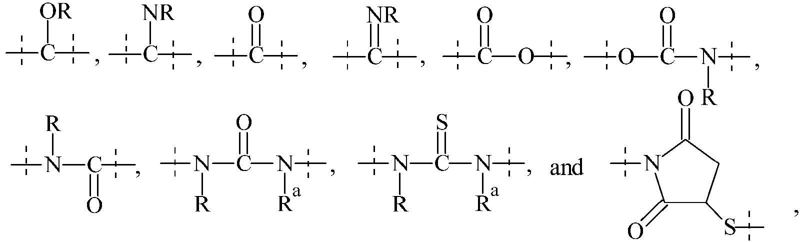 wherein
dashed lines indicate attachment to the remainder of the moiety or reagent; and
-R and -R^{a} are independently of each other selected from the group consisting of -H, methyl, ethyl, propyl, butyl, pentyl and hexyl.

As used herein, the term "C₃₋₁₀ cycloalkyl" means a cyclic alkyl chain having 3 to 10 carbon atoms, which may be saturated or unsaturated, e.g. cyclopropyl, cyclobutyl, cyclopentyl, cyclohexyl, cyclohexenyl, cycloheptyl, cyclooctyl, cyclononyl or cyclodecyl. Each hydrogen atom of a C₃₋₁₀ cycloalkyl carbon may be replaced by a substituent as defined above. The term "C₃₋₁₀ cycloalkyl" also includes bridged bicycles like norbornane or norbornene.

The term "8- to 30-membered carbopolycyclyl" or "8- to 30-membered carbopolycycle" means a cyclic moiety of two or more rings with 8 to 30 ring atoms, where two neighboring rings share at least one ring atom and that may contain up to the maximum number of double bonds (aromatic or non-aromatic ring which is fully, partially or un-saturated). Preferably a 8- to 30-membered carbopolycyclyl means a cyclic moiety of two, three, four or five rings, more preferably of two, three or four rings.

As used herein, the term "3- to 10-membered heterocyclyl" or "3- to 10-membered heterocycle" means a ring with 3, 4, 5, 6, 7, 8, 9 or 10 ring atoms that may contain up to the maximum number of double bonds (aromatic or non-aromatic ring which is fully, partially or un-saturated) wherein at least one ring atom up to 4 ring atoms are replaced by a heteroatom selected from the group consisting of sulfur (including -S(O)-, -S(O)₂-), oxygen and nitrogen (including =N(O)-) and wherein the ring is linked to the rest of the molecule via a carbon or nitrogen atom. Examples for 3- to 10-membered heterocycles include but are not limited to aziridine, oxirane, thiirane, azirine, oxirene, thiirene, azetidine, oxetane, thietane, furan, thiophene, pyrrole, pyrroline, imidazole, imidazoline, pyrazole, pyrazoline, oxazole, oxazoline, isoxazole, isoxazoline, thiazole, thiazoline, isothiazole, isothiazoline, thiadiazole, thiadiazoline, tetrahydrofuran, tetrahydrothiophene, pyrrolidine, imidazolidine, pyrazolidine, oxazolidine, isoxazolidine, thiazolidine, isothiazolidine, thiadiazolidine, sulfolane, pyran, dihydropyran, tetrahydropyran, imidazolidine, pyridine, pyridazine, pyrazine, pyrimidine, piperazine, piperidine, morpholine, tetrazole, triazole, triazolidine, tetrazolidine, diazepane, azepine and homopiperazine. Each hydrogen atom of a 3- to 10-membered heterocyclyl or 3-to 10-membered heterocyclic group may be replaced by a substituent as defined below.

As used herein, the term "8- to 11-membered heterobicyclyl" or "8- to 11-membered heterobicycle" means a heterocyclic moiety of two rings with 8 to 11 ring atoms, where at least one ring atom is shared by both rings and that may contain up to the maximum number of double bonds (aromatic or non-aromatic ring which is fully, partially or un-saturated) wherein at least one ring atom up to 6 ring atoms are replaced by a heteroatom selected from the group consisting of sulfur (including -S(O)-, -S(O)₂-), oxygen and nitrogen (including =N(O)-) and wherein the ring is linked to the rest of the molecule via a carbon or nitrogen atom. Examples for an 8- to 11-membered heterobicycle are indole, indoline, benzofuran, benzothiophene, benzoxazole, benzisoxazole, benzothiazole, benzisothiazole, benzimidazole, benzimidazoline, quinoline, quinazoline, dihydroquinazoline, quinoline, dihydroquinoline, tetrahydroquinoline, decahydroquinoline, isoquinoline, decahydroisoquinoline, tetrahydroisoquinoline, dihydroisoquinoline, benzazepine, purine and pteridine. The term 8-to 11-membered heterobicycle also includes spiro structures of two rings like 1,4-dioxa-8-azaspiro[4.5]decane or bridged heterocycles like 8-aza-bicyclo[3.2.1]octane. Each hydrogen atom of an 8- to 11-membered heterobicyclyl or 8- to 11-membered heterobicycle carbon may be replaced by a substituent as defined below.

Similary, the term "8- to 30-membered heteropolycyclyl" or "8- to 30-membered heteropolycycle" means a heterocyclic moiety of more than two rings with 8 to 30 ring atoms, preferably of three, four or five rings, where two neighboring rings share at least one ring atom and that may contain up to the maximum number of double bonds (aromatic or non-aromatic ring which is fully, partially or unsaturated), wherein at least one ring atom up to 10 ring atoms are replaced by a heteroatom selected from the group of sulfur (including -S(O)-, -S(O)₂-), oxygen and nitrogen (including =N(O)-) and wherein the ring is linked to the rest of a molecule via a carbon or nitrogen atom.

It is understood that the phrase "the pair R^{x}/R^{y} is joined together with the atom to which they are attached to form a C₃₋₁₀ cycloalkyl or a 3- to 10-membered heterocyclyl" in relation with a moiety of the structure 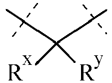 means that Rx and Ry form the following structure: 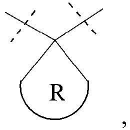 wherein R is C₃₋₁₀ cycloalkyl or 3- to 10-membered heterocyclyl.

It is also understood that the phrase "the pair R^{x}/R^{y} is joint together with the atoms to which they are attached to form a ring A" in relation with a moiety of the structure 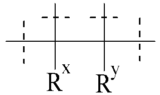 means that R^{x} and R^{y} form the following structure:

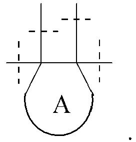

As used herein, "halogen" means fluoro, chloro, bromo or iodo. It is generally preferred that halogen is fluoro or chloro.

In general, the term "comprise" or "comprising" also encompasses "consist of" or "consisting of".

In certain embodiments the starting dose ranges from 0.8 to 4.9 nmol/day, preferably from 1 to 4.8 nmol/day, more preferably from 1.2 to 4.7 nmol/day, even more preferably from 1.5 to 4.6 nmol/day, even more preferably from 1.8 to 4.5 nmol/day and most preferably from 2 to 4.4 nmol/day.

In certain embodiments the starting dose ranges from 0.8 to 3.9 nmol/day, preferably from 1 to 3.7 nmol/day, even more preferably from 1.5 to 3.4 nmol/day, even more preferably from 2 to 3.2 nmol/day and most preferably from 2.5 to 3 nmol/day.

In certain embodiments the starting dose is at least 0.8 nmol/day. In certain embodiments the starting dose is at least 1.0 nmol/day. In certain embodiments the starting dose is at least 1.2 nmol/day. In certain embodiments the starting dose is at least 1.4 nmol/day. In certain embodiments the starting dose is at least 1.6 nmol/day. In certain embodiments the starting dose is at least 1.8 nmol/day. In certain embodiments the starting dose is at least 1.9 nmol/day. In certain embodiments the starting dose is at least 2.0 nmol/day. In certain embodiments the starting dose is at least 2.1 nmol/day. In certain embodiments the starting dose is at least 2.2 nmol/day. In certain embodiments the starting dose is at least 2.3 nmol/day. In certain embodiments the starting dose is at least 2.4 nmol/day. In certain embodiments the starting dose is at least 2.5 nmol/day. In certain embodiments the starting dose is at least 2.6 nmol/day. In certain embodiments the starting dose is at least 2.7 nmol/day. In certain embodiments the starting dose is at least 2.8 nmol/day. In certain embodiments the starting dose is at least 2.9 nmol/day. In certain embodiments the starting dose is at least 3.0 nmol/day. In certain embodiments the starting dose is at least 3.1 nmol/day. In certain embodiments the starting dose is at least 3.2 nmol/day. In certain embodiments the starting dose is at least 3.3 nmol/day. In certain embodiments the starting dose is at least 3.4 nmol/day. In certain embodiments the starting dose is at least 3.5 nmol/day. In certain embodiments the starting dose is at least 3.6 nmol/day. In certain embodiments the starting dose is at least 3.7 nmol/day. In certain embodiments the starting dose is at least 3.8 nmol/day. In certain embodiments the starting dose is at least 3.9 nmol/day. In certain embodiments the starting dose is at least 4.0 nmol/day.

**In certain** embodiments the starting dose is at most 4.9 nmol/day. In certain embodiments the starting dose is at most 4.8 nmol/day. In certain embodiments the starting dose is at most 4.7 nmol/day. In certain embodiments the starting dose is at most 4.6 nmol/day. In certain embodiments the starting dose is at most 4.5 nmol/day. In certain embodiments the starting dose is at most 4.4 nmol/day. In certain embodiments the starting dose is at most 4.3 nmol/day. In certain embodiments the starting dose is at most 4.2 nmol/day. In certain embodiments the starting dose is at most 4.1 nmol/day. In certain embodiments the starting dose is at most 4.0 nmol/day. In certain embodiments the starting dose is at most 3.9 nmol/day.

In certain embodiments the starting dose is at least 2.9 nmol/day and at most 4.9 nmol/day. In certain embodiments the starting dose ist at least 2.9 nmol/day and at most 4.5 nmol/day.

In one embodiment the starting dose is 1.5 ± 0.3 nmol/day, preferably 1.5 ± 0.2 nmol/day, even more preferably 1.5 ± 0.1 nmol/day. In another embodiment the starting dose is 1.7 ± 0.3 nmol/day, preferably 1.7 ± 0.2 nmol/day, even more preferably 1.7 ± 0.1 nmol/day. In another embodiment the starting dose is 2 ± 0.3 nmol/day, preferably 2 ± 0.2 nmol/day, even more preferably 2 ± 0.1 nmol/day. In another embodiment the starting dose is 2.3 ± 0.3 nmol/day, preferably 2.3 ± 0.2 nmol/day, even more preferably 2.3 ± 0.1 nmol/day. In another embodiment the starting dose is 2.5 ± 0.3 nmol/day, preferably 2.5 ± 0.2 nmol/day, even more preferably 2.5 ± 0.1 nmol/day. In another embodiment the starting dose is 2.7 ± 0.3 nmol/day, preferably 2.7 ± 0.2 nmol/day, even more preferably 2.7 ± 0.1 nmol/day. In another embodiment the starting dose is 2.9 ± 0.3 nmol/day, preferably 2.9 ± 0.2 nmol/day, even more preferably 2.9 ± 0.1 nmol/day. In another embodiment the starting dose is 3.2 ± 0.3 nmol/day, preferably 3.2 ± 0.2 nmol/day, even more preferably 3.2 ± 0.1 nmol/day. In another embodiment the starting dose is 3.5 ± 0.3 nmol/day, preferably 3.5 ± 0.2 nmol/day, even more preferably 3.5 ± 0.1 nmol/day. In another embodiment the starting dose is 3.7 ± 0.3 nmol/day, preferably 3.7 ± 0.2 nmol/day, even more preferably 3.7 ± 0.1 nmol/day. In another embodiment the starting dose is 3.8 ± 0.3 nmol/day, preferably 3.8 ± 0.2 nmol/day, even more preferably 3.8 ± 0.1 nmol/day. In another embodiment the starting dose is 3.9 ± 0.3 nmol/day, preferably 3.9 ± 0.2 nmol/day, even more preferably 3.9 ± 0.1 nmol/day. In another embodiment the starting dose is 4.0 ± 0.3 nmol/day, preferably 4.0 ± 0.2 nmol/day, even more preferably 4.0 ± 0.1 nmol/day. In another embodiment the starting dose is 4.1 ± 0.3 nmol/day, preferably 4.1 ± 0.2 nmol/day, even more preferably 4.1 ± 0.1 nmol/day. In another embodiment the starting dose is 4.2 ± 0.3 nmol/day, preferably 4.2 ± 0.2 nmol/day, even more preferably 4.2 ± 0.1 nmol/day. In another embodiment the starting dose is 4.2 ± 0.3 nmol/day, preferably 4.2 ± 0.2 nmol/day, even more preferably 4.2 ± 0.1 nmol/day. In another embodiment the starting dose is 4.3 ± 0.3 nmol/day, preferably 4.3 ± 0.2 nmol/day, even more preferably 4.3 ± 0.1 nmol/day. In another embodiment the starting dose is 4.4 ± 0.3 nmol/day, preferably 4.4 ± 0.2 nmol/day, even more preferably 4.4 ± 0.1 nmol/day. In another embodiment the starting dose is 4.5 ± 0.3 nmol/day, preferably 4.5 ± 0.2 nmol/day, even more preferably 4.5 ± 0.1 nmol/day. In another embodiment the starting dose is 4.6 ± 0.3 nmol/day, preferably 4.6 ± 0.2 nmol/day, even more preferably 4.6 ± 0.1 nmol/day.

The disease that can be treated, controlled, delayed or prevented with PTH is preferably selected from the group consisting of hypoparathyroidism, hyperphosphatemia, osteoporosis, fracture repair, osteomalacia, osteomalacia and osteoporosis in patients with hypophosphatasia, steroid-induced osteoporosis, male osteoporosis, arthritis, osteoarthritis, osteogenesis imperfect, fibrous dysplasia, rheumatoid arthritis, Paget's disease, humoral hypercalcemia associated with malignancy, osteopenia, periodontal disease, bone fracture, alopecia, chemotherapy-induced alopecia, and thrombocytopenia. Most preferably said disease is hypoparathyroidism.

Preferably, the disease to be treated with the PTH conjugate or a pharmaceutically acceptable salt thereof or the pharmaceutical composition comprising at least one PTH conjugate of the present invention occurs in a mammalian patient, more preferably in a human patient. Likewise, the patient suffering from a disease, which can be treated, controlled, delayed or prevented with PTH is preferably a mammalian patient, more preferably a human patient.

Preferably the PTH conjugate or a pharmaceutically acceptable salt thereof or the pharmaceutical composition comprising said PTH conjugate or pharmaceutically acceptable salt thereof is administered to the patient daily, i.e. once every day. Even more preferably the PTH conjugate or a pharmaceutically acceptable salt thereof or the pharmaceutical composition comprising said PTH conjugate or pharmaceutically acceptable salt thereof is administered to the patient once every 24 hours.

Preferably, the PTH conjugate or a pharmaceutically acceptable salt thereof or a pharmaceutical composition comprising said PTH conjugate or pharmaceutically acceptable salt thereof of the present invention is for use in the treatment of a disease that can be treated with PTH.

Preferably, the method of the present invention is a method of treating a patient suffering from a disease that can be treated with PTH.

The PTH conjugates release PTH under physiological conditions (aqueous buffer, pH 7.4 and 37°C) with a half-life ranging from 6 hours to one week, more preferably from 12 hours to 6 days, even more preferably from one day to 5 days, even more preferably from 2 days to 4 days, even more preferably from 2 days to 3 days.

Preferably, the total mass of the PTH conjugate of the present invention is at least 10 kDa, such as at least 12 kDa, such as at least 15 kDa, such as at least 20 kDa or such as at least 30 kDa. It is preferred that the total mass of the PTH conjugate of the present invention is at most 250 kDa, such as at most 200 kDa, at most 180 kDa, at most 150 kDa, at most 100 kDa, at most 80 kDa or at most 60 kDa.

Preferably, the PTH conjugate is of formula (Ia) or (Ib) 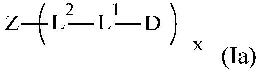 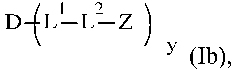 wherein
- D is a PTH moiety;
- L¹- is a linker moiety covalently and reversibly attached to -D;
- L²- is a chemical bond or is a spacer moiety;
- Z is a polymeric moiety or a substituted fatty acid moiety;

x is an integer selected from the group consisting of 1, 2, 3, 4, 5, 6, 7, 8, 9, 10, 11, 12, 13, 14, 15 or 16; and
y is an integer selected from the group consisting of 2, 3, 4 and 5.

Preferably, -D has the sequence of SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:107, SEQ ID NO:108, SEQ ID NO:109, SEQ ID NO:110, SEQ ID NO:111, SEQ ID NO:112, SEQ ID NO:113, SEQ ID NO:114 or SEQ ID NO:115. More preferably -D has the sequence of SEQ ID NO:50, SEQ ID NO:51, SEQ ID NO:52, SEQ ID NO:110, SEQ ID NO:111 or SEQ ID NO:112.

In one embodiment -D has the sequence of SEQ ID NO:50.

In another embodiment -D has the sequence of SEQ ID NO:52.

In another embodiment -D has the sequence of SEQ ID NO:110.

In another embodiment -D has the sequence of SEQ ID NO:111.

In another embodiment -D has the sequence of SEQ ID NO:112.

Most preferably -D has the sequence of SEQ ID NO:51.

The moiety -L¹- is either conjugated to a functional group of the side chain of an amino acid residue of -D, to the N-terminal amine functional group or to the C-terminal carboxyl functional group of -D or to a nitrogen atom in the backbone polypeptide chain of -D. Attachment to the N-terminus or C-terminus can either be directly through the corresponding amine or carboxyl functional group, respectively, or indirectly wherein a spacer moiety is first conjugated to the amine or carboxyl functional group to which spacer moiety -L¹- is conjugated.

Preferably, the amino acid residue of PTH to which -L¹- is conjugated comprises a functional group selected from the group consisting of carboxylic acid, primary and secondary amine, maleimide, thiol, sulfonic acid, carbonate, carbamate, hydroxyl, aldehyde, ketone, hydrazine, isocyanate, isothiocyanate, phosphoric acid, phosphonic acid, haloacetyl, alkyl halide, acryloyl, aryl fluoride, hydroxylamine, sulfate, disulfide, vinyl sulfone, vinyl ketone, diazoalkane, oxirane, guanidine and aziridine. Even more preferably the amino acid residue of PTH to which -L¹- is conjugated comprises a functional group selected from the group consisting hydroxyl, primary and secondary amine and guanidine. Even more preferably the amino acid residue of PTH to which -L¹- is conjugated comprises a primary or secondary amine functional group. Most preferably the amino acid residue of PTH to which -L¹- is conjugated comprises a primary amine functional group.

If the moiety -L¹- is conjugated to a functional group of the side chain of an amino acid residue of PTH said amino acid residue may be selected from the group consisting of proteinogenic amino acid residues and non-proteinogenic amino acid residues.

In one embodiment -L¹- is conjugated to a functional group of the side chain of a non-proteinogenic amino acid residue of PTH. It is understood that such non-proteinogenic amino acid is not found in the sequence of native PTH or fragments thereof and that it may only be present in variants, analogs, orthologs, homologs and derivatives of PTH.

In another embodiment -L¹- is conjugated to a functional group of the side chain of a proteinogenic amino acid residue of PTH. Preferably, said amino acid is selected from the group consisting of histidine, lysine, tryptophan, serine, threonine, tyrosine, aspartic acid, glutamic acid and arginine. Even more preferably said amino acid is selected from the group consisting of lysine, aspartic acid, arginine and serine. Even more preferably said amino acid is selected from the group consisting of lysine, arginine and serine.

In one embodiment -L¹- is conjugated to a functional group of the side chain of a histidine of PTH.

In another embodiment -L¹- is conjugated to a functional group of the side chain of a lysine of PTH.

In another embodiment -L¹- is conjugated to a functional group of the side chain of a tryptophan of PTH.

In another embodiment -L¹- is conjugated to a functional group of the side chain of a serine of PTH.

In another embodiment -L¹- is conjugated to a functional group of the side chain of a threonine of PTH.

In another embodiment -L¹- is conjugated to a functional group of the side chain of a tyrosine of PTH.

In another embodiment -L¹- is conjugated to a functional group of the side chain of a aspartic acid of PTH.

In another embodiment -L¹- is conjugated to a functional group of the side chain of a glutamic acid of PTH.

In another embodiment -L¹- is conjugated to a functional group of the side chain of an arginine of PTH.

It is understood that not every PTH moiety may comprise all of these amino acid residues.

In a preferred embodiment -L¹- is conjugated to the N-terminal amine functional group of PTH, either directly through the corresponding amine functional group or indirectly wherein a spacer moiety is first conjugated to the amine functional group to which spacer moiety -L¹- is conjugated. Even more preferably, -L¹- is directly conjugated to the N-terminal amine functional group of PTH, preferably PTH 1-34, i.e. PTH having the sequence of SEQ ID NO:51.

In another embodiment -L¹- is conjugated to the C-terminal functional group of PTH, either directly through the corresponding carboxyl functional group or indirectly wherein a spacer moiety is first conjugated to the carboxyl functional group to which spacer moiety -L¹- is conjugated.

Most preferably L¹- is directly conjugated to the N-terminal amine functional group of PTH.

The moiety -L¹- can be connected to -D through any type of linkage, provided that it is reversible. Preferably, -L¹- is connected to -D through a linkage selected from the group consisting of amide, ester, carbamate, acetal, aminal, imine, oxime, hydrazone, disulfide and acylguanidine. Even more preferably -L¹- is connected to -D through a linkage selected from the group consisting of amide, ester, carbamate and acylguanidin. It is understood that some of these linkages *per se* are not reversible, but that in the present invention neighboring groups comprised in -L¹- render these linkages reversible.

In one embodiment -L¹- is connected to -D through an ester linkage.

In another embodiment -L¹- is connected to -D through a carbamate linkage.

In another embodiment -L¹- is connected to -D through an acylguanidine.

In a preferred embodiment -L¹- is connected to -D through an amide linkage.

The moiety -L¹- is a reversible linker from which the drug, i.e. PTH, is released in its free form, i.e. it is a traceless linker. Suitable reversible linkers are known in the art, such as for example the reversible linker moieties disclosed in WO 2005/099768 A2, WO 2006/136586 A2, WO 2011/089216 A1 and WO 2013/024053 A1, which are herewith incorporated by reference.

In another embodiment -L¹- is a reversible linker as described in WO 2011/012722 A1, WO 2011/089214 A1, WO 2011/089215 A1, WO 2013/024052 A1 and WO 2013/160340 A1 which are herewith incorporated by reference.

A particularly preferred moiety -L¹- is disclosed in WO 2009/095479 A2. Accordingly, in a preferred embodiment the moiety -L¹- is of formula (II): 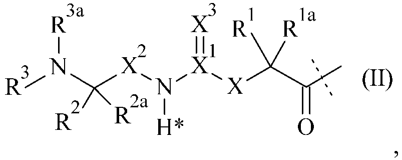 wherein the dashed line indicates the attachment to an amine, hydroxyl or thiol of -D which is a PTH moiety;
-X- is -C(R⁴R^{4a})-; -N(R⁴)-; -O-; -C(R⁴R^{4a})-C(R⁵R^{5a})-; -C(R⁵R^{5a})-C(R⁴R^{4a})-; -C(R⁴R^{4a})-N(R⁶)-; -N(R⁶)-C(R⁴R^{4a})-; -C(R⁴R^{4a})-O-; -O-C(R⁴R^{4a})-; or -C(R⁷R^{7a})-;
X¹ is C; or S(O);
-X²- is -C(R⁸R^{8a})-; or -C(R⁸R^{8a})-C(R⁹R^{9a})-;
=X³ is =O; =S; or =N-CN;
-R¹, -R^{1a}, -R², -R^{2a}, -R⁴, -R^{4a}, -R⁵, -R^{5a}, -R⁶, -R⁸, -R^{8a}, -R⁹, -R^{9a} are independently selected from the group consisting of -H; and C₁₋₆ alkyl;
-R³, -R^{3a} are independently selected from the group consisting of -H; and C₁₋₆ alkyl, provided that in case one of -R³, -R^{3a} or both are other than -H they are connected to N to which they are attached through an SP³-hybridized carbon atom;
-R⁷ is -N(R¹⁰R^{10a}); or -NR¹⁰-(C=O)-R¹¹;
-R^{7a}, -R¹⁰, -R^{10a}, -R¹¹ are independently of each other -H; or C₁₋₆ alkyl;
optionally, one or more of the pairs -R^{1a}/-R^{4a}, -R^{1a}/-R^{5a}, -R^{1a}/-R^{7a}, -R^{4a}/-R^{5a}, -R^{8a}/-R^{9a} form a chemical bond;
optionally, one or more of the pairs -R¹/-R^{1a}, -R²/-R^{2a}, -R⁴/-R^{4a}, -R⁵/-R^{5a}, -R⁸/-R^{8a}, -R⁹/-R^{9a} are joined together with the atom to which they are attached to form a C₃₋₁₀ cycloalkyl; or 3- to 10-membered heterocyclyl;
optionally, one or more of the pairs -R¹/-R⁴, -R¹/-R⁵, -R¹/-R⁶, -R¹/-R^{7a}, -R⁴/-R⁵, -R⁴/-R⁶, -R⁸/-R⁹, -R²/-R³ are joined together with the atoms to which they are attached to form a ring A;
optionally, R³/R^{3a} are joined together with the nitrogen atom to which they are attached to form a 3- to 10-membered heterocycle;
A is selected from the group consisting of phenyl; naphthyl; indenyl; indanyl; tetralinyl; C₃₋₁₀ cycloalkyl; 3- to 10-membered heterocyclyl; and 8- to 11-membered heterobicyclyl; and
wherein -L¹- is substituted with -L²-Z and wherein -L¹- is optionally further substituted, provided that the hydrogen marked with the asterisk in formula (II) is not replaced by -L²-Z or a substituent;
   wherein
   - L²- is a single chemical bond or a spacer;
   - Z is a polymeric moiety or a fatty acid-based moiety.

Preferably -L¹- of formula (II) is substituted with one moiety -L²-Z.

In one embodiment -L¹- of formula (II) is not further substituted.

It is understood that if -R³/-R^{3a} of formula (II) are joined together with the nitrogen atom to which they are attached to form a 3- to 10-membered heterocycle, only such 3- to 10-membered heterocycles may be formed in which the atoms directly attached to the nitrogen are SP³-hybridized carbon atoms. In other words, such 3- to 10-membered heterocycle formed by -R³/-R^{3a} together with the nitrogen atom to which they are attached has the following structure: 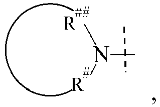 wherein
the dashed line indicates attachment to the rest of -L¹-;
the ring comprises 3 to 10 atoms comprising at least one nitrogen; and
R^{#} and R^{##} represent an SP³-hydridized carbon atom.

It is also understood that the 3- to 10-membered heterocycle may be further substituted.

Exemplary embodiments of suitable 3- to 10-membered heterocycles formed by -R³/-R^{3a} of formula (II) together with the nitrogen atom to which they are attached are the following: 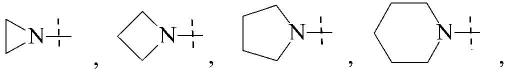 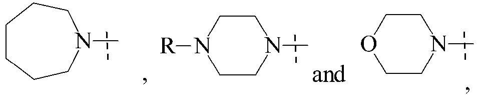 wherein
dashed lines indicate attachment to the rest of the molecule; and
-R is selected from the group consisting of -H and C₁₋₆ alkyl.

-L¹- of formula (II) may optionally be further substituted. In general, any substituent may be used as far as the cleavage principle is not affected, i.e. the hydrogen marked with the asterisk in formula (II) is not replaced and the nitrogen of the moiety 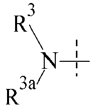 of formula (II) remains part of a primary, secondary or tertiary amine, i.e. -R³ and -R^{3a} are independently of each other -H or are connected to -N< through an SP³-hybridized carbon atom.

In one embodiment -R¹ or -R^{1a} of formula (II) is substituted with -L²-Z. In another embodiment -R² or -R^{2a} of formula (II) is substituted with -L²-Z. In another embodiment -R³ or -R^{3a} of formula (II) is substituted with -L²-Z. In another embodiment -R⁴ of formula (II) is substituted with -L²-Z. In another embodiment -R⁵ or -R^{5a} of formula (II) is substituted with -L²-Z. In another embodiment -R⁶ of formula (II) is substituted with -L²-Z. In another embodiment -R⁷ or -R^{7a} of formula (II) is substituted with -L²-Z. In another embodiment -R⁸ or -R^{8a} of formula (II) is substituted with -L²-Z. In another embodiment -R⁹ or -R^{9a} of formula (II) is substituted with -L²-Z. In another embodiment -R¹⁰ is substituted with -L²-Z. In another embodiment -R¹¹ is substituted with -L²-Z.

Preferably, -X- of formula (II) is selected from the group consisting of -C(R⁴R^{4a})-, -N(R⁴)- and -C(R⁷R^{7a})-.

In one embodiment -X- of formula (II) is -C(R⁴R^{4a})-.

In one preferred embodiment -X- of formula (II) is -C(R⁷R^{7a})-.

Preferably, -R⁷ of formula (II) is -NR¹⁰-(C=O)-R¹¹.

Preferably, -R^{7a} of formula (II) is selected from -H, methyl and ethyl. Most preferably -R^{7a} of formula (II) is -H.

Preferably, -R¹⁰ is selected from -H, methyl and ethyl. Most preferably -R¹⁰ is methyl.

Preferably, -R¹¹ is selected from -H, methyl and ethyl. Most preferably -R¹¹ is -H.

Preferably, -R¹¹ is substituted with -L²-Z.

In another preferred embodiment -X- of formula (II) is -N(R⁴)-.

Preferably, -R⁴ is selected from the group consisting of -H, methyl and ethyl. Preferably, -R⁴ is -H.

Preferably, X¹ of formula (II) is C.

Preferably, =X³ of formula (II) is =O.

Preferably, -X²- of formula (II) is -C(R⁸R^{8a})-.

Preferably -R⁸ and -R^{8a} of formula (II) are independently selected from the group consisting of -H, methyl and ethyl. More preferably at least one of -R⁸ and -R^{8a} of formula (II) is -H. Even more preferably both -R⁸ and -R^{8a} of formula (II) are -H.

Preferably, -R¹ and -R^{1a} of formula (II) are independently selected from the group consisting of -H, methyl and ethyl.

In one preferred embodiment at least one of -R¹ and -R^{1a} of formula (II) is -H, more preferably both -R¹ and -R^{1a} of formula (II) are -H.

In another preferred embodiment at least one of -R¹ and -R^{1a} of formula (II) is methyl, more preferably both -R¹ and -R^{1a} of formula (II) are methyl.

Preferably, -R² and -R^{2a} of formula (II) are independently selected from the group consisting of -H, methyl and ethyl. More preferably, at least one of -R² and -R^{2a} of formula (II) is -H. Even more preferably both -R² and -R^{2a} of formula (II) are H.

Preferably, -R³ and -R^{3a} of formula (II) are independently selected from the group consisting of -H, methyl, ethyl, propyl and butyl.

In one preferred embodiment at least one of -R³ and -R^{3a} of formula (II) is methyl, more preferably -R³ of formula (II) is methyl and -R^{3a} of formula (II) is -H.

In another preferred embodiment -R³ and -R^{3a} of formula (II) are both -H.

Preferably, -D is connected to -L¹- through an amine by forming an amide bond.

In a preferred embodiment the moiety -L¹- is of formula (IIb-i) 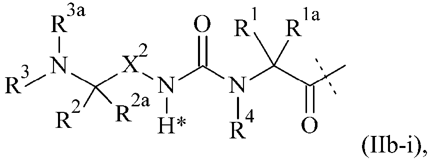 wherein
the dashed line indicates the attachment to an amine of -D which is a PTH moiety by forming an amide bond;
-R¹, -R^{1a}, -R², -R^{2a}, -R³, -R^{3a}, -R⁴ and -X²- are used as defined in formula (II); and
wherein -L¹- is substituted with -L²-Z and wherein -L¹- is optionally further substituted, provided that the hydrogen marked with the asterisk in formula (IIb-i) is not replaced by -L²-Z or a substituent.

Preferably -L¹- of formula (IIb-i) is substituted with one moiety -L²-Z.

Preferably the moiety -L¹- of formula (IIb-i) is not further substituted.

Preferably, -R¹ and -R^{1a} of formula (IIb-i) are independently selected from the group consisting of -H, methyl and ethyl. More preferably, at least one of -R¹ and -R^{1a} of formula (IIb-i) is methyl.

Even more preferably both -R¹ and -R^{1a} of formula (IIb-i) are methyl.

Preferably, -R⁴ of formula (IIb-i) is selected from the group consisting of -H, methyl and ethyl. More preferably, -R⁴ of formula (IIb-i) is -H.

Preferably, -X²- of formula (IIb-i) is -C(R⁸R^{8a})-.

Preferably -R⁸ and -R^{8a} of formula (IIb-i) are independently selected from the group consisting of -H, methyl and ethyl. More preferably at least one of -R⁸ and -R^{8a} of formula (IIb-i) is -H. Even more preferably both -R⁸ and -R^{8a} of formula (IIb-i) are -H.

Preferably, -R² and -R^{2a} of formula (IIb-i) are independently selected from the group consisting of -H, methyl and ethyl. More preferably, at least one of -R² and -R^{2a} of formula (IIb-i) is -H. Even more preferably both -R² and -R^{2a} of formula (IIb-i) are H.

Preferably, -R³ and -R^{3a} of formula (IIb-i) are independently selected from the group consisting of -H, methyl, ethyl, propyl and butyl. Even more preferably at least one of -R³ and -R^{3a} of formula (IIb-i) is -H. Even more preferably both -R³ and -R^{3a} of formula (IIb-i) are -H.

Preferably, -R³ or -R^{3a} of formula (IIb-i) is substituted with -L²-Z.

More preferably the moiety -L¹- is of formula (IIb-ii): 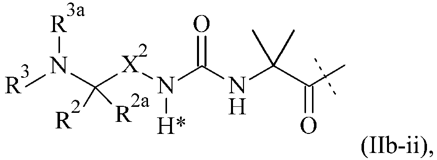
wherein the dashed line indicates the attachment to an amine of -D which is a PTH moiety by forming an amide bond;
-R², -R^{2a}, -R³, -R^{3a} and -X²- are used as defined in formula (II); and
wherein -L¹- is substituted with -L²-Z and wherein -L¹- is optionally further substituted, provided that the hydrogen marked with the asterisk in formula (IIb-ii) is not replaced by -L²-Z or a substituent.

Preferably -L¹- of formula (IIb-ii) is substituted with one moiety -L²-Z.

Preferably the moiety -L¹- of formula (IIb-ii) is not further substituted.

Preferably, -X²- of formula (IIb-ii) is -C(R⁸R^{8a})-.

Preferably -R⁸ and -R^{8a} of formula (IIb-ii) are independently selected from the group consisting of -H, methyl and ethyl. More preferably at least one of -R⁸ and -R^{8a} of formula (IIb-ii) is -H. Even more preferably both -R⁸ and -R^{8a} of formula (IIb-ii) are -H.

Preferably, -R² and -R^{2a} of formula (IIb-ii) are independently selected from the group consisting of -H, methyl and ethyl. More preferably, at least one of -R² and -R^{2a} of formula (IIb-ii) is -H. Even more preferably both -R² and -R^{2a} of formula (IIb-ii) are H.

Preferably, -R³ and -R^{3a} of formula (IIb-ii) are independently selected from the group consisting of -H, methyl, ethyl, propyl and butyl. Even more preferably at least one of -R³ and -R^{3a} of formula (IIb-ii) is -H. Even more preferably both -R³ and -R^{3a} of formula (IIb-ii) are -H.

Preferably, -R³ or -R^{3a} of formula (IIb-ii) is substituted with -L²-Z.

Even more preferably the moiety -L¹- is of formula (IIb-ii'): 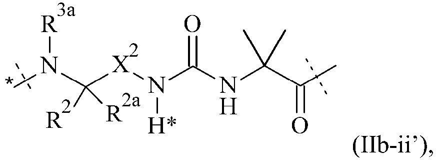 wherein
the unmarked dashed line indicates the attachment to an amine of -D which is a PTH moiety by forming an amide bond and the dashed line marked with the asterisk indicates attachment to -L²-;
-R², -R^{2a}, -R³, -R^{3a} and -X²- are used as defined in formula (II); and
wherein -L¹- is substituted with one moiety -L²-Z as indicated and wherein -L¹- is optionally further substituted, provided that the hydrogen marked with the asterisk in formula (IIb-ii') is not replaced by a substituent.

Preferably the moiety -L¹- of formula (IIb-ii') is not further substituted.

Preferably, -X²- of formula (IIb-ii') is -C(R⁸R^{8a})-.

Preferably -R⁸ and -R^{8a} of formula (IIb-ii') are independently selected from the group consisting of -H, methyl and ethyl. More preferably at least one of -R⁸ and -R^{8a} of formula (IIb-ii') is -H. Even more preferably both -R⁸ and -R^{8a} of formula (IIb-ii') are -H.

Preferably, -R² and -R^{2a} of formula (IIb-ii') are independently selected from the group consisting of -H, methyl and ethyl. More preferably, at least one of -R² and -R^{2a} of formula (IIb-ii') is -H. Even more preferably both -R² and -R^{2a} of formula (IIb-ii') are H.

Preferably, -R³ and -R^{3a} of formula (IIb-ii') are independently selected from the group consisting of -H, methyl, ethyl, propyl and butyl. Even more preferably at least one of -R³ and -R^{3a} of formula (IIb-ii') is -H. Even more preferably both -R³ and -R^{3a} of formula (IIb-ii') are -H.

Even more preferably the moiety -L¹- is of formula (IIb-iii): 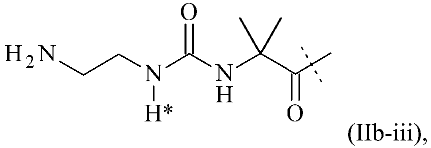 wherein
the dashed line indicates the attachment to an amine of -D which is a PTH moiety by forming an amide bond; and
wherein -L¹- is substituted with -L²-Z and wherein -L¹- is optionally further substituted, provided that the hydrogen marked with the asterisk in formula (IIb-iii) is not replaced by -L²-Z or a substituent.

Preferably -L¹- of formula (IIb-iii) is substituted with one moiety -L²-Z.

Preferably the moiety -L¹- of formula (IIb-iii) is not further substituted.

Preferably, -R³ or -R^{3a} of formula (IIb-iii) is substituted with -L²-Z.

Most preferably the moiety -L¹- is of formula (IIb-iii'): 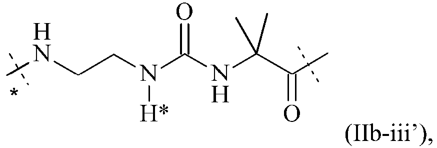 wherein
the dashed line indicates the attachment to an amine of -D which is a PTH moiety by forming an amide bond and the dashed line marked with the asterisk indicates attachment to -L²-;
-R², -R^{2a}, -R³, -R^{3a} and -X²- are used as defined in formula (II); and
wherein -L¹- is substituted with -L²-Z as indicated and wherein -L¹- is optionally further substituted, provided that the hydrogen marked with the asterisk in formula (IIb-iii') is not replaced by a substituent.

Preferably the moiety -L¹- of formula (IIb-iii') is not further substituted.

Another preferred moiety -L¹- is disclosed in WO2016/020373A1. Accordingly, in another preferred embodiment the moiety -L¹- is of formula (III): 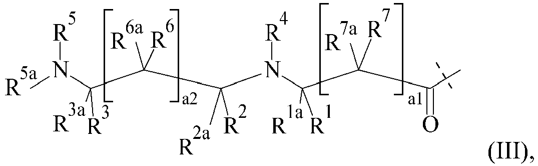 wherein
the dashed line indicates attachment to a primary or secondary amine or hydroxyl of -D which is a PTH moiety by forming an amide or ester linkage, respectively;
-R¹, -R^{1a}, -R², -R^{2a}, -R³ and -R^{3a} are independently of each other selected from the group consisting of -H, -C(R⁸R^{8a}R^{8b}), -C(=O)R⁸, -C≡N, -C(=NR⁸)R^{8a}, -CR⁸(=CR^{8a}R^{8b}), -C≡CR⁸ and -T;
-R⁴, -R⁵ and -R^{5a} are independently of each other selected from the group consisting of -H, -C(R⁹R^{9a}R^{9b}) and -T;
a1 and a2 are independently of each other 0 or 1;
each -R⁶, -R^{6a}, -R⁷, -R^{7a}, -R⁸, -R^{8a}, -R^{8b}, -R⁹, -R^{9a}, -R^{9b} are independently of each other selected from the group consisting of -H, halogen, -CN, -COOR¹⁰, -OR¹⁰, -C(O)R¹⁰, -C(O)N(R¹⁰R^{10a}), -S(O)₂N(R¹⁰R^{10a}), -S(O)N(R¹⁰R^{10a}), -S(O)₂R¹⁰, -S(O)R¹⁰, -N(R¹⁰)S(O)₂N(R^{10a}R^{10b}), -SR¹⁰, -N(R¹⁰R^{10a}), -NO₂, -OC(O)R¹⁰, -N(R¹⁰)C(O)R^{10a}, -N(R¹⁰)S(O)₂R^{10a}, -N(R¹⁰)S(O)R^{10a}, -N(R¹⁰)C(O)OR^{10a}, -N(R¹⁰)C(O)N(R^{10a}R^{10b}), -OC(O)N(R¹⁰R^{10a}), -T, C₁₋₂₀ alkyl, C₂₋₂₀ alkenyl, and C₂₋₂₀ alkynyl; wherein -T, C₁₋₂₀ alkyl, C₂₋₂₀ alkenyl, and C₂₋₂₀ alkynyl are optionally substituted with one or more -R¹¹, which are the same or different and wherein C₁₋₂₀ alkyl, C₂₋₂₀ alkenyl, and C₂₋₂₀ alkynyl are optionally interrupted by one or more groups selected from the group consisting of -T-, -C(O)O-, -O-, -C(O)-, -C(O)N(R¹²)-, -S(O)₂N(R¹²)-, -S(O)N(R¹²)-, -S(O)₂-, -S(O)-, -N(R¹²)S(O)₂N(R^{12a})-, -S-, -N(R¹²)-, -OC(OR¹²)(R^{12a})-, -N(R¹²)C(O)N(R^{12a})-, and -OC(O)N(R¹²)-;
each -R¹⁰, -R^{10a}, -R^{10b} is independently selected from the group consisting of -H, -T, C₁₋₂₀ alkyl, C₂₋₂₀ alkenyl, and C₂₋₂₀ alkynyl; wherein -T, C₁₋₂₀ alkyl, C₂₋₂₀ alkenyl, and C₂₋₂₀ alkynyl are optionally substituted with one or more -R¹¹, which are the same or different and wherein C₁₋₂₀ alkyl, C₂₋₂₀ alkenyl, and C₂₋₂₀ alkynyl are optionally interrupted by one or more groups selected from the group consisting of -T-, -C(O)O-, -O-, -C(O)-, -C(O)N(R¹²)-, -S(O)₂N(R¹²)-, -S(O)N(R¹²)-, -S(O)₂-, -S(O)-, -N(R¹²)S(O)₂N(R^{12a})-, -S-, -N(R¹²)-, -OC(OR¹²)(R^{12a})-, -N(R¹²)C(O)N(R^{12a})-, and -OC(O)N(R¹²)-;
each T is independently of each other selected from the group consisting of phenyl, naphthyl, indenyl, indanyl, tetralinyl, C₃₋₁₀ cycloalkyl, 3- to 10-membered heterocyclyl, and 8- to 11-membered heterobicyclyl; wherein each T is independently optionally substituted with one or more -R¹¹, which are the same or different;
each -R¹¹ is independently of each other selected from halogen, -CN, oxo (=O), -COOR¹³, -OR¹³, -C(O)R¹³, -C(O)N(R¹³R^{13a}), -S(O)₂N(R¹³R^{13a}), -S(O)N(R¹³R^{13a}), -S(O)₂R¹³, -S(O)R¹³, -N(R¹³)S(O)₂N(R^{13a}R^{13b}), -SR¹³, -N(R¹³R^{13a}), -NO₂, -OC(O)R¹³, -N(R¹³)C(O)R^{13a}, -N(R¹³)S(O)₂R^{13a}, -N(R¹³)S(O)R^{13a}, -N(R¹³)C(O)OR^{13a}, -N(R¹³)C(O)N(R^{13a}R^{13b}), -OC(O)N(R¹³R^{13a}), and C₁₋₆ alkyl; wherein C₁₋₆ alkyl is optionally substituted with one or more halogen, which are the same or different;
each -R¹², -R^{12a}, -R¹³, -R^{13a}, -R^{13b} is independently selected from the group consisting of -H, and C₁₋₆ alkyl; wherein C₁₋₆ alkyl is optionally substituted with one or more halogen, which are the same or different;
optionally, one or more of the pairs -R¹/-R^{1a}, -R²/-R^{2a}, -R³/-R^{3a}, -R⁶/-R^{6a}, -R⁷/-R^{7a} are joined together with the atom to which they are attached to form a C₃₋₁₀ cycloalkyl or a 3- to 10-membered heterocyclyl;
optionally, one or more of the pairs -R¹/-R², -R¹/-R³, -R¹/-R⁴, -R¹/-R⁵, -R¹/-R⁶, -R¹/-R⁷, -R²/-R³, -R²/-R⁴, -R²/-R⁵, -R²/-R⁶, -R²/-R⁷ , -R³/-R⁴, -R³/-R⁵, -R³/-R⁶, -R³/-R⁷, -R⁴/-R⁵, -R⁴/-R⁶, -R⁴/-R⁷, -R⁵/-R⁶, -R⁵/-R⁷, -R⁶/-R⁷ are joint together with the atoms to which they are attached to form a ring A;
A is selected from the group consisting of phenyl; naphthyl; indenyl; indanyl; tetralinyl; C₃₋₁₀ cycloalkyl; 3- to 10-membered heterocyclyl; and 8- to 11-membered heterobicyclyl;
wherein -L¹- is substituted with -L²-Z and wherein -L¹- is optionally further substituted;
   wherein
   -L²- is a single chemical bond or a spacer; and
   -Z is a polymeric moiety or fatty acid-based moiety.

The optional further substituents of -L¹- of formula (III) are preferably as described above.

Preferably -L¹- of formula (III) is substituted with one moiety -L²-Z.

In one embodiment -L¹- of formula (III) is not further substituted.

Additional preferred embodiments for -L¹- are disclosed in EP1536334B1, WO2009/009712A1, WO2008/034122A1, WO2009/143412A2, WO2011/082368A2, and US8618124B2, which are herewith incorporated by reference in their entirety.

Additional preferred embodiments for -L¹- are disclosed in US8946405B2 and US8754190B2, which are herewith incorporated by reference in their entirety. Accordingly, a preferred moiety -L¹- is of formula (IV): 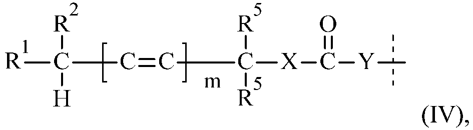 wherein
the dashed line indicates attachment to -D which is a PTH moiety and wherein attachment is through a functional group of -D selected from the group consisting of -OH, -SH and -NH₂;
m is 0 or 1;
at least one or both of -R¹ and -R² is/are independently of each other selected from the group consisting of -CN, -NO₂, optionally substituted aryl, optionally substituted heteroaryl, optionally substituted alkenyl, optionally substituted alkynyl, -C(O)R³, -S(O)R³, -S(O)₂R³, and -SR⁴,
one and only one of -R¹ and -R² is selected from the group consisting of -H, optionally substituted alkyl, optionally substituted arylalkyl, and optionally substituted heteroarylalkyl;
-R³ is selected from the group consisting of -H, optionally substituted alkyl, optionally substituted aryl, optionally substituted arylalkyl, optionally substituted heteroaryl, optionally substituted heteroarylalkyl, -OR⁹ and -N(R⁹)₂;
-R⁴ is selected from the group consisting of optionally substituted alkyl, optionally substituted aryl, optionally substituted arylalkyl, optionally substituted heteroaryl, and optionally substituted heteroarylalkyl;
each -R⁵ is independently selected from the group consisting of -H, optionally substituted alkyl, optionally substituted alkenylalkyl, optionally substituted alkynylalkyl, optionally substituted aryl, optionally substituted arylalkyl, optionally substituted heteroaryl and optionally substituted heteroarylalkyl;
-R⁹ is selected from the group consisting of -H and optionally substituted alkyl;
-Y- is absent and -X- is -O- or -S-; or
-Y- is -N(Q)CH₂- and -X- is -O-;
Q is selected from the group consisting of optionally substituted alkyl, optionally substituted aryl, optionally substituted arylalkyl, optionally substituted heteroaryl and optionally substituted heteroarylalkyl;
optionally, -R¹ and -R² may be joined to form a 3 to 8-membered ring; and
optionally, both -R⁹ together with the nitrogen to which they are attached form a heterocyclic ring;
wherein -L¹- is substituted with -L²-Z and wherein -L¹- is optionally further substituted;
   wherein
   - -L²-: is a single chemical bond or a spacer; and
   - -Z: is a polymeric moiety or fatty acid-based moiety.

Only in the context of formula (IV) the terms used have the following meaning:
The term "alkyl" as used herein includes linear, branched or cyclic saturated hydrocarbon groups of 1 to 8 carbon atoms, or in some embodiments 1 to 6 or 1 to 4 carbon atoms.

The term "alkoxy" includes alkyl groups bonded to oxygen, including methoxy, ethoxy, isopropoxy, cyclopropoxy, cyclobutoxy, and similar.

The term "alkenyl" includes non-aromatic unsaturated hydrocarbons with carbon-carbon double bonds.

The term "alkynyl" includes non-aromatic unsaturated hydrocarbons with carbon-carbon triple bonds.

The term "aryl" includes aromatic hydrocarbon groups of 6 to 18 carbons, preferably 6 to 10 carbons, including groups such as phenyl, naphthyl, and anthracenyl. The term "heteroaryl" includes aromatic rings comprising 3 to 15 carbons containing at least one N, O or S atom, preferably 3 to 7 carbons containing at least one N, O or S atom, including groups such as pyrrolyl, pyridyl, pyrimidinyl, imidazolyl, oxazolyl, isoxazolyl, thiazolyl, isothiazolyl, quinolyl, indolyl, indenyl, and similar.

In some instance, alkenyl, alkynyl, aryl or heteroaryl moieties may be coupled to the remainder of the molecule through an alkylene linkage. Under those circumstances, the substituent will be referred to as alkenylalkyl, alkynylalkyl, arylalkyl or heteroarylalkyl, indicating that an alkylene moiety is between the alkenyl, alkynyl, aryl or heteroaryl moiety and the molecule to which the alkenyl, alkynyl, aryl or heteroaryl is coupled.

The term "halogen" includes bromo, fluoro, chloro and iodo.

The term "heterocyclic ring" refers to a 4 to 8 membered aromatic or non-aromatic ring comprising 3 to 7 carbon atoms and at least one N, O, or S atom. Examples are piperidinyl, piperazinyl, tetrahydropyranyl, pyrrolidine, and tetrahydrofuranyl, as well as the exemplary groups provided for the term "heteroaryl" above.

When a ring system is optionally substituted, suitable substituents are selected from the group consisting of alkyl, alkenyl, alkynyl, or an additional ring, each optionally further substituted. Optional substituents on any group, including the above, include halo, nitro, cyano, -OR, -SR, -NR₂, -OCOR, -NRCOR, -COOR, -CONR₂, -SOR, -SO₂R, -SONR₂, -SO₂N R₂, wherein each R is independently alkyl, alkenyl, alkynyl, aryl or heteroaryl, or two R groups taken together with the atoms to which they are attached form a ring.

Preferably -L¹- of formula (IV) is substituted with one moiety -L²-Z.

An additional preferred embodiment for -L¹- is disclosed in WO2013/036857A1, which is herewith incorporated by reference in its entirety. Accordingly, a preferred moiety -L¹- is of formula (V): 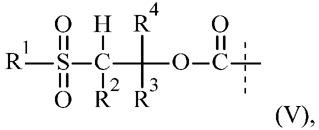 wherein
the dashed line indicates attachment to -D which is a PTH moiety and wherein attachment is through an amine functional group of -D;
-R¹ is selected from the group consisting of optionally substituted C₁-C₆ linear, branched, or cyclic alkyl; optionally substituted aryl; optionally substituted heteroaryl; alkoxy; and -NR⁵₂;
-R² is selected from the group consisting of -H; optionally substituted C₁-C₆ alkyl; optionally substituted aryl; and optionally substituted heteroaryl;
-R³ is selected from the group consisting of -H; optionally substituted C₁-C₆ alkyl; optionally substituted aryl; and optionally substituted heteroaryl;
-R⁴ is selected from the group consisting of -H; optionally substituted C₁-C₆ alkyl; optionally substituted aryl; and optionally substituted heteroaryl;
each -R⁵ is independently of each other selected from the group consisting of -H; optionally substituted C₁-C₆ alkyl; optionally substituted aryl; and optionally substituted heteroaryl; or when taken together two -R⁵ can be cycloalkyl or cycloheteroalkyl;
wherein -L¹- is substituted with -L²-Z and wherein -L¹- is optionally further substituted;
   wherein
   - -L²-: is a single chemical bond or a spacer;
   - -Z: is a polymeric moiety or fatty acid-based moiety.

Only in the context of formula (V) the terms used have the following meaning:
"Alkyl", "alkenyl", and "alkynyl" include linear, branched or cyclic hydrocarbon groups of 1-8 carbons or 1-6 carbons or 1-4 carbons wherein alkyl is a saturated hydrocarbon, alkenyl includes one or more carbon-carbon double bonds and alkynyl includes one or more carbon-carbon triple bonds. Unless otherwise specified these contain 1-6 C.

"Aryl" includes aromatic hydrocarbon groups of 6-18 carbons, preferably 6-10 carbons, including groups such as phenyl, naphthyl, and anthracene "Heteroaryl" includes aromatic rings comprising 3-15 carbons containing at least one N, O or S atom, preferably 3-7 carbons containing at least one N, O or S atom, including groups such as pyrrolyl, pyridyl, pyrimidinyl, imidazolyl, oxazolyl, isoxazolyl, thiszolyl, isothiazolyl, quinolyl, indolyl, indenyl, and similar.

The term "substituted" means an alkyl, alkenyl, alkynyl, aryl, or heteroaryl group comprising one or more substituent groups in place of one or more hydrogen atoms. Substituents may generally be selected from halogen including F, Cl, Br, and I; lower alkyl including linear, branched, and cyclic; lower haloalkyl including fluoroalkyl, chloroalkyl, bromoalkyl, and iodoalkyl; OH; lower alkoxy including linear, branched, and cyclic; SH; lower alkylthio including linear, branched and cyclic; amino, alkylamino, dialkylamino, silyl including alkylsilyl, alkoxysilyl, and arylsilyl; nitro; cyano; carbonyl; carboxylic acid, carboxylic ester, carboxylic amide, aminocarbonyl; aminoacyl; carbamate; urea; thiocarbamate; thiourea; ketne; sulfone; sulfonamide; aryl including phenyl, naphthyl, and anthracenyl; heteroaryl including 5-member heteroaryls including as pyrrole, imidazole, furan, thiophene, oxazole, thiazole, isoxazole, isothiazole, thiadiazole, triazole, oxadiazole, and tetrazole, 6-member heteroaryls including pyridine, pyrimidine, pyrazine, and fused heteroaryls including benzofuran, benzothiophene, benzoxazole, benzimidazole, indole, benzothiazole, benzisoxazole, and benzisothiazole.

Preferably -L¹- of formula (V) is substituted with one moiety -L²-Z.

A further preferred embodiment for -L¹- is disclosed in US7585837B2, which is herewith incorporated by reference in its entirety. Accordingly, a preferred moiety -L¹- is of formula (VI): 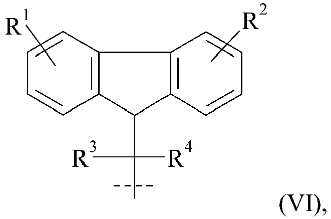 wherein
the dashed line indicates attachment to -D which is a PTH moiety and wherein attachment is through an amine functional group of -D;
R¹ and R² are independently selected from the group consisting of hydrogen, alkyl, alkoxy, alkoxyalkyl, aryl, alkaryl, aralkyl, halogen, nitro, -SO₃H, -SO₂NHR⁵, amino, ammonium, carboxyl, PO₃H₂, and OPO₃H₂;
R³, R⁴, and R⁵ are independently selected from the group consisting of hydrogen, alkyl, and aryl;
wherein -L¹- is substituted with -L²-Z and wherein -L¹- is optionally further substituted;
   wherein
   - -L²-: is a single chemical bond or a spacer; and
   - -Z: is a polymeric moiety or fatty acid-based moiety.

Suitable substituents for formulas (VI) are alkyl (such as C₁₋₆ alkyl), alkenyl (such as C₂₋₆ alkenyl), alkynyl (such as C₂₋₆ alkynyl), aryl (such as phenyl), heteroalkyl, heteroalkenyl, heteroalkynyl, heteroaryl (such as aromatic 4 to 7 membered heterocycle) or halogen moieties.

Only in the context of formula (VI) the terms used have the following meaning:
The terms "alkyl", "alkoxy", "alkoxyalkyl", "aryl", "alkaryl" and "aralkyl" mean alkyl radicals of 1-8, preferably 1-4 carbon atoms, e.g. methyl, ethyl, propyl, isopropyl and butyl, and aryl radicals of 6-10 carbon atoms, e.g. phenyl and naphthyl. The term "halogen" includes bromo, fluoro, chloro and iodo.

Preferably -L¹- of formula (VI) is substituted with one moiety -L²-Z.

A further preferred embodiment for -L¹- is disclosed in WO2002/089789A1, which is herewith incorporated by reference in its entirety. Accordingly, a preferred moiety -L¹- is of formula (VII): 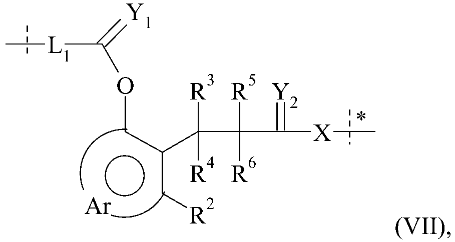 wherein
the dashed line indicates attachment to -D which is a PTH moiety and wherein attachment is through an amine functional group of -D;
L₁ is a bifunctional linking group,
Y₁ and Y₂ are independently O, S or NR⁷;
R², R³, R⁴, R⁵, R⁶ and R⁷ are independently selected from the group consisting of hydrogen, C₁₋₆ alkyls, C₃₋₁₂ branched alkyls, C₃₋₈ cycloalkyls, C₁₋₆ substituted alkyls, C₃₋₈ substituted cycloalkyls, aryls, substituted aryls, aralkyls, C₁₋₆ heteroalkyls, substituted C₁₋₆ heteroalkyls, C₁₋₆ alkoxy, phenoxy, and C₁₋₆ heteroalkoxy;
Ar is a moiety which when included in formula (VII) forms a multisubstituted aromatic hydrocarbon or a multi-substituted heterocyclic group;
X is a chemical bond or a moiety that is actively transported into a target cell, a hydrophobic moiety, or a combination thereof,
y is 0 or 1;
wherein -L¹- is substituted with -L²-Z and wherein -L¹- is optionally further substituted;
   wherein
   - -L²-: is a single chemical bond or a spacer; and
   - -Z: is a polymeric moiety or fatty acid-based moiety.

Only in the context of formula (VII) the terms used have the following meaning:
The term "alkyl" shall be understood to include, e.g. straight, branched, substituted C₁₋₁₂ alkyls, including alkoxy, C₃₋₈ cycloalkyls or substituted cycloalkyls, etc.

The term "substituted" shall be understood to include adding or replacing one or more atoms contained within a functional group or compounds with one or more different atoms.

Substituted alkyls include carboxyalkyls, aminoalkyls, dialkylaminos, hydroxyalkyls and mercaptoalkyls; substtued cycloalkyls include moieties such as 4-chlorocyclohexyl; aryls include moieties such as napthyl; substituted aryls include moieties such as 3-bromo-phenyl; aralkyls include moieties such as toluyl; heteroalkyls include moieties such as ethylthiophene; substituted heteroalkyls include moieties such as 3-methoxythiophone; alkoxy includes moieities such as methoxy; and phenoxy includes moieties such as 3-nitrophenoxy. Halo- shall be understood to include fluoro, chloro, iodo and bromo.

Preferably -L¹- of formula (VII) is substituted with one moiety -L²-Z.

In another preferred embodiment -L¹- comprises a substructure of formula (VIII) 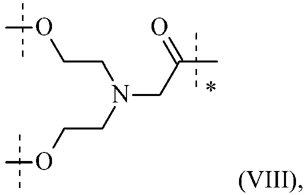 wherein
the dashed line marked with the asterisk indicates attachment to a nitrogen of -D which is a PTH moiety by forming an amide bond;
the unmarked dashed lines indicate attachment to the remainder of -L¹-; and wherein -L¹- is substituted with -L²-Z and wherein -L¹- is optionally further substituted;
   wherein
   - -L²-: is a single chemical bond or a spacer; and
   - -Z: is a polymeric moiety or fatty acid-based moiety.

Preferably -L¹- of formula (VIII) is substituted with one moiety -L²-Z.

In one embodiment -L¹- of formula (VIII) is not further substituted.

In another preferred embodiment -L¹- comprises a substructure of formula (IX) 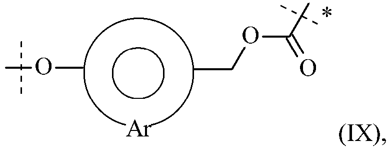 wherein
the dashed line marked with the asterisk indicates attachment to a nitrogen of -D which is a PTH moiety by forming a carbamate bond;
the unmarked dashed lines indicate attachment to the remainder of -L¹-; and
wherein -L¹- is substituted with -L²-Z and wherein -L¹- is optionally further substituted;
   wherein
   - -L²-: is a single chemical bond or a spacer; and
   - -Z: is a polymeric moiety or fatty acid-based moiety.

Preferably -L¹- of formula (IX) is substituted with one moiety -L²-Z.

In one embodiment -L¹- of formula (IX) is not further substituted.

In the conjugates of the present invention -L²- is a chemical bond or a spacer moiety.

In one embodiment -L²- is a chemical bond.

In another embodiment -L²- is a spacer moiety.

When -L²- is other than a single chemical bond, -L²- is preferably selected from the group consisting of -T-, -C(O)O-, -O-, -C(O)-, -C(O)N(R^{y1})-, -S(O)₂N(R^{y1})-, -S(O)N(R^{y1})-, -S(O)₂-, -S(O)-, -N(R^{y1})S(O)₂N(R^{y1a})-, -S-, -N(R^{y1})-, -OC(OR^{y1})(R^{y1a})-, -N(R^{y1})C(O)N(R^{y1a})-, -OC(O)N(R^{y1})-, C₁₋₅₀ alkyl, C₂₋₅₀ alkenyl, and C₂₋₅₀ alkynyl; wherein -T-, C₁₋₅₀ alkyl, C₂₋₅₀ alkenyl, and C₂₋₅₀ alkynyl are optionally substituted with one or more -R^{y2}, which are the same or different and wherein C₁₋₅₀ alkyl, C₂₋₅₀ alkenyl, and C₂₋₅₀ alkynyl are optionally interrupted by one or more groups selected from the group consisting of -T-, -C(O)O-, -O-, -C(O)-, -C(O)N(R^{y3})-, -S(O)₂N(R^{y3})-, -S(O)N(R^{y3})-, -S(O)₂-, -S(O)-, -N(R^{y3})S(O)₂N(R^{y3a})-, -S-, -N(R^{y3})-, -OC(OR^{y3})(R^{y3a})-, -N(R^{y3})C(O)N(R^{y3a})-, and -OC(O)N(R^{y3})-;
-R^{y1} and -R^{y1a} are independently of each other selected from the group consisting of -H, -T, C₁₋₅₀ alkyl, C₂₋₅₀ alkenyl, and C₂₋₅₀ alkynyl; wherein -T, C₁₋₅₀ alkyl, C₂₋₅₀ alkenyl, and C₂₋₅₀ alkynyl are optionally substituted with one or more -R^{y2}, which are the same or different, and wherein C₁₋₅₀ alkyl, C₂₋₅₀ alkenyl, and C₂₋₅₀ alkynyl are optionally interrupted by one or more groups selected from the group consisting of -T-, -C(O)O-, -O-, -C(O)-, -C(O)N(R^{y4})-, -S(O)₂N(R^{y4})-, -S(O)N(R^{y4})-, -S(O)₂-, -S(O)-, -N(R^{y4})S(O)₂N(R^{y4a})-, -S-, -N(R^{y4})-, -OC(OR^{y4})(R^{y4a})-, -N(R^{y4})C(O)N(R^{y4a})-, and -OC(O)N(R^{y4})-;
each T is independently selected from the group consisting of phenyl, naphthyl, indenyl, indanyl, tetralinyl, C₃₋₁₀ cycloalkyl, 3- to 10-membered heterocyclyl, 8- to 11-membered heterobicyclyl, 8-to 30-membered carbopolycyclyl, and 8- to 30-membered heteropolycyclyl; wherein each T is independently optionally substituted with one or more -R^{y2}, which are the same or different;
each -R^{y2} is independently selected from the group consisting of halogen, -CN, oxo (=O), -COOR^{y5}, -OR^{y5}, -C(O)R^{y5}, -C(O)N(R^{y5}R^{y5a}), -S(O)₂N(R^{y5}R^{y5a}), -S(O)N(R^{y5}R^{y5a}), -S(O)₂R^{y5}, -S(O)R^{y5}, -N(R^{y5})S(O)₂N(R^{y5a} R^{y5b}), -SR^{y5}, -N(R^{y5}R^{y5a}), -NO₂, -OC(O)R^{y5}, -N(R^{y5})C(O)R^{y5a}, -N(R^{y5})S(O)₂R^{y5a}, -N(R^{y5})S(O)R^{y5a}, -N(R^{y5})C(O)OR^{y5a}, -N(R^{y5})C(O)N(R^{y5a}R^{y5b}), -OC(O)N(R^{y5}R^{y5a}), and C₁₋₆ alkyl; wherein C₁₋₆ alkyl is optionally substituted with one or more halogen, which are the same or different; and
each -R^{y3}, -R^{y3a}, -R^{y4}, -R^{y4a}, -R^{y5}, -R^{y5a} and -R^{y5b} is independently selected from the group consisting of -H, and C₁₋₆ alkyl, wherein C₁₋₆ alkyl is optionally substituted with one or more halogen, which are the same or different.
When -L²- is other than a single chemical bond, -L²- is even more preferably selected from -T-, -C(O)O-, -O-, -C(O)-, -C(O)N(R^{y1})-, -S(O)₂N(R^{y1})-, -S(O)N(R^{y1})-, -S(O)₂-, -S(O)-, -N(R^{y1})S(O)₂N(R^{y1a})-, -S-, -N(R^{y1})-, -OC(OR^{y1})(R^{y1a})-, -N(R^{y1})C(O)N(R^{y1a})-, -OC(O)N(R^{y1})-, C₁₋₅₀ alkyl, C₂₋₅₀ alkenyl, and C₂₋₅₀ alkynyl; wherein -T-, C₁₋₂₀ alkyl, C₂₋₂₀ alkenyl, and C₂₋₂₀ alkynyl are optionally substituted with one or more -R^{y2}, which are the same or different and wherein C₁₋₂₀ alkyl, C₂₋₂₀ alkenyl, and C₂₋₂₀ alkynyl are optionally interrupted by one or more groups selected from the group consisting of -T-, -C(O)O-, -O-, -C(O)-, -C(O)N(R^{y3})-, -S(O)₂N(R^{y3})-, -S(O)N(R^{y3})-, -S(O)₂-, -S(O)-, -N(R^{y3})S(O)₂N(R^{y3a})-, -S-, -N(R^{y3})-, -OC(OR^{y3})(R^{y3a})-, -N(R^{y3})C(O)N(R^{y3a})-, and -OC(O)N(R^{y3})-;
-R^{y1} and -R^{y1a} are independently of each other selected from the group consisting of -H, -T, C₁₋₁₀ alkyl, C₂₋₁₀ alkenyl, and C₂₋₁₀ alkynyl; wherein -T, C₁₋₁₀ alkyl, C₂₋₁₀ alkenyl, and C₂₋₁₀ alkynyl are optionally substituted with one or more -R^{y2}, which are the same or different, and wherein C₁₋₁₀ alkyl, C₂₋₁₀ alkenyl, and C₂₋₁₀ alkynyl are optionally interrupted by one or more groups selected from the group consisting of -T-, -C(O)O-, -O-, -C(O)-, -C(O)N(R^{y4})-, -S(O)₂N(R^{y4})-, -S(O)N(R^{y4})-, -S(O)₂-, -S(O)-, -N(R^{y4})S(O)₂N(R^{y4a})-, -S-, -N(R^{y4})-, -OC(OR^{y4})(R^{y4a})-, -N(R^{y4})C(O)N(R^{y4a})-, and -OC(O)N(R^{y4})-;
each T is independently selected from the group consisting of phenyl, naphthyl, indenyl, indanyl, tetralinyl, C₃₋₁₀ cycloalkyl, 3- to 10-membered heterocyclyl, 8- to 11-membered heterobicyclyl, 8-to 30-membered carbopolycyclyl, and 8- to 30-membered heteropolycyclyl; wherein each T is independently optionally substituted with one or more -R^{y2}, which are the same or different;
-R^{y2} is selected from the group consisting of halogen, -CN, oxo (=O), -COOR^{y5}, -OR^{y5}, -C(O)R^{y5}, -C(O)N(R^{y5}R^{y5a}), -S(O)₂N(R^{y5}R^{y5a}), -S(O)N(R^{y5}R^{y5a}), -S(O)₂R^{y5}, -S(O)R^{y5}, -N(R^{y5})S(O)₂N(R^{y5a}R^{y5b}), -SR^{y5}, -N(R^{y5}R^{y5a}), -NO₂, -OC(O)R^{y5}, -N(R^{y5})C(O)R^{y5a}, -N(R^{y5})S(O)₂R^{y5a}, -N(R^{y5})S(O)R^{y5a}, -N(R^{y5})C(O)OR^{y5a}, -N(R^{y5})C(O)N(R^{y5a}R^{y5b}), -OC(O)N(R^{y5}R^{y5a}), and C₁₋₆ alkyl; wherein C₁₋₆ alkyl is optionally substituted with one or more halogen, which are the same or different; and
each -R^{y3}, -R^{y3a}, -R^{y4}, -R^{y4a}, -R^{y5}, -R^{y5a} and -R^{y5b} is independently of each other selected from the group consisting of -H, and C₁₋₆ alkyl; wherein C₁₋₆ alkyl is optionally substituted with one or more halogen, which are the same or different.

When -L²- is other than a single chemical bond, -L²- is even more preferably selected from the group consisting of -T-, -C(O)O-, -O-, -C(O)-, -C(O)N(R^{y1})-, -S(O)₂N(R^{y1})-, -S(O)N(R^{y1})-, -S(O)₂-, -S(O)-, -N(R^{y1})S(O)₂N(R^{y1a})-, -S-, -N(R^{y1})-, -OC(OR^{y1})(R^{y1a})-, -N(R^{y1})C(O)N(R^{y1a})-, -OC(O)N(R^{y1})-, C₁₋₅₀ alkyl, C₂₋₅₀ alkenyl, and C₂₋₅₀ alkynyl; wherein -T-, C₁₋₅₀ alkyl, C₂₋₅₀ alkenyl, and C₂₋₅₀ alkynyl are optionally substituted with one or more -R^{y2}, which are the same or different and wherein C₁₋₅₀ alkyl, C₂₋₅₀ alkenyl, and C₂₋₅₀ alkynyl are optionally interrupted by one or more groups selected from the group consisting of -T-, -C(O)O-, -O-, -C(O)-, -C(O)N(R^{y3})-, -S(O)₂N(R^{y3})-, -S(O)N(R^{y3})-, -S(O)₂-, -S(O)-, -N(R^{y3})S(O)₂N(R^{y3a})-, -S-, -N(R^{y3})-, -OC(OR^{y3})(R^{y3a})-, -N(R^{y3})C(O)N(R^{y3a})-, and -OC(O)N(R^{y3})-;
-R^{y1} and -R^{y1a} are independently selected from the group consisting of -H, -T, C₁₋₁₀ alkyl, C₂₋₁₀ alkenyl, and C₂₋₁₀ alkynyl;
each T is independently selected from the group consisting of phenyl, naphthyl, indenyl, indanyl, tetralinyl, C₃₋₁₀ cycloalkyl, 3- to 10-membered heterocyclyl, 8- to 11-membered heterobicyclyl, 8-to 30-membered carbopolycyclyl, and 8- to 30-membered heteropolycyclyl;
each -R^{y2} is independently selected from the group consisting of halogen, and C₁₋₆ alkyl; and
each -R^{y3}, -R^{y3a}, -R^{y4}, -R^{y4a}, -R^{y5}, -R^{y5a} and -R^{y5b} is independently of each other selected from the group consisting of -H, and C₁₋₆ alkyl; wherein C₁₋₆ alkyl is optionally substituted with one or more halogen, which are the same or different.

Even more preferably, -L²- is a C₁₋₂₀ alkyl chain, which is optionally interrupted by one or more groups independently selected from -O-, -T- and -C(O)N(R^{y1})-; and which C₁₋₂₀ alkyl chain is optionally substituted with one or more groups independently selected from -OH, -T and -C(O)N(R^{y6}R^{y6a}); wherein -R^{y1}, -R^{y6}, -R^{y6a} are independently selected from the group consisting of H and C₁₋₄ alkyl and wherein T is selected from the group consisting of phenyl, naphthyl, indenyl, indanyl, tetralinyl, C₃₋₁₀ cycloalkyl, 3- to 10-membered heterocyclyl, 8- to 11-membered heterobicyclyl, 8-to 30-membered carbopolycyclyl, and 8- to 30-membered heteropolycyclyl.

Preferably, -L²- has a molecular weight in the range of from 14 g/mol to 750 g/mol.

Preferably, -L²- comprises a moiety selected from 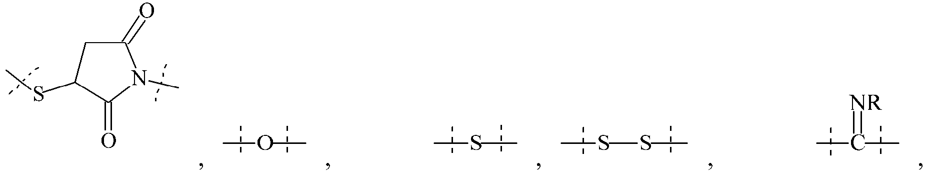 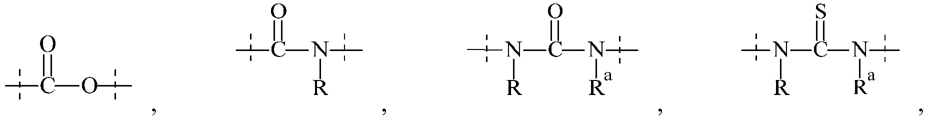 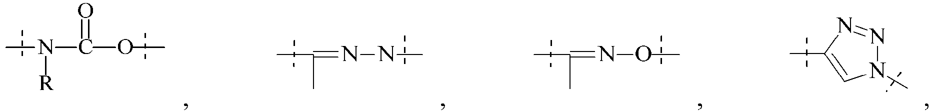 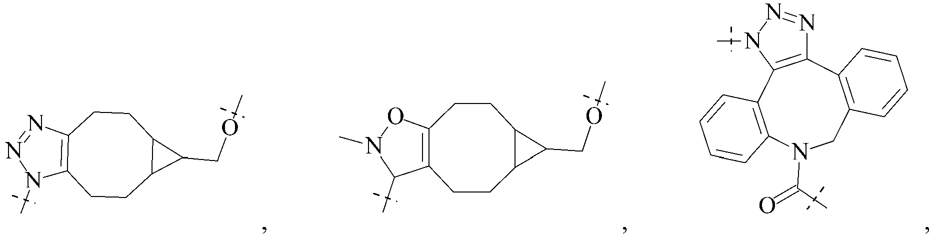 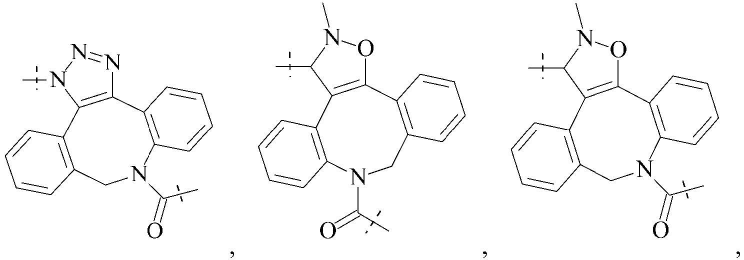 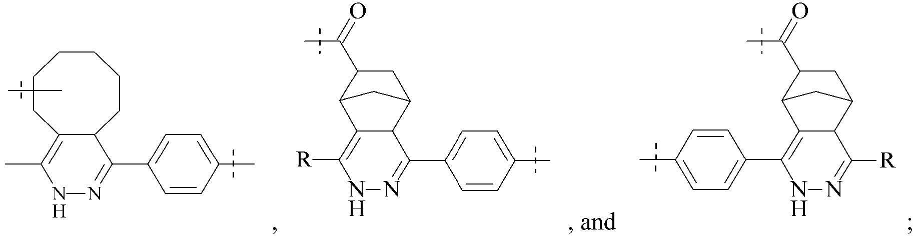 wherein
dashed lines indicate attachment to the rest of -L²-, -L¹-, and/or -Z, respectively; and
-R and -R^{a} are independently of each other selected from the group consisting of -H, methyl, ethyl, propyl, butyl, pentyl and hexyl.

In one preferred embodiment -L²- has a chain lengths of 1 to 20 atoms.

As used herein the term "chain length" with regard to the moiety -L²- refers to the number of atoms of -L²- present in the shortest connection between -L¹- and -Z.

Preferably, -L²- is of formula (i) 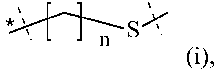 wherein
the dashed line marked with the asterisk indicates attachment to -L¹-;
the unmarked dashed line indicates attachment to -Z;
n is selected from the group consisting of 0, 1, 2, 3, 4, 5, 6, 7, 8, 9, 10, 11, 12, 13, 14, 15, 16, 17 and 18; and
wherein the moiety of formula (i) is optionally further substituted.

Preferably, n of formula (i) is selected from the group consisting of 3, 4, 5, 6, 7, 8, and 9. Even more preferably n of formula (i) is 4, 5, 6, or 7. In one embodiment n of formula (i) is 4. In another embodiment n of formula (i) is 5. In another embodiment n of formula (i) is 6.

In one preferred embodiment the moiety -L¹-L²- is selected from the group consisting of 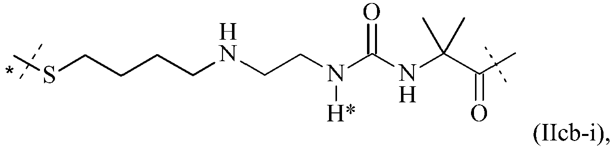 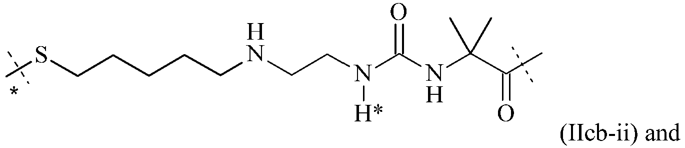 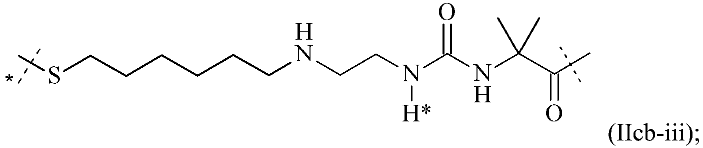 wherein
the unmarked dashed line indicates the attachment to a nitrogen of -D which is a PTH moiety by forming an amide bond; and
the dashed line marked with the asterisk indicates attachment to -Z.

In one embodiment the moiety -L¹-L²- is of formula (IIcb-i).

In another embodiment the moiety -L¹-L²- is of formula (IIcb-ii).

In another embodiment the moiety -L¹-L²- is of formula (IIcb-iii).
-Z is a polymeric moiety or a fatty acid-based moiety. In one embodiment -Z is a fatty acid-based moiet. In another embodiment -Z is a polymeric moiety, preferably a polymeric moiety comprising a polymer selected from the group consisting of 2-methacryloyl-oxyethyl phosphoyl cholins, poly(acrylic acids), poly(acrylates), poly(acrylamides), poly(alkyloxy) polymers, poly(amides), poly(amidoamines), poly(amino acids), poly(anhydrides), poly(aspartamides), poly(butyric acids), poly(glycolic acids), polybutylene terephthalates, poly(caprolactones), poly(carbonates), poly(cyanoacrylates), poly(dimethylacrylamides), poly(esters), poly(ethylenes), poly(ethyleneglycols), poly(ethylene oxides), poly(ethyl phosphates), poly(ethyloxazolines), poly(glycolic acids), poly(hydroxyethyl acrylates), poly(hydroxyethyl-oxazolines), poly(hydroxymethacrylates), poly(hydroxypropylmethacrylamides), poly(hydroxypropyl methacrylates), poly(hydroxypropyloxazolines), poly(iminocarbonates), poly(lactic acids), poly(lactic-co-glycolic acids), poly(methacrylamides), poly(methacrylates), poly(methyloxazolines), poly(organophosphazenes), poly(ortho esters), poly(oxazolines), poly(propylene glycols), poly(siloxanes), poly(urethanes), poly(vinyl alcohols), poly(vinyl amines), poly(vinylmethylethers), poly(vinylpyrrolidones), silicones, celluloses, carbomethyl celluloses, hydroxypropyl methylcelluloses, chitins, chitosans, dextrans, dextrins, gelatins, hyaluronic acids and derivatives, functionalized hyaluronic acids, mannans, pectins, rhamnogalacturonans, starches, hydroxyalkyl starches, hydroxyethyl starches and other carbohydrate-based polymers, xylans, and copolymers thereof.

Preferably, -Z has a molecular weight ranging from 5 to 200 kDa. Even more preferably, -Z has a molecular weight ranging from 8 to 100 kDa, even more preferably ranging from 10 to 80 kDa, even more preferably from 12 to 60, even more preferably from 15 to 40 and most preferably -Z has a molecular weight of about 20 kDa. In another equally preferred embodiment -Z has a molecular weight of about 40 kDa.

In one embodiment such -Z comprises a protein. Preferred proteins are selected from the group consisting of carboxyl-terminal polypeptide of the chorionic gonadotropin as described in US 2012/0035101 A1 which are herewith incorporated by reference; albumin; XTEN sequences as described in WO 2011123813 A2 which are herewith incorporated by reference; proline/alanine random coil sequences as described in WO 2011/144756 A1 which are herewith incorporated by reference; proline/alanine/serine random coil sequences as described in WO 2008/155134 A1 and WO 2013/024049 A1 which are herewith incorporated by reference; and Fc fusion proteins.

In one embodiment -Z is a polysarcosine.

In another embodiment -Z comprises a poly(N-methylglycine).

In another embodiment -Z comprises a random coil protein moiety.

In one embodiment such random coil protein moiety comprises at least 25 amino acid residues and at most 2000 amino acids. Preferably such random coil protein moiety comprises at least 30 amino acid residues and at most 1500 amino acid residues. Even more preferably such random coil protein moiety comprises at least 50 amino acid residues and at most 500 amino acid residues.

In one embodiment -Z comprises a random coil protein moiety of which at least 80%, preferably at least 85%, even more preferably at least 90%, even more preferably at least 95%, even more preferably at least 98% and most preferably at least 99% of the total number of amino acids forming said random coil protein moiety are selected from alanine and proline. Even more preferably, at least 10%, but less than 75%, preferably less than 65%, of the total number of amino acid residues of such random coil protein moiety are proline residues. Preferably, such random coil protein moiety is as described in WO 2011/144756 A1, which is hereby incorporated by reference in its entirety. Even more preferably -Z comprises at least one moiety selected from the group consisting of SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:51 and SEQ ID NO:61 as disclosed in WO2011/144756 which are hereby incorporated by reference.

In another embodiment -Z comprises a random coil protein moiety of which at least 80%, preferably at least 85%, even more preferably at least 90%, even more preferably at least 95%, even more preferably at least 98% and most preferably at least 99% of the total number of amino acids forming said random coil protein moiety are selected from alanine, serine and proline. Even more preferably, at least 4%, but less than 40% of the total number of amino acid residues of such random coil protein moiety are proline residues. Preferably, such random coil protein moiety is as described in WO 2008/155134 A1, which is hereby incorporated by reference in its entirety. Even more preferably -Z comprises at least one moiety selected from the group consisting of SEQ ID NO:2, SEQ ID NO:4, SEQ ID NO:6, SEQ ID NO:8, SEQ ID NO:10, SEQ ID NO:12, SEQ ID NO:14, SEQ ID NO:16, SEQ ID NO:18, SEQ ID NO:20, SEQ ID NO:22, SEQ ID NO:24, SEQ ID NO:26, SEQ ID NO:28, SEQ ID NO:30, SEQ ID NO:32, SEQ ID NO:34, SEQ ID NO:36, SEQ ID NO:40, SEQ ID NO:42, SEQ ID NO:44, SEQ ID NO:46, SEQ ID NO:50, SEQ ID NO:52, SEQ ID NO:54 and SEQ ID NO:56 as disclosed in WO 2008/155134 A1, which are hereby incorporated by reference.

In another embodiment -Z comprises a random coil protein moiety of which at least 80%, preferably at least 85%, even more preferably at least 90%, even more preferably at least 95%, even more preferably at least 98% and most preferably at least 99% of the total number of amino acids forming said random coil protein moiety are selected from alanine, glycine, serine, threonine, glutamate and proline. Preferably, such random coil protein moiety is as described in WO 2010/091122 A1, which is hereby incorporated by reference. Even more preferably -Z comprises at least one moiety selected from the group consisting of SEQ ID NO:182, SEQ ID NO:183, SEQ ID NO:184; SEQ ID NO:185, SEQ ID NO:186, SEQ ID NO:187, SEQ ID NO:188, SEQ ID NO:189, SEQ ID NO:190, SEQ ID NO:191, SEQ ID NO:192, SEQ ID NO:193, SEQ ID NO:194, SEQ ID NO:195, SEQ ID NO:196, SEQ ID NO:197, SEQ ID NO:198, SEQ ID NO:199, SEQ ID NO:200, SEQ ID NO:201, SEQ ID NO:202, SEQ ID NO:203, SEQ ID NO:204, SEQ ID NO:205, SEQ ID NO:206, SEQ ID NO:207, SEQ ID NO:208, SEQ ID NO:209, SEQ ID NO:210, SEQ ID NO:211, SEQ ID NO:212, SEQ ID NO:213, SEQ ID NO:214, SEQ ID NO:215, SEQ ID NO:216, SEQ ID NO:217, SEQ ID NO:218, SEQ ID NO:219, SEQ ID NO:220, SEQ ID NO:221, SEQ ID NO:759, SEQ ID NO:760, SEQ ID NO:761, SEQ ID NO:762, SEQ ID NO:763, SEQ ID NO:764, SEQ ID NO:765, SEQ ID NO:766, SEQ ID NO:767, SEQ ID NO:768, SEQ ID NO:769, SEQ ID NO:770, SEQ ID NO:771, SEQ ID NO:772, SEQ ID NO:773, SEQ ID NO:774, SEQ ID NO:775, SEQ ID NO:776, SEQ ID NO:777, SEQ ID NO:778, SEQ ID NO:779, SEQ ID NO:1715, SEQ ID NO:1716, SEQ ID NO:1718, SEQ ID NO:1719, SEQ ID NO:1720, SEQ ID NO:1721 and SEQ ID NO:1722 as disclosed in WO2010/091122A1, which are hereby incorporated by reference.

In another embodiment -Z is a fatty acid-based moiety. Preferred fatty acid-based moieties are those disclosed in WO 2005/027978 A2 and WO 2014/060512 A1, which are herewith incorporated by reference.

In another embodiment -Z is a hyaluronic acid-based polymer.

In one embodiment -Z is a carrier as disclosed in WO 2012/02047 A1, which is herewith incorporated by reference.

In another embodiment -Z is a carrier as disclosed in WO 2013/024048 A1, which is herewith incorporated by reference.

In another preferred embodiment -Z is a PEG-based polymer, such as a linear, branched or multi-arm PEG-based polymer.

In one embodiment -Z is a linear PEG-based polymer.

In another embodiment -Z is a multi-arm PEG-based polymer. Preferably, -Z is a multi-arm PEG-based polymer having at least 4 PEG-based arms.

Preferably, such multi-arm PEG-based polymer -Z is connected to a multitude of moieties -L²-L¹-D, wherein each moiety -L²-L¹-D is preferably connected to the end of an arm, preferably to the end of an arm. Preferably such multi-arm PEG-based polymer -Z is connected to 2, 3, 4, 5, 6, 7, 8, 9, 10, 11, 12, 13, 14, 15 or 16 moieties -L²-L¹-D. Even more preferably such multi-arm PEG-based polymer -Z is connected to 2, 3, 4, 6 or 8 moieties -L²-L¹-D. Even more preferably such multi-arm PEG-based polymer -Z is connected to 2, 4 or 6 moieties -L²-L¹-D, even more preferably such multi-arm PEG-based polymer -Z is connected to 4 or 6 moieties -L²-L¹-D, and most preferably such multi-arm PEG-based polymer -Z is connected to 4 moieties -L²-L¹-D.

Preferably, such multi-arm PEG-based polymer -Z is a multi-arm PEG derivative as, for instance, detailed in the products list of JenKem Technology, USA (accessed by download from http://www.jenkemusa.com/Pages/PEGProducts.aspx on Dec 18, 2014), such as a 4-arm-PEG derivative, in particular a 4-arm-PEG comprising a pentaerythritol core, an 8-arm-PEG derivative comprising a hexaglycerin core, and an 8-arm-PEG derivative comprising a tripentaerythritol core. More preferably, the water-soluble PEG-based carrier -Z comprises a moiety selected from:
a 4-arm PEG Amine comprising a pentaerythritol core: 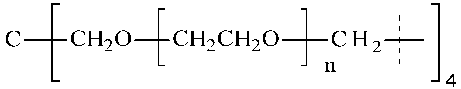
with n ranging from 20 to 500;
an 8-arm PEG Amine comprising a hexaglycerin core: 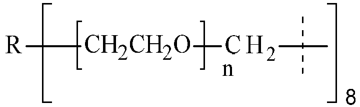
with n ranging from 20 to 500; and
R = hexaglycerin or tripentaerythritol core structure; and
a 6-arm PEG Amine comprising a sorbitol or dipentaerythritol core: 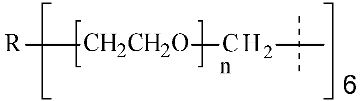
with n ranging from 20 to 500; and
R = comprising a sorbitol or dipentaerythritol core;
and wherein dashed lines indicate attachment to the rest of the PTH conjugate.

In a preferred embodiment -Z is a branched PEG-based polymer. In one embodiment -Z is a branched PEG-based polymer having one, two, three, four, five or six branching points. Preferably, -Z is a branched PEG-based polymer having one, two or three branching points. In one embodiment -Z is a branched PEG-based polymer having one branching point. In another embodiment -Z is a branched PEG-based polymer having two branching points. In another embodiment -Z is a branched PEG-based polymer having three branching points.

A branching point is preferably selected from the group consisting of -N<, -CH< and >C<.

Preferably, such branched PEG-based moiety -Z has a molecular weight of at least 10 kDa.

In one embodiment such branched moiety -Z has a molecular weight ranging from and including 10 kDa to 500 kDa, more preferably ranging from and including 10 kDa to 250 Da, even more preferably ranging from and including 10 kDa to 150 kDa, even more preferably ranging from and including 12 kDa to 100 kDa and most preferably ranging from and including 15 kDa to 80 kDa.

Preferably, such branched moiety -Z has a molecular weight ranging from and including 10 kDa to 80 kDa. In one embodiment the molecular weight is about 10 kDa. In another embodiment the molecular weight of such branched moiety -Z is about 20 kDa. In another embodiment the molecular weight of such branched moiety -Z is about 30 kDa. In another embodiment the molecular weight of such a branched moiety -Z is about 40 kDa. In another embodiment the molecular weight of such a branched moiety -Z is about 50 kDa. In another embodiment the molecular weight of such a branched moiety -Z is about 60 kDa. In another embodiment the molecular weight of such a branched moiety -Z is about 70 kDa. In another embodiment the molecular weight of such a branched moiety -Z is about 80 kDa. Most preferably, such branched moiety -Z has a molecular weight of about 40 kDa.

Preferably, -Z comprises a moiety

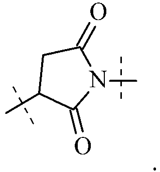

In an equally preferred embodiment -Z comprises an amide bond.

Preferably -Z comprises a moiety of formula (a) 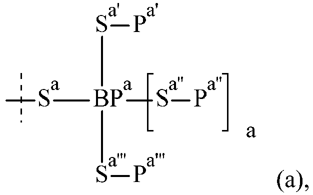 wherein
the dashed line indicates attachment to -L²- or to the remainder of -Z;
BP^{a} is a branching point selected from the group consisting of -N<, -CR< and >C<;
-R is selected from the group consisting of -H and C₁₋₆ alkyl;
a is 0 if BP^{a} is -N< or -CR< and n is 1 if BP^{a} is >C<;
-S^{a}-, -S^{a'}-, -S^{a"}- and -S^{a‴}- are independently of each other a chemical bond or are selected from the group consisting of C₁₋₅₀ alkyl, C₂₋₅₀ alkenyl, and C₂₋₅₀ alkynyl; wherein C₁₋₅₀ alkyl, C₂₋₅₀ alkenyl, and C₂₋₅₀ alkynyl are optionally substituted with one or more -R¹, which are the same or different and wherein C₁₋₅₀ alkyl, C₂₋₅₀ alkenyl, and C₂₋₅₀ alkynyl are optionally interrupted by one or more groups selected from the group consisting of -T-, -C(O)O-, -O-, -C(O)-, -C(O)N(R²)-, -S(O)₂N(R²)-, -S(O)N(R²)-, -S(O)₂-, -S(O)-, -N(R²)S(O)₂N(R^{2a})-, -S-, -N(R²)-, -OC(OR²)(R^{2a})-, -N(R²)C(O)N(R^{2a})-, and -OC(O)N(R²)-;
each -T- is independently selected from the group consisting of phenyl, naphthyl, indenyl, indanyl, tetralinyl, C₃₋₁₀ cycloalkyl, 3- to 10-membered heterocyclyl, 8- to 11-membered heterobicyclyl, 8-to 30-membered carbopolycyclyl, and 8- to 30-membered heteropolycyclyl; wherein each -T- is independently optionally substituted with one or more -R¹, which are the same or different;
each -R¹ is independently selected from the group consisting of halogen, -CN, oxo (=O), -COOR³, -OR³, -C(O)R³, -C(O)N(R³R^{3a}), -S(O)₂N(R³R^{3a}), -S(O)N(R³R^{3a}), -S(O)₂R³, -S(O)R³, -N(R³)S(O)₂N(R^{3a}R^{3b}), -SR³, -N(R³R^{3a}), -NO₂, -OC(O)R³, -N(R³)C(O)R^{3a}, -N(R³)S(O)₂R^{3a}, -N(R³)S(O)R^{3a}, -N(R³)C(O)OR^{3a}, -N(R³)C(O)N(R^{3a}R^{3b}), -OC(O)N(R³R^{3a}), and C₁₋₆ alkyl; wherein C₁₋₆ alkyl is optionally substituted with one or more halogen, which are the same or different;
each -R², -R^{2a}, -R³, -R^{3a} and -R^{3b} is independently selected from the group consisting of -H, and C₁₋₆ alkyl, wherein C₁₋₆ alkyl is optionally substituted with one or more halogen, which are the same or different; and
-P^{a'}, -P^{a"} and -P^{a‴} are independently a polymeric moiety.

In one embodiment BP^{a} of formula (a) is -N<.

In another embodiment BP^{a} of formula (a) is >C<.

In a preferred embodiment BP^{a} of formula (a) is -CR<. Preferably, -R is -H. Accordingly, a of formula (a) is 0.

In one embodiment -S^{a}- of formula (a) is a chemical bond.

In another embodiment -S^{a}- of formula (a) is selected from the group consisting of C₁₋₁₀ alkyl, C₂₋₁₀ alkenyl and C₂₋₁₀ alkynyl, which C₁₋₁₀ alkyl, C₂₋₁₀ alkenyl and C₂₋₁₀ alkynyl are optionally interrupted by one or more chemical groups selected from the group consisting of -T-, -C(O)O-, -O-, -C(O)-, -C(O)N(R⁴)-, -S(O)₂N(R⁴)-, -S(O)N(R⁴)-, -S(O)₂-, -S(O)-, -N(R⁴)S(O)₂N(R^{4a})-, -S-, -N(R⁴)-, -OC(OR⁴)(R^{4a})-, -N(R⁴)C(O)N(R^{4a})-, and -OC(O)N(R⁴)-; wherein -T- is a 3- to 10-membered heterocyclyl; and -R⁴ and -R^{4a} are independently selected from the group consisting of -H, methyl, ethyl, propyl and butyl.

Preferably -S^{a}- of formula (a) is selected from the group consisting of C₁₋₁₀ alkyl which is interrupted by one or more chemical groups selected from the group consisting of -T-, -C(O)N(R⁴)- and -O-.

In one embodiment -S^{a'}- of formula (a) is a chemical bond.

In another embodiment -S^{a'}- of formula (a) is selected from the group consisting of C₁₋₁₀ alkyl, C₂₋₁₀ alkenyl and C₂₋₁₀ alkynyl, which C₁₋₁₀ alkyl, C₂₋₁₀ alkenyl and C₂₋₁₀ alkynyl are optionally interrupted by one or more chemical groups selected from the group consisting of -C(O)O-, -O-, -C(O)-, -C(O)N(R⁴)-, -S(O)₂N(R⁴)-, -S(O)N(R⁴)-, -S(O)₂-, -S(O)-, -N(R⁴)S(O)₂N(R^{4a})-, -S-, -N(R⁴)-, -OC(OR⁴)(R^{4a})-, -N(R⁴)C(O)N(R^{4a})-, and -OC(O)N(R⁴)-; wherein -R⁴ and -R^{4a} are independently selected from the group consisting of -H, methyl, ethyl, propyl and butyl. Preferably -S^{a'}- of formula (a) is selected from the group consisting of methyl, ethyl, propyl, butyl, which are optionally interrupted by one or more chemical groups selected from the group consisting of -O-, -C(O)- and -C(O)N(R⁴)-.

In one embodiment -S^{a"}- of formula (a) is a chemical bond.

In another embodiment -S^{a"}- of formula (a) is selected from the group consisting of C₁₋₁₀ alkyl, C₂₋₁₀ alkenyl and C₂₋₁₀ alkynyl, which C₁₋₁₀ alkyl, C₂₋₁₀ alkenyl and C₂₋₁₀ alkynyl are optionally interrupted by one or more chemical groups selected from the group consisting of -C(O)O-, -O-, -C(O)-, -C(O)N(R⁴)-, -S(O)₂N(R⁴)-, -S(O)N(R⁴)-,-S(O)₂-, -S(O)-, -N(R⁴)S(O)₂N(R^{4a})-, -S-, -N(R⁴)-, -OC(OR⁴)(R^{4a})-, -N(R⁴)C(O)N(R^{4a})-, and -OC(O)N(R⁴)-; wherein -R⁴ and -R^{4a} are independently selected from the group consisting of -H, methyl, ethyl, propyl and butyl. Preferably -S^{a}"- of formula (a) is selected from the group consisting of methyl, ethyl, propyl, butyl, which are optionally interrupted by one or more chemical groups selected from the group consisting of -O-, -C(O)- and -C(O)N(R⁴)-.

In one embodiment -S^{a‴}- of formula (a) is a chemical bond.

In another embodiment -S^{a‴}- of formula (a) is selected from the group consisting of C₁₋₁₀ alkyl, C₂₋₁₀ alkenyl and C₂₋₁₀ alkynyl, which C₁₋₁₀ alkyl, C₂₋₁₀ alkenyl and C₂₋₁₀ alkynyl are optionally interrupted by one or more chemical groups selected from the group consisting of -C(O)O-, -O-, -C(O)-, -C(O)N(R⁴)-, -S(O)₂N(R⁴)-, -S(O)N(R⁴)-,-S(O)₂-, -S(O)-, -N(R⁴)S(O)₂N(R^{4a})-, -S-, -N(R⁴)-, -OC(OR⁴)(R^{4a})-, -N(R⁴)C(O)N(R^{4a})-, and -OC(O)N(R⁴)-; wherein -R⁴ and -R^{4a} are independently selected from the group consisting of -H, methyl, ethyl, propyl and butyl. Preferably -S^{a‴}- of formula (a) is selected from the group consisting of methyl, ethyl, propyl, butyl, which are optionally interrupted by one or more chemical groups selected from the group consisting of -O-, -C(O)- and -C(O)N(R⁴)-.

Preferably, -P^{a'}, -P^{a"} and -P^{a‴} of formula (a) independently comprise a polymer selected from the group consisting of 2-methacryloyl-oxyethyl phosphoyl cholins, poly(acrylic acids), poly(acrylates), poly(acrylamides), poly(alkyloxy) polymers, poly(amides), poly(amidoamines), poly(amino acids), poly(anhydrides), poly(aspartamides), poly(butyric acids), poly(glycolic acids), polybutylene terephthalates, poly(caprolactones), poly(carbonates), poly(cyanoacrylates), poly(dimethylacrylamides), poly(esters), poly(ethylenes), poly(ethyleneglycols), poly(ethylene oxides), poly(ethyl phosphates), poly(ethyloxazolines), poly(glycolic acids), poly(hydroxyethyl acrylates), poly(hydroxyethyl-oxazolines), poly(hydroxymethacrylates), poly(hydroxypropylmethacrylamides), poly(hydroxypropyl methacrylates), poly(hydroxypropyloxazolines), poly(iminocarbonates), poly(lactic acids), poly(lactic-co-glycolic acids), poly(methacrylamides), poly(methacrylates), poly(methyloxazolines), poly(organophosphazenes), poly(ortho esters), poly(oxazolines), poly(propylene glycols), poly(siloxanes), poly(urethanes), poly(vinyl alcohols), poly(vinyl amines), poly(vinylmethylethers), poly(vinylpyrrolidones), silicones, celluloses, carbomethyl celluloses, hydroxypropyl methylcelluloses, chitins, chitosans, dextrans, dextrins, gelatins, hyaluronic acids and derivatives, functionalized hyaluronic acids, mannans, pectins, rhamnogalacturonans, starches, hydroxyalkyl starches, hydroxyethyl starches and other carbohydrate-based polymers, xylans, and copolymers thereof.

More preferably, -P^{a'}, -P^{a"} and -P^{a‴} of formula (a) independently comprise a PEG-based moiety. Even more preferably, -P^{a'}, -P^{a"} and -P^{a‴} of formula (a) independently comprise a PEG-based moiety comprising at least 20% PEG, even more preferably at least 30%, even more preferably at least 40% PEG, even more preferably at least 50% PEG, even more preferably at least 60% PEG, even more preferably at least 70% PEG, even more preferably at least 80% PEG and most preferably at least 90% PEG.

Preferably, -P^{a'}, -P^{a"} and -P^{a‴} of formula (a) independently have a molecular weight ranging from and including 5 kDa to 50 kDa, more preferably have a molecular weight ranging from and including 5 kDa to 40 kDa, even more preferably ranging from and including 7.5 kDa to 35 kDa, even more preferably ranging from and 7.5 to 30 kDa, even more preferably ranging from and including 10 to 30 kDa.

In one embodiment -P^{a'}, -P^{a"} and -P^{a‴} of formula (a) have a molecular weight of about 5 kDa.

In another embodiment -P^{a'}, -P^{a"} and -P^{a‴} of formula (a) have a molecular weight of about 7.5 kDa.

In another embodiment -P^{a'}, -P^{a"} and -P^{a‴} of formula (a) have a molecular weight of about 10 kDa.

In another embodiment -P^{a'}, -P^{a"} and -P^{a‴} of formula (a) have a molecular weight of about 12.5 kDa.

In another embodiment -P^{a'}, -P^{a"} and -P^{a‴} of formula (a) have a molecular weight of about 15 kDa.

In another embodiment -P^{a'}, -P^{a"} and -P^{a‴} of formula (a) have a molecular weight of about 20 kDa.

In one embodiment -Z comprises one moiety of formula (a).

In another embodiment -Z comprises two moieties of formula (a).

In another embodiment -Z comprises three moieties of formula (a).

Preferably, -Z is a moiety of formula (a).

More preferably, -Z comprises a moiety of formula (b) 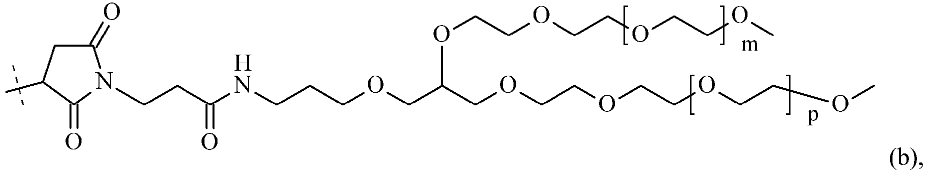 wherein
the dashed line indicates attachment to -L²- or to the remainder of -Z; and
m and p are independently of each other an integer ranging from and including 150 to 1000; preferably an integer ranging from and including 150 to 500; more preferably an integer ranging from and including 200 to 500; and most preferably an integer ranging from and including 400 to 500.

Preferably, m and p of formula (b) are the same integer.

Most preferably m and p of formula (b) are about 450.

Preferably, -Z is a moiety of formula (b).

In a preferred embodiment the PTH conjugate of the present invention is of formula (IIe-i): 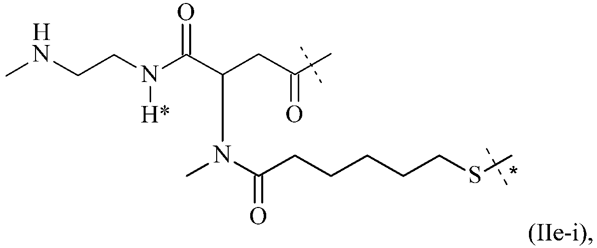 wherein
the unmarked dashed line indicates the attachment to a nitrogen of -D which is a PTH moiety by forming an amide bond; and
the dashed line marked with the asterisk indicates attachment to a moiety 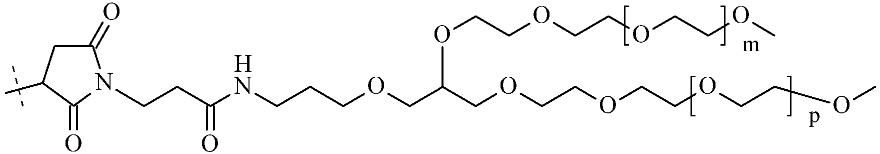 wherein
   m and p are independently an integer ranging from and including 400 to 500.

Preferably, -D is attached to the PTH conjugate of formula (IIe-i) through the N-terminal amine functional group of the PTH moiety.

In another preferred embodiment the PTH conjugate of the present invention is of formula (IIf-i): 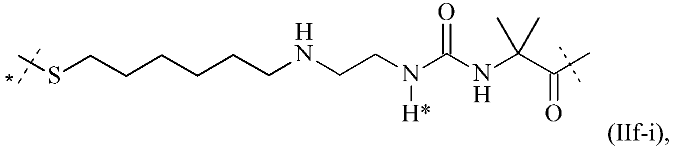 wherein
the unmarked dashed line indicates the attachment to a nitrogen of -D which is a PTH moiety by forming an amide bond; and
the dashed line marked with the asterisk indicates attachment to a moiety 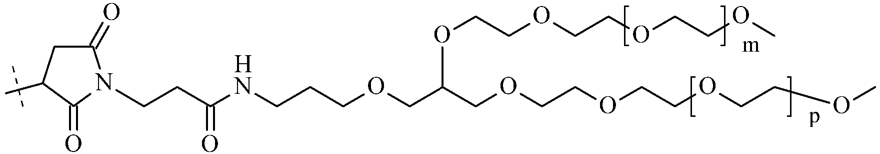 wherein
   m and p are independently an integer ranging from and including 400 to 500.

Preferably, -D is attached to the PTH conjugate of formula (IIf-i) through the N-terminal amine functional group of the PTH moiety.

If the PTH conjugate is present in a pharmaceutical composition such pharmaceutical composition comprises at least one PTH conjugate of the present invention and at least one excipient.

In one embodiment the pharmaceutical composition is a dry formulation. It is understood that such dry formulation requires reconstitution prior to administration to a patient. In another embodiment the pharmaceutical composition is a liquid formualation.

Such liquid or dry pharmaceutical composition comprises at least one excipient. Excipients used in parenteral formulations may be categorized as, for example, buffering agents, isotonicity modifiers, preservatives, stabilizers, anti-adsorption agents, oxidation protection agents, viscosifiers/viscosity enhancing agents, or other auxiliary agents. However, in some cases, one excipient may have dual or triple functions. Preferably, the at least one excipient comprised in the pharmaceutical composition of the present invention is selected from the group consisting of
(i) Buffering agents: physiologically tolerated buffers to maintain pH in a desired range, such as sodium phosphate, bicarbonate, succinate, histidine, citrate and acetate, sulphate, nitrate, chloride, pyruvate; antacids such as Mg(OH)₂ or ZnCO₃ may be also used;
(ii) Isotonicity modifiers: to minimize pain that can result from cell damage due to osmotic pressure differences at the injection depot; glycerin and sodium chloride are examples; effective concentrations can be determined by osmometry using an assumed osmolality of 285-315 mOsmol/kg for serum;
(iii) Preservatives and/or antimicrobials: multidose parenteral formulations require the addition of preservatives at a sufficient concentration to minimize risk of patients becoming infected upon injection and corresponding regulatory requirements have been established; typical preservatives include m-cresol, phenol, methylparaben, ethylparaben, propylparaben, butylparaben, chlorobutanol, benzyl alcohol, phenylmercuric nitrate, thimerosol, sorbic acid, potassium sorbate, benzoic acid, chlorocresol, and benzalkonium chloride;
(iv) Stabilizers: Stabilisation is achieved by strengthening of the protein-stabilising forces, by destabilisation of the denatured state, or by direct binding of excipients to the protein; stabilizers may be amino acids such as alanine, arginine, aspartic acid, glycine, histidine, lysine, proline, sugars such as glucose, sucrose, trehalose, polyols such as glycerol, mannitol, sorbitol, salts such as potassium phosphate, sodium sulphate, chelating agents such as EDTA, hexaphosphate, ligands such as divalent metal ions (zinc, calcium, etc.), other salts or organic molecules such as phenolic derivatives; in addition, oligomers or polymers such as cyclodextrins, dextran, dendrimers, PEG or PVP or protamine or HSA may be used;
(v) Anti-adsorption agents: Mainly ionic or non-ionic surfactants or other proteins or soluble polymers are used to coat or adsorb competitively to the inner surface of the formulation's container; e.g., poloxamer (Pluronic F-68), PEG dodecyl ether (Brij 35), polysorbate 20 and 80, dextran, polyethylene glycol, PEG-polyhistidine, BSA and HSA and gelatins; chosen concentration and type of excipient depends on the effect to be avoided but typically a monolayer of surfactant is formed at the interface just above the CMC value;
(vi) Oxidation protection agents: antioxidants such as ascorbic acid, ectoine, methionine, glutathione, monothioglycerol, morin, polyethylenimine (PEI), propyl gallate, and vitamin E; chelating agents such as citric acid, EDTA, hexaphosphate, and thioglycolic acid may also be used;
(vii) Viscosifiers or viscosity enhancers: in case of a suspension retard settling of the particles in the vial and syringe and are used in order to facilitate mixing and resuspension of the particles and to make the suspension easier to inject (i.e., low force on the syringe plunger); suitable viscosifiers or viscosity enhancers are, for example, carbomer viscosifiers like Carbopol 940, Carbopol Ultrez 10, cellulose derivatives like hydroxypropylmethylcellulose (hypromellose, HPMC) or diethylaminoethyl cellulose (DEAE or DEAE-C), colloidal magnesium silicate (Veegum) or sodium silicate, hydroxyapatite gel, tricalcium phosphate gel, xanthans, carrageenans like Satia gum UTC 30, aliphatic poly(hydroxy acids), such as poly(D,L- or L-lactic acid) (PLA) and poly(glycolic acid) (PGA) and their copolymers (PLGA), terpolymers of D,L-lactide, glycolide and caprolactone, poloxamers, hydrophilic poly(oxyethylene) blocks and hydrophobic poly(oxypropylene) blocks to make up a triblock of poly(oxyethylene)-poly(oxypropylene)-poly(oxyethylene) (e.g. Pluronic^{®}), polyetherester copolymer, such as a polyethylene glycol terephthalate/polybutylene terephthalate copolymer, sucrose acetate isobutyrate (SAIB), dextran or derivatives thereof, combinations of dextrans and PEG, polydimethylsiloxane, collagen, chitosan, polyvinyl alcohol (PVA) and derivatives, polyalkylimides, poly (acrylamide-co-diallyldimethyl ammonium (DADMA)), polyvinylpyrrolidone (PVP), glycosaminoglycans (GAGs) such as dermatan sulfate, chondroitin sulfate, keratan sulfate, heparin, heparan sulfate, hyaluronan, ABA triblock or AB block copolymers composed of hydrophobic A-blocks, such as polylactide (PLA) or poly(lactide-co-glycolide) (PLGA), and hydrophilic B-blocks, such as polyethylene glycol (PEG) or polyvinyl pyrrolidone; such block copolymers as well as the abovementioned poloxamers may exhibit reverse thermal gelation behavior (fluid state at room temperature to facilitate administration and gel state above sol-gel transition temperature at body temperature after injection);
(viii) Spreading or diffusing agent: modifies the permeability of connective tissue through the hydrolysis of components of the extracellular matrix in the intrastitial space such as but not limited to hyaluronic acid, a polysaccharide found in the intercellular space of connective tissue; a spreading agent such as but not limited to hyaluronidase temporarily decreases the viscosity of the extracellular matrix and promotes diffusion of injected drugs; and
(ix) Other auxiliary agents: such as wetting agents, viscosity modifiers, antibiotics, hyaluronidase; acids and bases such as hydrochloric acid and sodium hydroxide are auxiliary agents necessary for pH adjustment during manufacture.

The pharmaceutical composition comprising at least one PTH conjugate may be administered to a patient by various modes of administration, such as via topical, enteral or parenteral administration and by methods of external application, injection or infusion, including intraarticular, periarticular, intradermal, subcutaneous, intramuscular, intravenous, intraosseous, intraperitoneal, intrathecal, intracapsular, intraorbital, intravitreal, intratympanic, intravesical, intracardiac, transtracheal, subcuticular, subcapsular, subarachnoid, intraspinal, intraventricular, intrasternal injection and infusion, direct delivery to the brain via implanted device allowing delivery of the invention or the like to brain tissue or brain fluids (e.g., Ommaya Reservoir), direct intracerebroventricular injection or infusion, injection or infusion into brain or brain associated regions, injection into the subchoroidal space, retro-orbital injection and ocular instillation. Preferably the pharmaceutical composition comprising at least one PTH prodrug is administered via subcutaneous injection.

Preferably, the entire starting dose is administered to the patient in one step, preferably as one subcutaneous injection.

Subcutaneous injection is preferably done with a syringe and needle or with a pen injector, even more preferably with a pen injector.

In another aspect the present invention relates to the use of a PTH conjugate, in which a PTH moiety is reversibly conjugated to a polymeric moiety or fatty acid-based moiety, or a pharmaceutically acceptable salt thereof or a pharmaceutical composition comprising said PTH conjugate or pharmaceutically acceptable salt thereof for the manufacture of a medicament for the treatment, control, delay or prevention of a disease that can be treated, controlled, delayed or prevented with PTH, wherein the starting dose of said medicament ranges from 0.8 to 4.9 nmol/day. In certain embodiments the starting dose of such medicament ranges from 0.8 to 3.9 nmol/day.

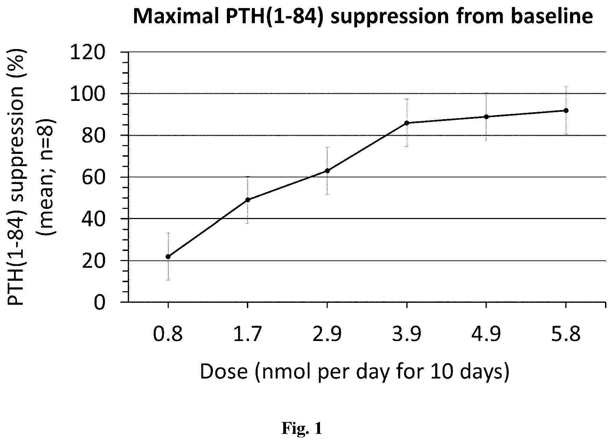

Figure 1 shows the mean of maximal PTH (1-84) suppression plotted for each dose level tested.

### Material and Methods

Compound **1** is shown below may be synthesized as described for compound 18 of example 18 of WO2017/148883A1:

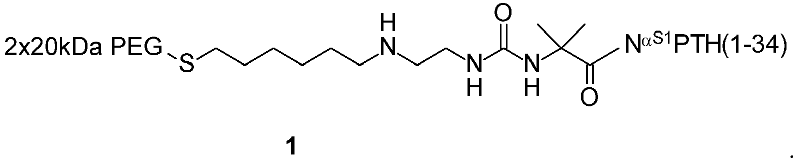

### Examples

### Example 1

To identify a safe starting dose for treating patients suffering from hypoparathyroidism, we investigated the pharmacodynamic effects of single and repeated daily dosing of a long-acting PTH molecule.

The safety, efficacy and pharmacokinetics of compound **1** was tested in a phase 1 study in healthy volunteers. The study investigated effects after single and multiple ascending doses in a randomized and placebo-controlled trial design. Each cohort consisted of 10 subjects (8 active, 2 placebo) and was administered either a single dose or 10 daily doses of TransCon PTH. The single dose cohorts were administered a single dose in the range 0.8-30.1 nmol while the multiple dose cohorts received one dose per day for 10 days in the range 0.8-5.8 nmol.

Compound 1 was generally well tolerated, with no drug-related serious or severe adverse events. The primary efficacy endpoints included albumin-adjusted serum calcium and endogenous secreted intact PTH(1-84).

The maximum tolerated dose (MTD) was 4.9 nmol/day of compound 1; 5 of 8 multiple ascending dose cohort participants (all female) who received compound 1 at 5.8, and both female placebo subjects, nmol/day had orthostatic hypotension, palpitations, and/or tachycardia and 1 had syncope. Two of 3 males had asymptomatic hypercalcemia. Observed adverse effects leading to MTD reflected known PTH pharmacology.

Total calcium was measured in serum by complexation using 2,2'-[1,8-Dihydroxy-3,6-disulphonaphthylene-2,7-bisazo]-bisbenzenearsonic acid (Arsenazo III, Beckman Coulter, Inc., USA) and detection by bichromatic absorbance at 600/700 nM. Albumin was measured by dye-binding method using 5,5-dibromo-o-cresolsulfonphthalein (Bromocresol Purple, Randox Laboratories, UK) and detection by bichromatic absorbance at 600/700 nm.

Albumin-adjusted serum calcium was calculated as total calcium (mg/dL) + 0.8 (4 - albumin in g/dL). Intact PTH(1-84) was measured with the "Architect Intact PTH" assay from Abbott Laboratories; an in vitro chemiluminescent microparticle immunoassay (CMIA) for the quantitative determination of intact parathyroid hormone (PTH) in human serum and plasma. All analytical methods were basically performed as described by the manufacturer.

In the multiple dose cohorts albumin adjusted serum calcium was measured daily for two days before first dosing, before first dose and daily for two weeks after the first dose. Intact PTH(1-84) was measured the day before dosing, before first dose and 1, 2, 3, 4, 9, 11 and 13 days after the first dose.

The effect of compound **1** was demonstrated by dose dependent responses in the efficacy parameters as presented in figure 1 and the table below.

Maximal PTH (1-84) suppresion was calculated for individual subjects as the ratio (in percent) of the highest change from baseline during the sampling period and the difference between the subject's baseline and the assay's lower limit of quantification (3 pg/mL). Baseline was defined as the PTH(1-84) measurement before the first dose. The mean of maximal PTH (1-84) suppression was then plotted for each dose level (figure 1). Daily doses ranging from 0.8 to 3.9 nmol induced a linear suppression of PTH(1-84) secretion, with endogenous PTH(1-84) secretion reaching maximal suppression at doses above 3.9 nmol.

Maximal increase from baseline in albumin adjusted serum calcium was calculated for individual subjects as the highest change from baseline during that subject's sampling period. Baseline was calculated as the mean (n=3) of samples collected before dosing. The mean of maximal increase was calculated for each dose level and presented in the table below.

| **Dose level** | **Albumin adjusted Serum Calcium (mg/dL)** |
|---|---|
| (daily for 10 days) | Mean of maximal increase from baseline (n=8) |
| 0.8 nmol | 0.44 |
| 1.7 nmol | 0.35 |
| 2.9 nmol | 0.66 |
| 3.9 nmol | 0.82 |
| 4.9 nmol | 1.21 |
| 5.8 nmol | 1.42 |

It was surprisingly found that a starting dose for hypoparathyroidism patients for a PTH conjugate could be identified by gradually increasing the dose in healthy subjects. The range for the starting dose will be the lowest dose to increase serum calcium by about 0.3-0.4 mg/dL from baseline in healthy subjects, and upper dose range will be the highest dose that suppress endogenous PTH(1-84) secretion and does not cause hypercalcemia in healthy subjects.

### Abbreviations

- HP: hypoparathyroidism
- PTH: parathyroid hormone

## Claims

1. A PTH conjugate, in which a PTH moiety is reversibly conjugated to a polymeric moiety or fatty acid-based moiety, or a pharmaceutically acceptable salt thereof or a pharmaceutical composition comprising said PTH conjugate or pharmaceutically acceptable salt thereof for use in the treatment, control, delay or prevention of a disease that can be treated, controlled, delayed or prevented with PTH, wherein the starting dose ranges from 0.8 to 4.9 nmol/day and wherein the PTH conjugate is of formula (Ia) or (Ib) 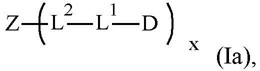 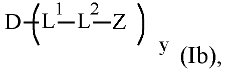 wherein
-D is a PTH moiety of SEQ ID NO:51;
-L¹- is a linker moiety covalently and reversibly attached to -D of formula (IIb-ii'): 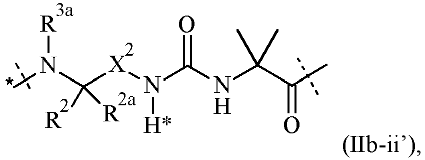 wherein
the unmarked dashed line indicates the attachment to an amine of -D by forming an amide bond and the dashed line marked with the asterisk indicates attachment to -L²-;
-X²- is -C(R⁸R^{8a})- or -C(R⁸R^{8a})-C(R⁹R^{9a})-;
-R², -R^{2a}, -R⁸, -R^{8a}, -R⁹ and -R^{9a} are independently -H or C₁₋₆ alkyl;
-R^{3a} is -H or C₁₋₆ alkyl, provided that in case -R^{3a} is other than -H it is connected to the nitrogen, to which it is attached, through an sp³-hybridized carbon atom;
-L¹- is substituted with one moiety -L²-Z and -L¹- is optionally further substituted, provided that the hydrogen marked with the asterisk in formula (IIb-ii') is not replaced by a substituent;
-L²- is a chemical bond or a spacer moiety;
-Z is a branched PEG-based polymer;
x is an integer selected from the group consisting of 1, 2, 3, 4, 5, 6, 7, 8, 9, 10, 11, 12, 13, 14, 15 and 16; and
y is an integer selected from the group consisting of 2, 3, 4 and 5.

2. The PTH conjugate of claim 1, wherein the starting dose is 4.4 ± 0.3 nmol/day.

3. The PTH conjugate of claim 1 or 2, wherein the starting dose is 4.4 ± 0.2 nmol/day.

4. The PTH conjugate of any one of claims 1 to 3, wherein the starting dose is 4.4 ± 0.1 nmol/day.

5. The PTH conjugate of any one of claims 1 to 4, wherein the starting dose is 4.4 nmol/day.

6. The PTH conjugate of any one of claims 1 to 5, wherein the disease that can be treated, controlled, delayed or prevented with PTH is selected from the group consisting of hypoparathyroidism, hyperphosphatemia, osteoporosis, fracture repair, osteomalacia, osteomalacia and osteoporosis in patients with hypophosphatasia, steroid-induced osteoporosis, male osteoporosis, arthritis, osteoarthritis, osteogenesis imperfecta, fibrous dysplasia, rheumatoid arthritis, Paget's disease, humoral hypercalcemia associated with malignancy, osteopenia, periodontal disease, bone fracture, alopecia, chemotherapy-induced alopecia, and thrombocytopenia.

7. The PTH conjugate of any one of claims 1 to 6, wherein the disease that can be treated, controlled, delayed or prevented with PTH is hypoparathyroidism.

8. The PTH conjugate of any one of claims 1 to 7, wherein the starting dose is administered to a patient as one subcutaneous injection.

9. The PTH conjugate of any one of claims 1 to 8, wherein -L²- is a spacer moiety.

10. The PTH conjugate of any one of claims 1 to 9, wherein -L²- is of formula (i): 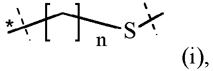 wherein
the dashed line marked with the asterisk indicates attachment to -L¹-;
the unmarked dashed line indicates attachment to -Z;
n is selected from the group consisting of 0, 1, 2, 3, 4, 5, 6, 7, 8, 9, 10, 11, 12, 13, 14, 15, 16, 17 and 18; and
wherein the moiety of formula (i) is optionally further substituted.

11. The PTH conjugate of any one of claims 1 to 10, wherein -Z comprises a moiety of formula (b): 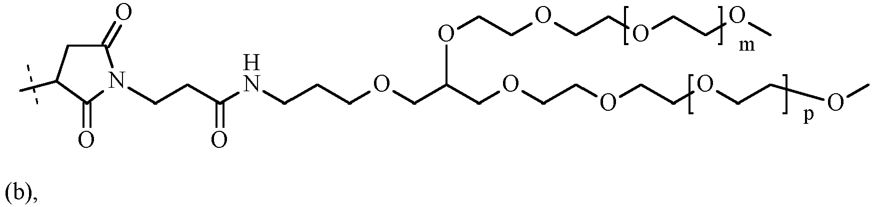 wherein
the dashed line indicates attachment to -L²- or to the remainder of -Z; and
m and p are independently of each other an integer ranging from and including 150 to 1000.

12. The PTH conjugate of any one of claims 1 to 11, wherein the PTH conjugate is of formula (IIf-i): 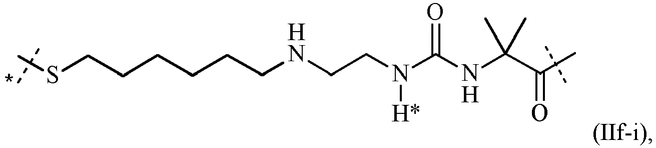 wherein
the unmarked dashed line indicates the attachment to a nitrogen of -D by forming an amide bond; and
the dashed line marked with the asterisk indicates attachment to a moiety 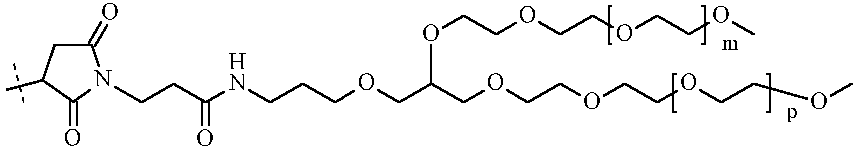 wherein
m and p are independently an integer ranging from and including 400 to 500.

13. The PTH conjugate of claim 12, wherein -D is attached to the PTH conjugate of formula (IIf-i) through the N-terminal amine functional group of -D.
